(19) Europäisches Patentamt
European Patent Office
Office européen des brevets

(11) Numéro de publication: **0 153 890**
**B1**

# (12) FASCICULE DE BREVET EUROPÉEN

(45) Date de publication du fascicule du brevet: **04.01.89**

(21) Numéro de dépôt: **85400254.0**

(22) Date de dépôt: **14.02.85**

(51) Int. Cl.⁴: **C 07 C 91/16**, C 07 C 93/14, C 07 C 147/06, C 07 C 147/14, C 07 C 149/42, C 07 D 213/38, C 07 D 213/70, C 07 D 295/08, A 61 K 31/135, A 61 K 31/395

## (54) Nouveaux dérivés de la phényl-3 propène-2 amine, leur préparation et les medicaments qui les contiennent.

(30) Priorité: **16.02.84 FR 8402338**

(43) Date de publication de la demande: **04.09.85 Bulletin 85/36**

(45) Mention de la délivrance du brevet: **04.01.89 Bulletin 89/1**

(84) Etats contractants désignés: **AT BE CH DE FR GB IT LI LU NL SE**

(56) Documents cité: **EP-A-0 080 721**

(73) Titulaire: **RHONE- POULENC SANTE, 20, avenue Raymond Aron, F-92160 Antony (FR)**

(72) Inventeur: **Barriere, Jean- Claude, 21, rue Henri Gilbert, F-91300 Massy (FR)**
Inventeur: **Corbet, Jean- Pierre, "Les Marronniers" Résidence "Charrière Blanche", F-69130 Ecully (FR)**
Inventeur: **Cotrel, Claude, 17 A, avenue du Docteur Arnold Netter, F-75012 Paris (FR)**
Inventeur: **Farge, Daniel, 30, rue des Pins Sylvestres, F-94320 Thiais (FR)**
Inventeur: **Paris, Jean- Marc, 8, rue des Acacias, F-77360 Vaires S/Marne (FR)**

(74) Mandataire: **Le Goff, Yves, RHONE- POULENC RECHERCHES Brevets Pharma 25, Quai Paul Doumer, F-92408 Courbevoie (FR)**

EP 0 153 890 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

**Description**

La présente invention concerne de nouveaux dérivés de la phényl-3 propène-2 amine de formule générale:

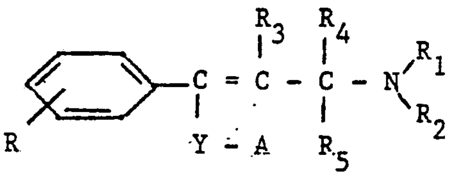

(I)

leurs sels, leur préparation et les compositions pharmaceutiques qui les contiennent.

Dans la formule générale (I), R représente un atome d'hydrogène ou d'halogène ou un radical alcoyle, alcoyloxy, alcoylthio, amino, alcoylamino, dialcoylamino ou trifluorométhyle, R représente un atome d'hydrogène ou un radical alcoyle,

- soit $R_4$ et $R_5$ représentent un atome d'hydrogène et $R_1$ et $R_2$, identiques ou différents, représentent un atome d'hydrogène ou un radical alcoyle éventuellement substitué par un radical alcényle contenant 2 à 4 atomes de carbone, ou bien $R_1$ et $R_2$ forment ensemble avec l'atome d'azote auquel ils sont liés un radical hétérocyclique saturé contenant 4 à 7 chaînons et contenant éventuellement un autre hétéroatome tel que l'oxygène, le soufre ou l'azote éventuellement substitué par un radical alcoyle,

- soit $R_4$ représente un atome d'hydrogène, $R_1$ représente un atome d'hydrogène ou un radical alcoyle et $R_2$ et $R_5$ forment ensemble un radical alcoylène contenant 3 ou 4 atomes de carbone,

et i) ou bien A représente un radical alcoyle ou un radical phényle non substitué ou substitué par un ou deux substituants choisis parmi les atomes d'halogène et les radicaux alcoyle, alcoyloxy, alcoylthio, amino, alcoylamino, dialcoylamino, nitro ou trifluorométhyle ou bien représente un radical pyridyle, benzyle ou cycloalcoyle contenant 3 à 6 atomes de carbone et Y représente un atome de soufre, un radical sulfinyle ou sulfonyle ou encore un radical de formule générale:

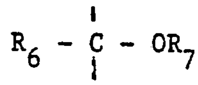

(II)

dans laquelle $R_6$ représente un atome d'hydrogène ou un radical alcoyle et $R_7$ représente un atome d'hydrogène ou un radical alcoylcarbonyle, alcoyloxycarbonyle, alcoylaminocarbonyle ou benzoyle éventuellement substitué par un ou deux substituants choisis parmi les atomes d'halogène et les radicaux alcoyle, alcoyloxy, alcoylthio, amino, alcoylamino, dialcoylamino, nitro ou trifluorométhyle, ii) ou bien Y et A forment ensemble un radical hydroxy-1 cycloalcoyle dont le cycle contient 5 ou 6 atomes de carbone, éventuellement accolé à un cycle benzénique,

étant entendu que dans les définitions qui précèdent et celles qui suivent, les radicaux alcoyle et portions alcoyle contiennent 1 à 4 atomes de carbone en chaîne droite ou ramifiée et que l'invention concerne tous les isoméres géométriques et optiques possibles ainsi que leurs mélanges.

Selon l'invention, les produits de formule générale (I) dans laquelle Y représente un radical de formule générale (II) dans laquelle $R_6$ est défini comme précédemment et $R_7$ représente un atome d'hydrogène et A, R, $R_1$ et $R_2$ sont définis comme précédemment à l'exception pour $R_1$ et/ou $R_2$ de représenter un atome d'hydrogène, et qui se présentent sous la configuration Z, peuvent être préparés par action d'un produit de formule générale:

$$A - CO - R_6 \qquad (III)$$

dans laquelle A et $R_6$ sont définis comme précédemment sur un carbanion de formule générale:

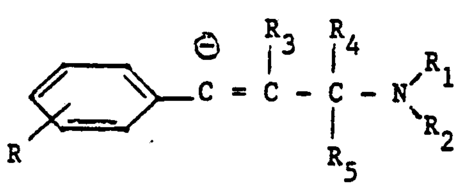

(IV)

dans laquelle les symboles sont définis comme précédemment à l'exception pour $R_1$ et/ou $R_2$ de représenter un atome d'hydrogène.

Généralement la réaction s'effectue dans un solvant organique inerte tel que l'hexane, le pentane, l'éther éthylique, le diméthoxy-1,2 éthane ou le tétrahydrofuranne ou un mélange de ces solvants, à une température

voisine de -30° C.

Le carbanion de formule générale (IV) peut être obtenu selon l'une des méthodes suivantes selon la nature des radicaux:

A) - Lorsque R est défini comme précédemment à l'exception de représenter un atome d'halogène ou un radical trifluorométhyle et $R_1$ et $R_2$ sont definis comme précédemment à l'exception pour $R_1$ et/ou $R_2$ de représenter un atome d'hydrogène, il peut être obtenu par action d'une base organométallique sur un produit de formule générale:

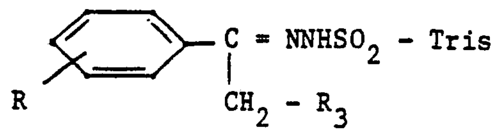

(V)

dans laquelle R est défini comme précédemment à l'exception de représenter un atome d'halogène ou un radical trifluorométhyle et le symbole Tris représente le radical tris-isopropyl-2,4,6 phényle, suivie de l'action d'un produit de formule générale:

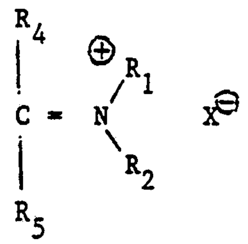

(VI)

dans laquelle les symboles sont définis comme précédemment à l'exception pour $R_1$ et/ou $R_2$ de représenter un atome d'hydrogène et $X^{\ominus}$ représente un ion halogénure (de préférence iodure), pour obtenir un intermédiaire de formule générale:

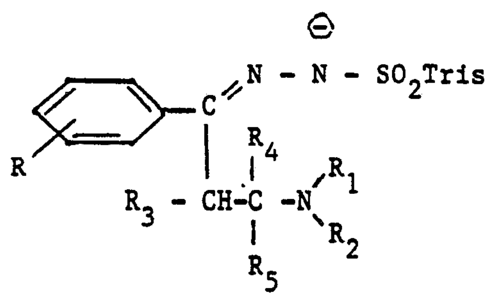

(VII)

dans laquelle les symboles sont définis comme précédemment à l'exception pour R de représenter un atome d'halogène ou un radical trifluorométhyle et pour $R_1$ et/ou $R_2$ de représenter un atome d'hydrogène et le symbole Tris est défini comme ci-dessus, cet intermédiaire de formule générale (VII) étant ensuite traité par une base organométallique pour donner, après fragmentation, le carbanion de formule générale (IV) correspondant.

Dans la pratique, il est avantageux de dissoudre le produit de formule générale (V) dans un solvant inerte tel que le diméthoxcy-1,2 éthane, de traiter la solution obtenue par deux équivalents de butyllithium en solution dans l'hexane en opérant à une température comprise entre -70 et -78° C, de condenser le produit de formule générale (VI) à une température voisine de -50° C, de traiter l'intermédiaire obtenu de formule générale (VII) par un équivalent de butyllithium en solution dans l'hexane à une température comprise entre -70 et -78° C et de laisser remonter la température au voisinage de 0° C. On obtient dans ce cas le carbanion de formule générale (IV) correspondant en solution dans un mélange de diméthoxy-1,2 éthane et d'hexane.

Les produits de formule générale (V) peuvent être préparés par action de la tris-isopropyl-2,4,6 benzènesulfonylhydrazine sur un produit de formule générale:

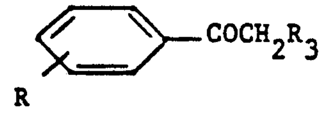

(VIII)

dans laquelle R et $R_3$ sont définis comme précédemment à l'exception pour R de représenter un atome d'halogène ou un radical trifluorométhyle, en opérant selon la méthode décrite par M.F. LIPTON et R.H.

SHAPIRO, J.Org. Chem. 43, 1409 (1978).

B) - Lorsque les symboles R, $R_1$, $R_2$ et $R_3$ sont définis comme dans la formule générale (IV), et $R_4$ et $R_5$ représentent un atome d'hydrogène, le carbanion peut être obtenu par action d'une base organométallique sur un produit de formule générale:

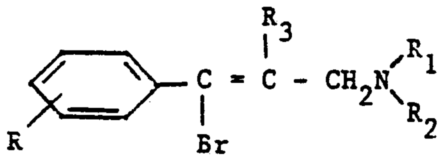

(IX)

dans laquelle les symboles ont la définition donnée pour le carbanion de formule générale (IV).

Dans la pratique, il est avantageux d'utiliser comme base organométallique le butylithium en solution dans l'hexane et d'effectuer la réaction dans un solvant organique inerte tel qu'un carbure (par exemple le pentane) ou un éther (par exemple l'éther éthylique) à une température comprise entre -78 et -10°C. Dans ce cas, on obtient le carbanion de formule générale (IV) en solution dans un mélange de pentane et d'hexane ou dans un mélange d'éther éthylique et d'hexane.

Les produits de formule générale (IX) peuvent être préparés à partir d'un produit de formule générale:

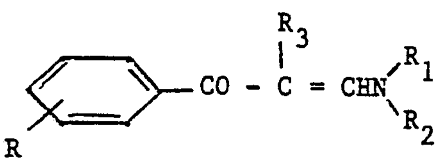

(X)

dans laquelle R, $R_1$, $R_2$ et $R_3$ ont les définitions données précédemment pour le carbanion de formule générale (IV) par action successive d'oxybromure de phosphore, de méthanol et d'un borohydrure de métal alcalin tel que le cyanoborohydrure de sodium.

On opère généralement dans un solvant organique tel que le chlorure de méthylène à une température comprise entre 0 et 10°C.

Les produits de formule générale (X) peuvent être préparés par application ou adaptation de la méthode décrite par H. MEERWEIN, W. FLORIAN, N. SCHON et G. STOPP, Ann. Chem., 641, 1, (1961).

C) - Lorsque R est défini comme précédemment à l'exception de représenter un atome d'halogène ou un radical trifluorométhyle et $R_1$ et/ou $R_2$ sont définis comme précédemment à l'exception de représenter un atome d'hydrogène, le carbanion peut être obtenu par action d'une base organométallique sur un produit de formule générale:

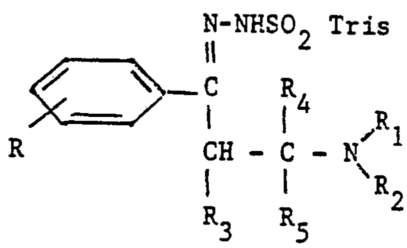

(XI)

dans laquelle R, $R_1$, $R_2$, $R_3$, $R_L$ et $R_5$ sont définis comme précédemment à l'exception pour R de représenter un atome d'halogène ou un radical trifluorométhyle et pour $R_1$ et/ou $R_2$ de représenter un atome d'hydrogène et le symbole Tris est défini comme précédemment dans la formule générale (V), pour obtenir un intermédiaire de formule générale:

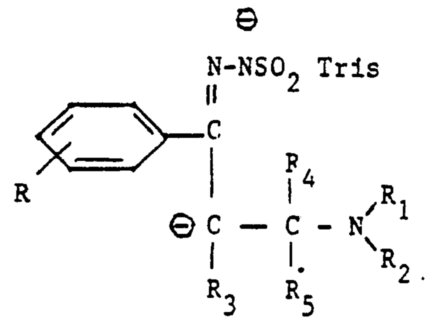

(XII)

dans laquelle R, $R_1$, $R_2$, $R_3$, $R_4$, $R_5$ et Tris ont les définitions correspondantes, cet intermédiaire se fragmentant ensuite pour donner le carbanion de formule générale (IV) correspondant.

Dans la pratique, on utilise comme base organométallique le n-butyllithium ou le tert-butyllithium en solution dans un carbure aliphatique tel que l'hexane ou le pentane, et l'on effectue la réaction dans un solvant organique inerte tel que le diméthoxy-1,2 éthane à une température comprise entre -78 et -70°C, puis on laisse remonter la température au voisinage de 0°C. Dans ce cas, on obtient le carbanion de formule générale (IV) correspondant en solution dans un mélange de diméthoxy-1,2 éthane et d'hexane ou de diméthoxy-1,2 éthane et de pentane.

Les produits de formule générale (XI) peuvent être préparés par action de la tris-isopropyl-2,4,6 benzènesulfonylhydrazine sur un produit de formule générale:

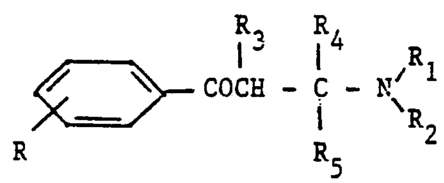

(XIII)

dans laquelle R, $R_1$, $R_2$, $R_3$, $R_4$ et $R_5$ sont définis comme précédemment à l'exception pour R de représenter un atome d'halogène ou un radical trifluorométhyle et pour $R_1$ et/ou $R_2$ de représenter un atome d'hydrogène.

On opère généralement dans un solvant organique tel qu'un alcool dans lequel on a dissout de l'acide chlorhydrique gazeux.

Les produits de formule générale (XIII) peuvent être préparés par application ou adaptation de la méthode décrite par C.E. MAXELL, Org. Syntheses, Coll. Vol. III, John WILLEY & SONS, Londres (1955) page 305.

Selon' l'invention, les produits de formule générale (I) dans laquelle Y représente un radical de formule générale (II) dans laquelle $R_6$ et $R_7$ représentent un atome d'hydrogène, A est défini précédemment en i), $R_1$ représente un atome d'hydrogène, $R_2$ représente un atome d'hydrogène ou un radical alcoyle éventuellement substitué par un radical alcényle contenant 2 à 4 atomes de carbone, R représente un atome d'hydrogène, $R_3$ est défini comme précédemment et $R_4$ et $R_5$ représentent un atome d'hydrogène, et qui se présentent sous la configuration 2, peuvent être préparés par ouverture du cycle dihydrofuranne dans un produit de formule générale:

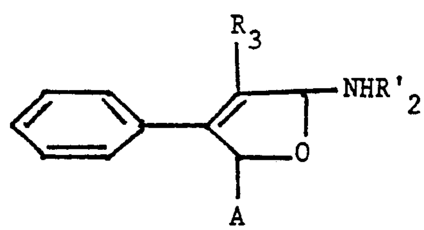

(XIV)

dans laquelle A est défini comme précédemment en i) et $R'_2$ représente un radical alcoyle éventuellement substitué par un radical alcényle contenant 2 à 4 atomes de carbone et $R_3$ est défini comme précédemment.

Généralement, on effectue l'ouverture du cycle dihydrofuranne au moyen d'un borohydrure de métal alcalin tel que le borohydrure de sodium dans un mélange hydro-alcoolique par exemple méthanol - eau, à une température comprise entre 0 et 20°C, de préférence à une température voisine de 5°C.

Lorsqu'on désire obtenir par ce procédé un produit de formule générale (I) dans laquelle Y représente un radical de formule générale (II) dans laquelle $R_6$ et $R_7$ représentent un atome d'hydrogène, A est défini comme précédemment en i), R, $R_1$, $R_2$, $R_4$ et $R_5$ représentent un atome d'hydrogène et $R_3$ est défini comme

précédemment, et qui se présentent sous la configuration 2, il est nécessaire de partir d'un produit de formule générale (XIV) dans laquelle $R'_2$ représente un radical alcoyle éventuellement substitué par un radical alcényle contenant 2 à 4 atomes de carbone, et d'éliminer ensuite ledit radical selon une méthode connue, par exemple la méthode de D. PICQ, M. COTTIN, D. ANKER et H. PACHECO, Tet. Letters (1983), 1399.

Les produits de formule générale (XIV) peuvent être préparés par action d'un produit de formule générale (XV), en équilibre. avec sa forme tautomère:

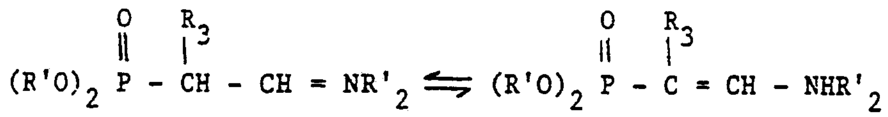 (XV)

dans laquelle $R'_2$ représente un radical alcoyle éventuellement substitué par un radical alcényle contenant 2 à 4 atomes de carbone et R' représente un radical alcoyle (de préférence éthyle) sur un produit de formule générale:

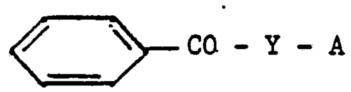 (XVI)

dans laquelle Y représente un radical de formule générale (II) dans laquelle $R_6$ et $R_7$ représentent un atome d'hydrogène et A est défini comme précédemment en i).

On opère généralement dans un solvant organique tel que le tétrahydrofuranne en présence d'un agent de condensation tel qu'un hydrure de métal alcalin, à une température comprise entre 20 et 100° C, de préférence à une température voisine de 60° C.

Les produits de formule générale (XV) peuvent être préparés selon la méthode décrite par N.D. DAWSON et A. BURCER, J. Am. Chem. Soc. 74, 5312, (1952).

Les produits de formule générale (XVI) peuvent être préparés par application ou adaptation des méthodes de L.R. KREPSKI, S.M. HEILMANN et J.K. RASMUSSEN, Tet. Letters, 4075, (1983) ou de R.E. KOENICKRAMER et H. ZIMMER Tet. Letters, 1017, (1980).

Selon l'invention, les produits de formule générale (I) dans laquelle Y représente un atome de soufre, $R_4$ et $R_5$ représentent un atome d'hydrogène, A est défini comme précédemment en i) et les autres symboles sont définis comme précédemment à l'exception pour $R_1$ et/ou $R_2$ de représenter un atome d'hydrogène et qui se présentent sous la configuration Z ou E peuvent être préparés par action d'un mercaptan de formule générale:

A - SH (XVII)

dans laquelle A est défini comme précédemment en i) sur un produit de formule générale:

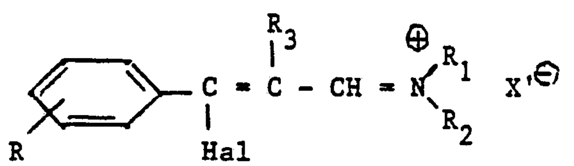 (XVIII)

dans laquelle R, $R_1$, $R_2$ et $R_3$ sont définis comme précédemment à l'exception pour $R_1$ et/ou $R_2$ de représenter un atome d'hydrogène, Hal représente un atome d'halogène tel que le chlore ou le brome, et $X'^{\ominus}$ représente un anion quelconque tel que halogénure, chlorate, perchlorate, phosphate ou fluoroborate, suivie d'une réduction de l'intermédiaire obtenu de formule générale:

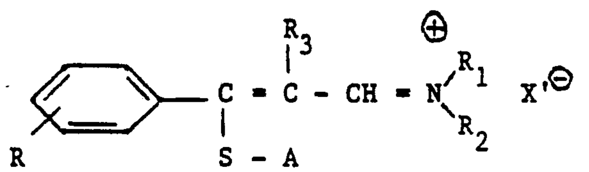 (XIX)

dans laquelle les symboles ont les définitions correspondantes.

La réaction du produit de formule (XVII) sur le produit de formule (XVIII) s'effectue généralement dans un solvant chloré, un alcool ou un éther ou un mélange de ces solvants, en présence d'un accepteur d'acide tel que la triéthylamine, à une température comprise entre 0 et 20° C.

La réduction de l'intermédiaire de formule générale (XIX) s'effectue généralement au moyen d'un borohydrure de métal alcalin tel que le borohydrure de sodium ou le cyanoborohydrure de sodium dans le

solvant dans lequel a été effectuée la condensation du produit de formule générale (XVII) sur le produit de formule générale (XVIII).

Les produits de formule générale (XVIII) peuvent être préparés par action d'un oxyhalogénure de phosphore sur un produit de formule générale (X) dans laquelle R, $R_1$ et $R_2$ sont définis comme précédemment à l'exception pour $R_1$ et/ou $R_2$ de représenter un atome d'hydrogène.

On opère généralement dans un solvant chloré tel que le chlorure de méthylène, à une température comprise entre 0 et 20° C.

Pour mettre en oeuvre la réaction consistant à faire réagir le produit de formule générale (XVII) sur le produit de formule générale (XVIII), il n'est pas nécessaire d'avoir isolé le produit de formule générale (XVIII). Après avoir préparé le produit de formule générale (XVIII) comme il a été dit précédemment, il suffit d'ajouter le produit de formule générale (XVII) et un accepteur d'acide dans le mélange réactionnel et de poursuivre la réaction à une température comprise entre 0 et 20° C, puis de réduire in situ l'intermédiaire de formule générale (XIX) comme il a été dit précédemment.

Selon l'invention, les produits de formule générale (I) dans laquelle Y représente un atome de soufre, $R_4$ et $R_5$ représentent um atome d'hydrogène, A est défini comme précédemment en i) et les autres symboles sont définis comme précédemment et qui se présentent sous la configuration Z ou E peuvent également être préparés par action de l'ammoniac ou d'une amine de formule générale:

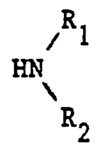
(XX)

dans laquelle $R_1$ et $R_2$ sont définis comme précédemment, sur un aldéhyde de formule générale:

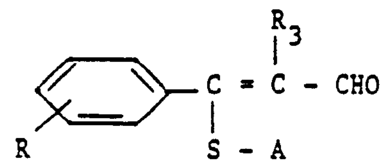
(XXI)

dans laquelle A est défini comme précédemment en i) et R et $R_3$ sont définis comme précédemment et qui se présente sous la configuration Z ou E.

On opère généralement dans un solvant organique tel qu'un alcool comme le méthanol, en présence d'un borohydrure alcalin comme le cyanoborohydrure de sodium et de tamis moléculaires, à une temprérarure voisine de 20° C.

Les produits de formule générale (XXI) peuvent être préparés par action d'un mercaptan de formule générale (XVII) défini comme précédemment sur un produit de formule générale:

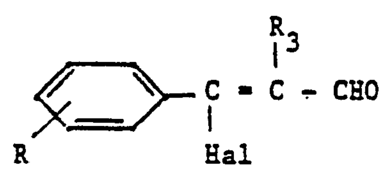
(XXII)

dans laquelle R et $R_3$ sont définis comme précédemment et Hal représente un atome d'halogène tel que le chlore ou le brome et qui se présente sous la configuration E ou Z.

On opère généralement dans un solvant organique tel que le chlorure de méthylène en présence d'un accepteur d'acide tel que la triéthylamine, à une température ccmprise entre 0 et 20° C.

Le produit de formule générale (XXII) peut être préparé par application ou adaptation de la méthode de C.M. BEATON, N.B. CHAPMAN et K. KLARKE, J.Chem. Soc. Perkin I, 2355, (1976).

Selon l'invention, les produits de formule générale (I) dans laquelle Y représente un radical de formule générale (II) dans laquelle $R_7$ représente un atome d'hydrogène ou bien Y est défini comme en il $R_3$ représente un atome d'hydrogène et les autres symboles sont definis comme précédemment et qui se présentent sous la configuration E, peuvent être préparés par action d'un dérivé organomagnésien de formule générale:

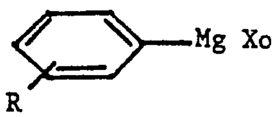

(XXIII)

dans laquelle R est défini comme précédemment et Xo représente un atome d'halogène (de préférence le brome) sur un produit de formule générale:

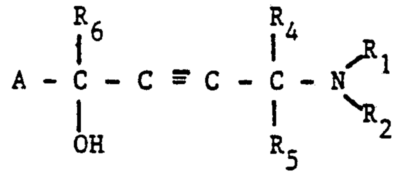

(XXIV)

dans laquelle les symboles sont définis comme précédemment.

On opère selon les méthodes connues de l'homme du métier pour faire réagir un dérivé organomagnésien sur un dérivé acétylénique sans toucher au reste de la molécule.

Lorsque $R_1$ et/ou $R_2$ représentent un atome d'hydrogène, il est évident pour l'homme du métier que la fonction amine correspondante devra être protégée dans le produit de formule générale (XXIV) avant de faire réagir le dérivé organomagmésien de formule (XXIII). On pourra employer tout moyen de blocage connu de l'homme du métier pour protéger une amine primaire ou secondaire, qui n'affecte pas le reste de la molécule et qui pourra être éliminée ensuite.

Les produits de formule générale (XXIV) peuvent être préparés par action d'un produit de formule générale:

$A - CO{-}R_6$ (XXV)

dans laquelle A et $R_6$ sont définis comme précédemment sur une propargylamine de formule générale:

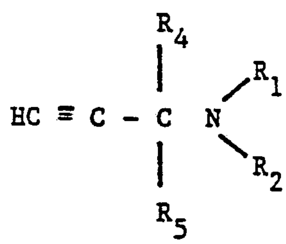

(XXVI)

dans laquelle $R_1$, $R_2$, $R_4$ et $R_5$ sont définis comme précédemment à l'exception pour $R_1$ et $R_2$ de représenter ensemble ou séparément un atome d'hydrogène.

Généralement on effectue la réaction dans un solvant organique inerte tel que le diméthoxy-1,2 éthane, à une température comprise entre -40 et 0°C, après avoir métallé par tout moyen connu la propargylamine de formule générale (XXVI). La métallation peut se faire par exemple au moyen d'une base organométallique tel que le n-butyllithium en solution dans l'hexane, à une température voisine dé -70°C.

Les produits de formule générale (XXVI) peuvent être préparés par action d'une amine de formule générale (XX) définie comme précédemment sur le bromure de propargyle, en opérant par application ou adaptation de la méthode de M. GAUDEMAR, Ann. Chim. (France) 13, 161, (1956).

Selon l'invention, les produits de formule générale (I) dans laquelle Y représente un radical de formule générale (II) dans laquelle $R_7$ représente un radical alcoylcarbonyle, alcoyloxycarbonyle ou benzoyle éventuellement substitué par un ou deux substituants choisis parmi les atomes d'halogène et les radicaux alcoyle, alcoyloxy, alcoylthio, amino, alcoylamino, dialcoylamino, nitro ou trifluorométhyle et les autres symboles sont définis comme précédemment, peuvent être obtenus par action d'un produit de formule générale:

$R'_7 — X_1$ (XXVII)

dans laquelle $R'_7$ représente un radical alcoylcarbonyle, alcoyloxycarbonyle ou benzoyle éventuellement substitué par un ou deux substituants choisis parmi les atomes d'halogène et les radicaux alcoyle, alcoyloxy, alcoylthio, amino, alcoylamino, dialcoylamino, nitro ou trifluorométhyle et $X_1$ représente un reste d'ester réactif tel qu'un halogénure sur un produit de formule générale (1) dans laquelle Y représente un radical de formule générale (II) dans laquelle $R_7$ représente un atome d'hydrogène et les autres symboles sont définis comme précédemment, c'est-à-dire un produit de formule générale:

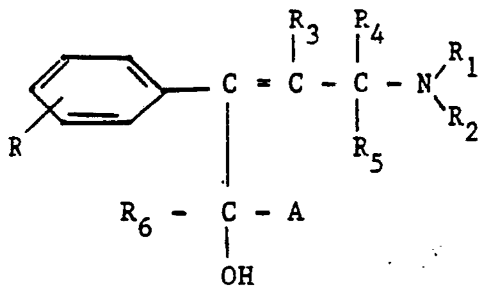

(XXVIII)

On opère par toute méthode connue de l'homme du métier pour acyler un alcool secondaire ou tertiaire, par exemple en opérant dans la pyridine à une température comprise entre 0 et 50° C ou bien dans un solvant inerte tel qu'un solvant chloré, en présence d'un accepteur d'acide lorsque $X_1$ représente un atome d'halogène.

Il est entendu pour l'homme du métier que, lorsque $R_1$ et/ou $R_2$ représentent un atome d'hydrogène, il est nécessaire de bloquer la fonction amine correspondante avant de faire réagir le produit de formule générale (XXVII) sur le produit de formule générale (XXVIII). La fonction amine est ensuite libérée après mise en oeuvre du procédé. Le blocage de l'amine peut se faire par exemple sous forme de radical tert-butyloxycarbonyle facilement clivable selon les méthodes connues sans toucher au radical $R_7$ introduit dans la molécule grâce au présent procédé.

Selon l'invention, les produits de formule générale (I) dans laquelle Y représente un atome de soufre ou un radical de formule générale (II) défini comme précédemment, $R_4$ et $R_5$ représentent un atome d'hydrogène et les autres symboles sont définis comme précédemment et qui se présentent sous la configuration E ou Z, peuvent être préparés par action de l'ammoniac, d'un précurseur d'amine ou d'une amine de formule générale (XX) sur un produit de formule générale:

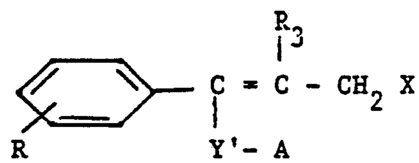

(XXIX)

dans laquelle A est defini comme précédemment en i) R et $R_3$ sont définis comme précédemment et

a) - soit X représente un atome de chlore et Y' représente un atome de soufre ou un radical de formule générale (II) dans laquelle $R_6$ et $R_7$ sont définis comme précédemment, à l'exception pour $R_7$ de représenter un atome d'hydrogène,

b) - soit X représente un atome de brome et Y' représente un atome de soufre,

et qui se présente sous la configuration E ou Z, suivie éventuellement d'une hydrolyse lorsqu'on veut obtenir le produit de formule générale (I) dans laquelle Y représente un radical de formule générale (II) dans laquelle $R_7$ représente un atome d'hydrogène, $R_4$ et $R_5$ représentent un atome d'hydrogène et les autres symboles sont définis comme précédemment.

On opère généralement dans un solvant organique tel qu'un alcool comme l'éthanol ou le diméthylformamide, en opérant éventuellement sous pression, à une température qui peut être comprise entre 0 et 100° C.

L'hydrolyse subséquente éventuelle peut être effectuée par tout moyen connu de l'homme du métier pour éliminer un radical alcoylcarbonyle, alcoyloxycarbonyle, benzoyle éventuellement substitué par un ou deux substituants choisis parmi les atomes d'halogène et les radicaux alcoyle, alcoyloxy, alcoylthio, amino, alcoylamino, dialcoylamino, nitro ou trifluorométhyle ou alcoylaminocarbonyle servant de blocage à une fonction alcool. On peut ainsi opérer dans un solvant tel qu'un alcool en présence d'une base comme la soude ou un carbonate alcalin.

On entend par précurseur d'amine tel qu'employé dans le présent procédé, un produit tel que l'azidure de sodium qui, après condensation sur le produit de formule générale (XXIX), peut conduire à l'amine cherchée par une opération subséquente connue de l'homme du métier; dans le cas de l'azidure de sodium, le produit de condensation de celui-ci et du produit de formule générale (XXIX) est traité par le propanedithiol-1,3 en présence d'un accepteur d'acide tel que la triéthylamine.

Les produits de formule générale (XXIX) définis comme précédemment en a) peuvent être obtenus par action d'un chloroformiate d'alcoyle sur un produit de formule générale:

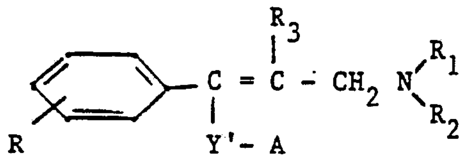

$(I_A)$

dans laquelle R, $R_1$, $R_2$, $R_3$ et A sont définis comme précédemment et Y' représente un atome de soufre ou un radical de formule générale (II) dans laquelle $R_6$ et $R_7$ sont définis comme précédemment et qui se présente sous la configuration E ou Z.

On opère généralement dans un solvant organique inerte tel qu'un carbure aromatique comme le benzène ou le toluène ou un solvant chloré comme le tétrachlorure de carbone, à une température comprise entre 60° C et la température de reflux du mélange réactionnel.

Les produits de formule générale (XXIX) définis comme précédemment en b) peuvent être obtenus par action du bromure de cyanogène sur un produit de formule générale ($I_A$) dans laquelle R, $R_1$ $R_2$, $R_3$ et A ont les définitions correspondantes et Y' représente un atome de soufre et qui se présente sous la configuration E ou Z.

On opère généralement dans un solvant chloré tel que le chlorure de méthylène ou le chloroforme, à une température comprise entre 0 et 60° C.

Les produits de formule générale ($I_A$), qui sont des produits selon l'invention, peuvent être préparés par l'une ou l'autre des méthodes exposées précédemment.

Selon l'invention, les produits de formule générale (I) dans laquelle Y représente un radical de formule générale (11) dans laquelle $R_7$ représente un radical alcoylaminocarbonyle et les autres symboles sont définis comme précédemment peuvent être obtenus par action d'un isocyanate de formule générale:

$$0 = C = N - R_7'' \qquad (XXX)$$

dans laquelle $R''_7$ représente un radical alcoyle sur un produit de formule générale (I) dans laquelle Y représente un radical de formule générale (II) dans laquelle $R_7$ représente un atome d'hydrogène et les autres symboles sont définis comme précédemment, c'est-à-dire un produit de formule générale (XXVIII).

On opère par toute méthode connue de l'homme du métier pour préparer un carbamate à partir d'un isocyanate et-d'un alcool, par exemple en opérant dans un solvant chloré tel que le chloroforme à une température comprise entre 0° C et la température de reflux du mélange réactionnel.

Il est entendu pour l'homme du métier que, lorsque $R_1$ et/ou $R_2$ représentent un atome d'hydrogène, il est nécessaire de bloquer la fonction amine correspondante avant de faire réagir le produit de formule générale (XXX) sur le produit de formule générale (XXVIII). La fonction amine est ensuite libérée après mise en oeuvre du procédé. Le blocage de l'amine peut se faire par exemple sous forme de radical tert-butyloxycarbonyle facilement clivable selon les méthodes connues sans toucher au radical $R_7$ introduit dans la molécule grâce au présent procédé.

Selon l'invention, les produits de formule générale (I) dans laquelle Y représente un radical sulfinyle ou sulfonyle et les autres symboles sont définis comme précédemment peuvent être préparés par oxydation d'un produit de formule générale (I) dans laquelle Y représente un atome de soufre et les autres symboles sont définis comme précédemment, c'est-à-dire un produit de formule générale:

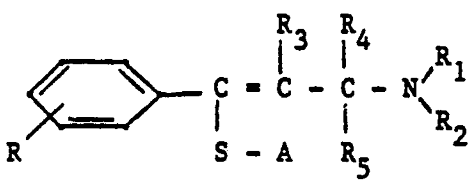

(XXXI)

L'oxydation peut être réalisée en employant un agent couramment utilisé pour passer d'un sulfure à un sulfoxyde ou à une sulfone, en opérant dans un solvant approprié, sans toucher au reste de la molécule. Par exemple, on peut employer l'eau oxygénée dans l'acétone ou l'acide acétique, un periodate alcalin dans un alcool ou l'acétonitrile, un peroxyacide carboxylique (acide peracétique, perbenzoïque, m-chloroperbenzoïque, p-nitroperbenzoïque ou perphtalique) dans un éther (dioxanne, tétrahydrofuranne), ou un solvant chloré (chlorure de méthylène, dichlcroéthane), l'acide acétique ou un mélange de ces solvants.

Lorsqu'on désire obtenir le sulfoxyde, c'est-à-dire le produit de formule générale (I) dans laquelle Y représente un radical sulfinyle, il est particulièrement avantageux d'opérer dans le chlorure de méthylène en présence de deux équivalents d'agent oxydant (de préférence l'acide m-chloroperbenzoique) à une température voisine de 0° C, de préférence en présence d'un équivalent d'acide minéral.

Lorsqu'on désire obtenir la sulfone, c'est-à-dire le produit de formule générale (I) dans laquelle Y représente un radical sulfonyle, il est particulièrement avantageux d'opérer en présence d'au moins 3 équivalents d'agent oxydant à une température comprise entre 20° C et la température de reflux du mélange réactionnel.

Il est entendu pour l'homme du métier qu'à chaque fois qu'un des procédés exposés précédemment met en oeuvre un dérivé organométallique et des molécules de produits réagissants contenant une fonction aminée, cette dernière doit être protégée au préalable. Pour cela, on opère selon les méthodes connues de l'homme du métier: On peut par exemple protéger la fonction aminée au moyen d'un dérivé silylé labile que l'on élimine facilement en fin de réaction.

Les nouveaux produits de formule générale (I) peuvent être purifiés par les méthodes habituelles telles que cristallisation, chromatographie ou extractions successives en milieu acide et basique.

Lorsque les produits de formule générale (I) ou leurs intermédiaires qui sont mentionnés dans la présente description existent sous formes E et Z en mélange dans le milieu réactionnel, leur séparation peut se faire par tout moyen connu de l'homme du métier, notamment par chromatographie.

Les nouveaux produits de formule générale (I) peuvent être transformés éventuellement en sels d'addition avec les acides par réaction avec un acide dans un solvant organique tel qu'un alcool, une cétone, un ester ou un solvant chloré; le sel formé précipite, éventuellement après concentration de sa solution; il est séparé par filtration ou décantation.

Les nouveaux produits de formule générale (I) et leurs sels présentent d'intéressantes propriétés pharmacologiques les rendant utiles comme antidépresseurs.

Ils se sont montrés actifs en particulier dans le test de l'action antagoniste vis-à-vis de la dépression induite par la tétrabénazine chez la souris à des doses comprises entre 1 et 100 mg/kg par voie orale.

Leur dose létale $DL_{50}$ est généralement comprise entre 50 et 900 mg/kg par voie orale.

Sont particulièrement importants les produits de formule générale (1) dans laquelle R représente un atome d'hydrogène ou d'halogène ou un radical alcoyle, alcoyloxy, alcoylthio, amino, alcoylamino, dialcoylamino ou trifluorométhyle, $R_3$ représente un atome d'hydrogène ou un radical alcoyle,

- soit $R_4$ et $R_5$ représentent un atome d'hydrogène et $R_1$ et $R_2$, identiques ou différents, représentent un atome d'hydrogène ou un radical alcoyle éventuellement substitué par un radical alcényle contenant 2 à 4 atomes de carbone, ou bien $R_1$ et $R_2$ forment ensemble avec l'atome d'azote auquel ils sont liés un radical hétérocyclique saturé contenant 4 à 7 chaînons (de préférence 5 ou 6 chaînons),

- soit $R_4$ représente un atome d'hydrogène, $R_1$ représente un atome d'hydrogène ou un radical alcoyle et $R_2$ et $R_5$ forment ensemble un radical alcoylène contenant 3 ou 4 atomes de carbone (de préférence 3 atomes de carbone),

et i) ou bien A représente un radical alcoyle ou un radical phényle non substitué ou substitué par un ou deux substituants choisis parmi les atomes d'halogène et les radicaux alcoyle, alcoyloxy, ou trifluorométhyle ou bien représente un radical pyridyle, benzyle ou cycloalcoyle contenant 3 à 6 atomes de carbone, Y représente un atome de soufre ou un radical sulfinyle ou encore un radical de formule générale:

$$R_6 - \overset{|}{\underset{|}{C}} - OR_7 \qquad (II)$$

dans laquelle $R_6$ représente un atome d'hydrogène ou un radical alcoyle et $R_7$ représente un atome d'hydrogène ou un radical alcoylcarbonyle, alcoyloxycarbonyle, benzoyle ou alcoylaminocarbonyle,

ii) ou bien Y et A forment ensemble un radical hydroxy-1 cycloalcoyle dont le cycle contient 5 ou 6 atomes de carbone éventuellement accolé à un cycle benzénique, se présentant sous la forme Z ou E, étant entendu que dans les définitions qui précèdent, les radicaux alcoyle et portions alcoyle contiennent 1 à 4 atomes de carbone en chaîne droite ou ramifiée.

Sont plus particulièrement importants, les produits de formule générale (I) dans laquelle R représente un atome d'hydrogène ou d'halogène ou un radical alcoyle, alcoyloxy, alcoylthio, amino, alcoylamino, dialcoylamino ou trifluorométhyle, $R_3$ représente un atome d'hydrogène, $R_4$ et $R_5$ représentent un atome d'hydrogène et $R_1$ et $R_2$, identiques ou différents, représentent un atome d'hydrogène ou un radical alcoyle éventuellement substitué par un radical alcényle contenant 2 à 4 atomes de carbone, et A représente un radical alcoyle ou un radical phényle non substitué ou substitué par un ou deux substituants choisis parmi les atomes d'halogène et les radicaux alcoyle, ou bien représente un radical pyridyle, cycloalcoyle contenant 3 à 6 atomes de carbone, Y représente un atome de soufre, ou encore un radical de formule générale:

$$R_6 - \overset{|}{\underset{|}{C}} - OR_7 \qquad (II)$$

dans laquelle $R_6$ représente un atome d'hydrogène et $R_7$ représente un atome d'hydrogène ou un radical alcoylcarbonyle, se présentant sous la forme Z,

étant entendu que dans les définitions qui précèdent les radicaux alcoyle et portions alcoyle contiennent 1 à 4 atomes de carbone en chaîne droite ou ramifiée.

Sont d'un intérêt tout particulier, les produits suivants:

- Diméthylamino-4 diphényl-1,2 butène-2 ol-1 (Z)
- Méthylamino-4 diphényl-1,2 butène-2 ol-1 (Z)
- (Fluoro-3 phényl)-1 diméthylamino-4 phényl-2 butène-2 ol-1 (Z)
- (Chloro-3 phényl)-1 diméthylamino-4 phényl-2 butène-2 ol-1 (Z)
- (Bromo-3 phényl)-1 diméthylamino-4 phényl-2 butène-2 ol-1 (Z)
- Diméthylamino-4 (méthyl-2 phényl)-1 phényl-2 butène-2 ol-1 (Z)
- Amino-4 diphényl-1,2 butène-2 ol-1 (Z)
- (Chloro-2 phénylthio)-3 diméthylamino-1 phényl-3 propène-2 (Z)
- (Chloro-3 phényl)-2 (fluoro-3 phényl)-1 diméthylamino-4 butène-2 ol-1 (Z)
- (Chloro-3 phényl)-2 diméthylamino-4 phényl-1 butène-2 ol-1 (Z)
- (Fluoro-3 phényl)-1 méthylamino-4 phényl-2 butène-2 ol-1 (Z)
- Amino-3 phényl-1 phénylthio-1 propène-1 (Z)
- (Chloro-4 phényl)-1 diméthylamino-4 phényl-2 butène-2 ol-1 (Z)
- (Bromo-4 phényl)-1 diméthylamino-4 phényl-2 butène-2 ol-1 (Z)
- Acétoxy-4 diméthylamino-1 diphényl-3,4 butène-2 (Z)
- Allylamino-4 diphényl-1,2 butène-2 ol-1 (Z)
- (Chloro-2 phényl)-1 diméthylamino-4 phényl-2 butène-2 ol-1 (Z)
- Diméthylamino-1 (fluoro-4 phénylthio)-3 phényl-3 propène-2 (Z)
- (Fluoro-2 phényl)-1 phényl-2 diméthylamino-4 butène-2 ol-1 (Z)
- Diméthylamino-1 méthyl-5 phényl-3 hexène-2 ol-4 (Z)
- Méthylamino-1 phényl-3-phénylthio-3 propène-2 (Z)
- Cyclohexyl-1 diméthylamino-4 phényl-2 butène-2 ol-1 (Z)
- Diméthylamino-4 phényl-2 (pyridyl-2)-1 butène-2 ol-1 (Z)
- Diméthylamino-4 phényl-2 (fluoro-4 phényl)-1 butène-2 ol-1 (Z)
- (Chloro-2 fluoro-6 phényl)-1 diméthylamino-4 phényl-2 butène-2 ol-1 (Z).

On connaît par le brevet belge 781 105 des propène-2 amines ayant des propriétés antidépressives; toutefois aucun des produits divulgués ne présente dans sa molécule un radical Y tel que défini dans la présente invention et rien dans l'art antérieur n'incitait l'homme du métier à introduire un tel radical.

Pour l'emploi médicinal, il peut être fait usage des nouveaux produits de formule générale (I) tels quels ou, le cas échéant, à l'état de sels pharmaceutiquement acceptables, c'est-à-dire non toxiques aux doses d'utilisation.

Comme exemples de sels pharmaceutiquement acceptables peuvent être cités les sels d'addition avec les acides minéraux (tels que chlorhydrates, sulfates, nitrates, phosphates) ou organiques (tels que acétates, propionates, succinates, benzoates, fumarates, maléates, méthanesulfonates, iséthionates, théophyllineacétates, salicylates, phénolphtalinates, méthylène-bis-β-oxy naphtoates) ou des dérivés de substitution de ces composés.

Les exemples suivants, donnés à titre non limitatif, montrent comment l'invention peut être mise en pratique. Dans certains exemples, les produits sont purifiés par chromatographie "flash"; on désigne sous ce nom une technique de purification caractérisée en ce qu'on utilise une colonne de chromatographie courte et qu'on opère sous une pression moyenne (50 kPa) en utilisant une silice de granulométrie 40 - 63 μm, selon W.C. STILL, M. KAHN et A. MITRA, J. Org. Chem., 43, 2923, (1978).

Dans les spectres de R.M.N. qui sont donnés, les lettres minuscules ont les significations suivantes: s = singulet, d = doublet, t = triplet, m = multiplet.

**EXEMPLE 1**

A une solution de 24 g de tris-isopropyl-2,4,6 benzène sulfonylhydrazone de l'acétophénone dans 1000 $cm^3$ de diméthoxy-1,2 éthane, maintenue sous une atmosphère d'azote on ajoute, à une température voisine de -75°C, 83 $cm^3$ d'une solution 1,5 M de n-butyllithium dans l'hexane. A la solution orangée obtenue on ajoute 11,1 g d'iodure de N,N-diméthylméthylèneammmonium; la suspension obtenue est agitée violemment pendant 20 minutes à une température voisine de -50°C; on ajoute alors; une température voisine de -75°C, 41,5 $cm^3$ d'une solution 1,5 M de n-butyllithium dans l'hexane. Le mélange réactionnel est réchauffé lentement jusqu'à une température voisine de 0°C. A partir de -30°C, on observe un dégagement gazeux qui devient pratiquement nul lorsque la température est voisine de 0°C. Le mélange réactionnel est alors refroidi à une température voisine de -30°C. On y ajoute alors 7,7 g de benzaldéhyde en solution dans 200 $cm^3$ de diméthoxy-1,2 éthane et poursuit l'agitation pendant 2 heures à une température voisine de 20°C. On ajoute alors successivement 250 $cm^3$ d'eau distillée, 50 $cm^3$ d'acide chlorhydrique concentré et 300 $cm^3$ d'éther éthylique. La phase aqueuse est séparée par décantation; la phase organique est extraite par 125 $cm^3$ d'une solution aqueuse 1N d'acide chlorhydrique. Les phases aqueuses sont réunies, alcalinisées par une solution aqueuse 10N de soude jusqu'à un pH voisin de 11 puis extraites par 3 fois 200 $cm^3$ d'acétate d'éthyle. Les phases organiques sont réunies, séchées sur du sulfate de magnésium, filtrées puis concentrées à sec sous pression réduite (2,7 kPa) à 40°C; le résidu obtenu est dissous dans un mélange de 140 $cm^3$ d'éther éthylique et de 70

$cm^3$ d'éthanol; on ajoute à la solution obtenue 20 $cm^3$ d'une solution 3,5N d'acide chlorhydrique gazeux dans l'éther éthylique. Le solide formé est séparé par filtration puis recristallisé dans l'acétonitrile. On obtient ainsi 10 g de chlorhydrate de diméthylamino-4 diphényl-1,2 butène-2 ol-1 (Z), sous forme d'une poudre blanche fondant à 178° C.

La tris-isopropyl-2,4,6 benzènesulfonylhydrazone de l'acétophénone peut être préparée par la méthode décrite par M.F. LIPTON et R.H. SHAPIRO, J. Org. Chem., 43, 1409, (1978).

**EXEMPLE 2**

En opérant d'une manière analogue à celle décrite à l'exemple 1, mais à partir de 36,1 g de tris-isopropyl-2,4,6 benzènesulfonylhydrazone de l'acétophénone, de 18,5 g d'iodure de N,N-diméthylméthylèneammonium et de 11,2 g de cyclohexanecarboxaldéhyde, on obtient, après recristallisation dans l'acétonitrile, 7,5 g de chlorhydrate de cyclohexyl-1 diméthylamino-4 phényl-2 butène-2 ol-1 (Z), sous forme d'une poudre blanche fondant à 222° C.

**EXEMPLE 3**

En opérant d'une manière analogue à celle décrite à l'exemple 1, mais à partir de 12 g de tris-isopropyl-2,4,6 benzènesulfonylhydrazone de l'acétophénone, de 6,1 g d'iodure de N,N-diméthylméthylèneammonium et de 4,7 g de chloro-4 benzaldéhyde; on obtient, après recristallisation dans l'acétone, 3 g de chlorhydrate de (chloro-4 phényl)-1 diméthylamino-4 phényl-2 butène-2 ol-1 (Z), sous forme d'une poudre blanche fondant à 170° C.

**EXEMPLE 4**

En opérant d'une manière analogue à celle décrite à l'exemple 1, mais à partir de 12 g de tris-isopropyl-2,4,6 benzènesulfonylhydrazone de l'acétone, de 6,1 g d'iodure de N,N-diméthylméthylèneammonium et de 4 g d'acétophénone, on obtient, après recristallisation dans l'acétone, 2,5 g de chlorhydrate de diméthylamino-4 diphényl-1,2 méthyl-1 butène-2 ol-1 (Z), sous forme d'une poudre blanche fondant à 160° C.

**EXEMPLE 5**

En opérant d'une manière analogue à celle décrite à l'exemple 1, mais à partir de 12 g de tris-isopropyl-2,4,6 benzènesulfonylhydrazone de l'acétophénone, de 6,1 g d'iodure de N,N-diméthylméthylèneammonium et de 6,1 g de bromo-4 benzaldéhyde, on obtient, après recristallisation dans l'acétonitrile, 2,1 g de chlorhydrate de (bromo-4 phényl)-1 diméthylamino-4 phényl-2 butène-2 ol-1 (Z), sous forme d'une poudre blanche fondant à 163° C.

**EXEMPLE 6**

En opérant d'une manière analogue à celle décrite à l'exemple 1, mais à partir de 12 g de tris-isopropyl-2,4,6 benzènesulfonylhydrazone de l'acétophénone, de 6,1 g de N,N-diméthylméthylèneammonium et de 3,9 g de phénylacétaldéhyde, on obtient, après recristallisation dans l'éthanol, 2,3 g de chlorhydrate de diméthylamino-5 diphényl-1,3 pentène-3 ol-2 (Z), sous forme d'une poudre blanche fondant à 220° C.

**EXEMPLE 7**

En opérant d'une manière analogue à celle décrite à l'exemple 1, mais à partir de 12 g de tris-isopropyl-2,4,6 benzènesulfonylhydrazone de l'acétophénone, de 6,1 g d'iodure de N,N-diméthylméthylèneammonium et de 5,7 g de trifluorométhyl-3 benzaldéhyde, on obtient, après recristallisation dans l'acétone, 2,1 g de chlorhydrate de diméthylamino-4 phényl-2 (trifluorométhyl-3-phényl)-1 butène-2 ol-1 (Z), sous forme de cristaux blancs fondant à 180° C.

**EXEMPLE 8**

En opérant d'une manière analogue à celle décrite à l'exemple 1, mais à partir de 12,1 g de tris-isopropyl-2,4,6 benzènesulfonylhydrazone de la méthyl-4 acétophénone, de 6,1 g d'iodure de N,N-diméthylméthylèneammonium et de 3,5 g de benzaldéhyde, on obtient, après recristallisation dans l'éthanol, 2,3 g de chlorhydrate de diméthylamino-4 (méthyl-4 phényl)-2 phényl-1 butène-2 ol-1 (Z), sous forme de cristaux blancs fondant à 185° C.

La tris-isopropyl-2,4,6 benzènesulfonylhydrazone de la méthyl-4 acétophénone peut être préparée de la manière suivante: on ajoute 15,4 g de méthyl-4 acétophénone à une solution de 29,8 g de tris-isopropyl-2,4,6 benzènesulfonylhydrazine dans 60 $cm^3$ de méthanol contenant 37 $cm^3$ d'une solution 3N d'acide chlorhydrique gazeux dans l'éther éthylique. Le mélange réactionnel est conservé pendant 18 heures à une température voisine de 5° C, puis filtré. Les cristaux obtenus sont ajoutés à un mélange de 300 $cm^3$ de chlorure de méthylène et d'une solution aqueuse saturée de bicarbonate de sodium. La phase organique est séparée par décantation, séchée sur du sulfate de magnésium, filtrée puis concentrée à sec sous pression réduite (2,7 kPa) à 40° C. On obtient, après recristallisation dans l'éthanol, 21 g de tris-isopropyl-2,4,6 benzènesulfonylhydrazone de la méthyl-4 acétophénone, sous forme de cristaux blancs fondant à 202° C.

**EXEMPLE 9**

A une solution de 36,1 g de tris-isopropyl-2,4,6 benzènesulfonylhydrazone de l'acétophénone dans 1000 $cm^3$ de diméthoxy-1,2 éthane maintenue sous atmosphère d'azote à une température voisine de -75° C, on ajoute 134 $cm^3$ d'une solution 1,5 M de n-butyllithium dans l'hexane. A la solution orangée obtenue, on ajoute 18,5 g d'iodure de N,N-diméthylméthylèneammonium; la suspension obtenue est agitée violemment pendant 20 minutes à une température voisine de -50° C. On ajoute ensuite à une température voisine de -75° C, 67 $cm^3$ d'une solution 1,5 M de n-butyllithium dans l'hexane et on laisse ensuite la température remonter lentement au voisinage de 0° C.

Le mélange réactionnel est alors refroidi à une température voisine de -30° C; on ajoute alors 10,7 g de pyridine-3 carboxaldéhyde en solution dans 50 $cm^3$ de diméthoxy-1,2 éthane et laisse agiter pendant 2 heures à une température voisine de 20° C. On ajoute un mélange de 500 $cm^3$ d'éther éthylique, de 100 $cm^3$ d'une solution aqueuse d'acide chlorhydrique concentré et de 400 $cm^3$ d'eau distillée. La phase aqueuse est séparée par décantation; la phase organique est extraite par 50 $cm^3$ d'une solution aqueuse IN d'acide chlorhydrique. Les phases aqueuses sont réunies, alcalinisées par une solution aqueuse 10N de soude jusqu'à un pH voisin de 11 et extraites par 3 fois 100 $cm^3$ d'acétate d'éthyle. Les phases organiques sont réunies, séchées sur du sulfate de magnésium, filtrées et concentrées à sec sous pression réduite (2,7 kPa) à 40° C. Le résidu obtenu est dissous dans 100 $cm^3$ d'éthanol. A la solution obtenue on ajoute 7,7 g d'acide oxalique; les cristaux apparus sont séparés par filtration.

Après recristallisation dans le méthanol, on obtient 14,8 g de sesquioxalate de diméthylamino-4 phényl-2 (pyridyl-3)-1 butène-2 ol-1 (Z), sous forme d'une poudre blanche fondant à 195° C.

**EXEMPLE 10**

En opérant d'une manière analogue à celle décrite à l'exemple 9, mais à partir de 12 g de tris-isopropyl-2,4,6 benzènesulfonylhydrazone de l'acétophénone, de 6,2 g d'iodure de N,N-diméthylméthylèneammonium et de 3,6 g de pyridyl-2 carboxaldéhyde, on obtient, après recristallisation dans l'éthanol, 4,4 g de sesquioxalate de diméthylamino-4 phényl-2 (pyridyl-2)-1 butène-2 ol-1 (Z), sous forme de cristaux blancs fondant à 152° C.

**EXEMPLE 11**

En opérant d'une manière analogue à celle décrite à l'exemple 9 mais à partir de 12 g de tris-isopropyl-2,4,6 benzènesulfonylhydrazone de l'acétophénone, de 6,1 g d'iodure de N,N-diméthylméthylèneammonium et de 3,6 g de pyridine-4 carboxaldéhyde, on obtient, après recristallisation dans l'acétone, 2,5 g d'oxalate de diméthylamino-4 phényl-2 (pyridyl-4)-1 butène-2 ol-1 (Z) (2,5 moles d'acide oxalique par mole de produit), sous forme de cristaux jaunes fondant à 158° C.

**EXEMPLE 12**

En opérant d'une manière analogue à celle décrite à l'exemple 9, mais à partir de 12 g de tris-isopropyl-2,4,6 benzènesulfonylhydrazone de l'acétophénone, de 6,1 g d'iodure de N,N-diméthylméthylèneammonium et de 4,5 g de méthoxy-4 benzaldéhyde, on obtient, après recristallisation dans l'acétone, 3,5 g d'oxalate acide de diméthylamino-4 (méthoxy-4 phényl)-1 phényl-2 butène-2 ol-1 (Z), sous forme de cristaux blancs fondant à 110°C.

**EXEMPLE 13**

En opérant d'une manière analogue à celle décrite à l'exemple 9, mais à partir de 12,9 g de tris-isopropyl-2,4,6 benzènesulfonylhydrazone de la méthoxy-4 acétophénone, de 6,1 g d'iodure de N,N-diméthylméthylèneammonium et de 3,5 g de benzaldéhyde, on obtient, après recristallisation dans l'acétone, 1,6 g d'oxalate acide de diméthylamino-4 (méthoxy-4 phényl)-2 phényl-1 butène-2 ol-1 (Z), sous forme de cristaux blancs fondant à 98°C.

La tris-isopropyl-2,4,6 benzènesulfonylhydrazone de la méthoxy-4 acétophénone peut être préparée d'une manière analogue à celle décrite à l'exemple 8 pour la préparation de la tris-isoprol-pyl-2,4,6 benzènesulfonylhydrazone de la méthyl-4 acétophénone. A partir de 29,8 g de tris-isopropyl-2,4,6 benzènesulfonylhydrazine et de 15 g de méthoxy-4 acétophénone, on obtient, après recristallisation dans l'éthanol, 28 g de tris-isopropyl-2,4,6 benzènesulfonylhydrazone de la méthoxy-4 acétophénone, sous forme de cristaux blancs fondant à 210°C.

**EXEMPLE 14**

En opérant d'une manière analogue à celle décrite à l'exemple 9, mais à partir de 12 g de tris-isopropyl-2,4,6 benzènesulfonylhydrazone de l'acétophénone, de 6,1 g d'iodure de N,N-diméthylméthylèneammonium et de 4,1 g de fluoro-4 benzaldéhyde, on obtient, après recristallisation dans l'acétone, 2 g d'oxalate acide de diméthylamino-4 (fluoro-4 phényl)-1 phényl-2 butène-2 ol-1 (Z), sous forme de cristaux blancs fondant à 125°C.

**EXEMPLE 15**

En opérant d'une manière analogue à celle décrite à l'exemple 9, mais à partir de 12 g de tris-isopropyl-2,4,6 benzènesulfonylhydrazone de l'acétophénone, de 6,1 g d'iodure de N,N-diméthylméthylèneammonium et de 5,5 g de chloro-2 fluoro-6 benzaldéhyde, on obtient, après recristallisation dans l'acétone, 2,2 g d'oxalate acide de (chloro-2 fluoro-6 phényl)-1 diméthylamino-4 phényl-2 butène-2 ol-1 (Z), sous forme de cristaux blancs fondant à 88°C.

**EXEMPLE 16**

A une solution de 2,3 g de bromo-3 phényl-3 diméthylamino-1 propène-2 (Z) dans 40 $cm^3$ d'éther éthylique maintenue sous atmosphère d'azote à une température voisine de -78°C, on ajoute une solution de 10 $cm^3$ d'éther éthylique et de 6,4 $cm^3$ d'une solution de n-butyllithium 1,5N dans l'hexane et laisse agiter pendant 15 minutes. A la solution brune obtenue, on ajoute 1 $cm^3$ de benzaldéhyde en solution dans 10 $cm^3$ de diméthoxy-1,2 éthane et poursuit l'agitation pendant 2 heures à une température voisine de -10°C. Le mélange réactionnel est ensuite versé dans 50 $cm^3$ d'eau distillée, ajustée à pH 2 avec une solution aqueuse d'acide chlorhydrique concentré. La phase aqueuse est séparée par décantation, alcalinisée à pH 10 par addition d'une solution aqueuse de soude 10N puis extraite par 2 fois 200 $cm^3$ d'éther éthylique. Les phases organiques sont séchées sur du sulfate de sodium, filtrées puis concentrées à sec sous pression réduite (2,7 kPa) à 40°C. L'huile jaune clair obtenue est purifiée par chromatographie "flash" [éluant: acétate d'éthyleméthanol (50 - 50 en volumes)]. Après évaporation à sec sous pression réduite (2,7 kPa) à 40°C des fractions 38 à 53, on obtient un résidu que l'on dissout dans 1 $cm^3$ d'éthanol et 0,3 $cm^3$ d'éther éthylique. A la solution obtenue, on ajoute 0,7 $cm^3$ d'une solution 3,5N d'acide chlorhydrique gazeux dans l'éther éthylique. Un solide précipite; il est séparé par filtration. On obtient ainsi 0,3 g de chlorhydrate de diméthylamino-4 diphényl-1,2 butène-2 ol-1 (Z), sous forme d'une poudre blanche fondant à 172 - 174°C.

Le bromo-3 phényl-3 diméthylamino-1 propène-2 (Z) peut être préparé de la manière suivante: A une solution de 33,1 g de diméthylaminoacrylophénone dans 120 $cm^3$ de chlorure de méthylène, on ajoute à une température voisine de 10°C une solution de 54,2 g d'oxybromure de phosphore dans 45 $cm^3$ de chlorure de

méthylène et laisse agiter pendant 45 minutes à une température voisine de 10°C, puis 30 minutes à température ambiante. On ajoute alors à une température voisine de 0°C, 150 $cm^3$ de méthanol. Après 10 minutes, on ajoute 7,15 g de cyanoborohydrure de sodium et l'on poursuit l'agitation pendant 3 heures à température ambiante. Le mélange réactionnel est évaporé sec sous pression réduite (2,7 kPa) à 30°C: on obtient ainsi une huile orangée que l'on reprend au chlorure de méthylène; la solution est filtrée puis versée dans 200 $cm^3$ d'eau glacée. Après alcalinisation à pH 10 par addition d'une solution aqueuse de soude 10N, la phase organique est décantée, séchée sur sulfate de sodium, filtrée et évaporée à sec sous pression réduite (2,7 kPa) à 30°C. On obtient une huile rouge que l'on dissout dans un mélange de 50 $cm^3$ d'éthanol et de 75 $cm^3$ d'éther éthylique; à la solution obtenue, on ajoute 60 $cm^3$ d'une solution 3,5N d'acide chlorhydrique gazeux dans l'éther éthylique. Le solide formé est séparé par filtration puis recristallisé dans 720 $cm^3$ d'acétone. On obtient ainsi 15,3 g de bromo-3 phényl-3 diméthylamino-1 propène-2 (Z), sous forme d'une poudre blanche fondant à 174°C.

La diméthylaminoacrylophénone peut être préparée par la méthode décrite par H. MEERWEIN, W. FLORIAN, N. SCHOR, G. STOPP Ann. Chem. 641, 1, (1961).

## EXEMPLE 17

En opérant d'une manière analogue à celle décrite à l'exemple 16, mais à partir de 21,4 g de bromo-3 (chloro-4 phényl)-3 diméthylamino-1 propène-2 (Z), de 56 $cm^3$ d'une solution de n-butyllithium 1,5N dans l'hexane, de 9,8 $cm^3$ de benzaldéhyde et après purification par chromatographie "flash" [éluant: chlorure de méthylène - méthanol (95 - 5 en volumes)] et concentration à sec des fractions 30 à 54 sous pression réduite (2,7 kPa) à 40°C, on obtient un résidu que l'on dissout dans 10 $cm^3$ d'éthanol. A la solution obtenue, on ajoute 5 $cm^3$ d'une solution 3,5N d'acide chlorhydrique gazeux dans l'éther diéthylique. Le solide qui précipite est séparé par filtration puis recristallisé dans l'éthanol. On obtient ainsi 3 g de chlorhydrate de (chloro-4 phényl)-2 diméthylamino-4 phényl-1 butène-2 ol-1 (Z), sous forme d'une poudre blanche fondant à 172°C.

Le bromo-3 (chloro-4 phényl)-3 diméthylamino-1 propène-2 (Z) peut être préparé d'une manière analogue à celle décrite à l'exemple 16, mais à partir du (chloro-4 phényl)-1 diméthylamino-3 butène-2 one-1, de 61,3 g d'oxybromure de phosphore et de 8,10 g de cyanoborohydrure de sodium. L'évaporation à sec sous pression réduite (2,7 kPa) à 30°C du mélange réactionnel conduit à un solide jaune qui est repris au chloroforme. La solution est filtrée et le filtrat est évaporé sous pression réduite (2,7 kPa) à 30°C. Le solide obtenu est lavé avec un mélange de méthanol et d'éther éthylique (75 - 25 en volumes). On obtient ainsi 45,9 g de bromhydrate de bromo-3 (chloro-4 phényl)-3 diméthylamino-1 propène-2 (Z), sous forme d'un solide blanc fondant à 174°C.

La (chloro-4 phényl)-1 diméthylamino-3 butène-2 one-1 peut être préparée par la méthode décrite par YANG-I-LIN and S.A. LANG Jr., J. Org. Chem., 45, 4857, (1980).

## EXEMPLE 18

En opérant d'une manière analogue à celle décrite à l'exemple 16, mais à partir de 10 g de bromo-3 (chloro-4 phényl)-3 diméthylamino-1 propène-2 (Z), de 26 $cm^3$ d'une solution de n-butyllithium 1,5N dans l'hexane, de 4,20 g de pyridine-3 carboxaldéhyde, on obtient une huile que l'on purifie par chromatographie "flash" [éluant: chlorure de méthylène - méthanol (90 - 10 en volumes)]. Après évaporation à sec sous pression réduite (2,7 kPa) à 40°C des fractions 19 à 50, on obtient un résidu que l'on dissout dans 25 $cm^3$ d'éthanol. A la solution obtenue, on ajoute 13 $cm^3$ d'une solution éthanolique 1,7 M d'acide oxalique; le précipité qui se forme est séparé par filtration puis recristallisé dans 36 $cm^3$ de méthanol et 4 $cm^3$ d'eau distillée. On obtient ainsi 3,5 g de sesquioxalate de (chloro-4 phényl)-2 diméthylamino-4 (pyridyl-3)-1 butène-2 -ol-1 (Z), sous forme de cristaux blancs fondant à 187°C.

## EXEMPLE 19

En opérant d'une manière analogue à celle décrite à l'exemple 16, mais à partir de 20 g de bromo-3 (fluoro-4 phényl)-3 diméthylamino-1 propène (Z), de 57 $cm^3$ de solution de n-butyllithium 1,5N dans l'hexane et de 10 $cm^3$ de benzaldéhyde, on obtient une huile que l'on purifie par chromatographie "flash" [éluant: chlorure de méthylène - méthanol (90 - 10 en volumes)]. Après évaporation à sec sous pression réduite (2,7 kPa) à 40°C des fractions 36 à 79, on obtient un résidu que l'on dissout dans 20 $cm^3$ d'acétonitrile et 60 $cm^3$ d'éther éthylique. A la solution obtenue, on ajoute 5,6 $cm^3$ d'une solution 3N d'acide chlorhydrique gazeux dans l'éther éthylique. Le précipité qui se forme est séparé par filtration puis recristallisé dans 15 $cm^3$ d'isopropanol. On obtient ainsi 2 g de chlorhydrate de diméthylamino-4 (fluoro-4 phényl)-2 phényl-1 butène-2-ol-1 (Z), sous forme de cristaux blancs fondant à 141°C.

Le bromo-3 (fluoro-4 phényl)-3 diméthylamino-1 propène-2 (Z) peut être préparé d'une manière analogue à

celle décrite à l'exemple 16, mais à partir de 26,5 g de (fluoro-4 phényl)-1 diméthylamino-3 butène-2 one-1, de 39,15 g d'oxybromure de phosphore et de 5,15 g de cyanoborohydrure de sodium. L'évaporation à sec sous pression réduite (2,7 kPa) à 30°C du mélange réactionnel conduit à un solide jaune qui est repris au chloroforme. Après filtration, la phase chloroformique est évaporée sous pression réduite (2,7 kPa) à 30°C pour conduire à un solide qui est cristallisé dans 50 $cm^3$ de méthanol et 25 $cm^3$ d'éther éthylique. On obtient ainsi 30 g de bromhydrate de bromo-3 (fluoro-4 phényl)-3 diméthylamino-1 propène-2 (Z), sous forme d'un solide blanc fondant vers 156°C.

La (fluoro-4 phényl)-1 diméthylamino-3 butène-2-one-1 peut être préparée par la méthode décrite par J.D. ALBRIGHT et Coll. Brevet U.S.-4 209 621.

**EXEMPLE 20**

En opérant d'une manière analogue à celle décrite à l'exemple 16, mais à partir de 13,6 g de bromo-3 phényl-3 (N-pipéridino)-1 propène-2 (Z), de 36 $cm^3$ d'une solution de n-butyllithium 1,5N dans l'hexane et de 6,5 $cm^3$ de benzaldéhyde, et après addition au mélange réactionnel à 0°C de 50 $cm^3$ d'une solution aqueuse 1N d'acide chlorhydrique, on obtient un précipité blanc. Après séparation du précipité par filtration et lavage à l'éther éthylique, le solide obtenu est repris au chlorure de méthylène. La phase organique est séchée sur sulfate de sodium, filtrée et évaporée à sec sous pression réduite (2,7 kPa) à 30°C pour donner un résidu que l'on recristallise dans 80 $cm^3$ d'isopropanol. On obtient ainsi 2,7 g de chlorhydrate de diphényl-1,2 (N-pipéridino)-4 butène-2-ol-1 (Z), sous forme de cristaux blancs fondant à 195 - 196°C.

Le bromo-3 phényl-3 (N-pipéridino)-1 propène-2 (Z) peut être préparé d'une manière analogue à celle décrite à l'exemple 16, mais à partir de 29,1 g de (N-pipéridino)-3 acrylophénone, de 38,8 g d'oxybromure de phosphore et de 5,1 g de cyanoborohydrure de sodium. L'évaporation à sec sous pression réduite (2,7 kPa) à 30°C du mélange réactionnel conduit à un solide jaune que l'on reprend au chloroforme. Après filtration, la phase chloroformique est concentrée sous pression réduite (2,7 kPa) à 30°C pour donner un solide jaune que l'on cristallise dans l'isopropanol. On obtient ainsi 26,5 g de bromhydrate de bromo-3 phényl-3 (N-pipéridino)-1 propène-2 (Z), sous forme d'un solide blanc fondant à 204 - 205°C.

La N-pipéridino-3 acrylophénone peut être préparée d'une manière analogue à celle décrite par E. BENARY, Ber, 63, 1573 (1930).

**EXEMPLE 21**

En opérant d'une manière analogue à celle décrite à l'exemple 16, mais à partir de 21 g de bromo-3 (bromo-4 phényl)-3 dimèthylamino-1 propène-2 (Z), de 48 $cm^3$ d'une solution de n-butyllithium 1,5N dans l'hexane et de 8,5 $cm^3$ de benzaldéhyde, on obtient une huile que l'on purifie par chromatographie "flash" [éluant: dichlorométhane - méthanol (93 - 7 en volumes)]. Après évaporation à sec sous pression réduite (2,7 kPa) à 40°C des fractions 25 à 39, on obtient un résidu que l'on dissout dans 40 $cm^3$ d'acétonitrile. A la solution obtenue, on ajoute 9,7 $cm^3$ d'une solution 3N d'acide chlorhydrique gazeux dans l'éther éthylique; le solide qui se forme ést séparé par filtration puis recristallisé dans 100 $cm^3$ d'acétonitrile. On obtient ainsi 5,2 g de chlorhydrate de (bromo-4 phényl)-2 diméthylamino-4 phényl-1 butène-2 ol-1 (Z), sous forme d'un solide blanc fondant à 178°C.

Le bromo-3 (bromo-4 phényl)-3 diméthylamino-1 propène-2 (Z) peut être préparé d'une manière analogue à celle décrite à l'exemple 16, mais à partir de 38 g de (bromo-4 phényl)-1 diméthylamino-3 propène-2-one-1, de 42,9 g d'oxybromure de phosphore et de 5,68 g de cyanoborohydrure de sodium. L'évaporation à sec sous pression réduite (2,7 kPa) à 30°C du mélange réactionnel conduit à un solide jaune que l'en cristallise dans le méthanol. On obtient ainsi 28 g de bromhydrate de bromo-3 (bromo-4 phényl)-3 diméthylamino-1 propène-2 (Z), sous forme d'un solide blanc fondant à 180°C.

La (bromo-4 phényl)-1 diméthylamino-3 propène-2-one-1 peut être préparée d'une manière analogue à celle décrite par YANG-I LIN and S.A. LANG Jr., J. Org. Chem., 45, 4857, (1980).

**EXEMPLE 22**

A une solution de 9,15 g de tris-isopropyl-2,4,6 benzènesulfonylhydrazone de la β-diméthylaminopropiophénone dans 100 $cm^3$ de diméthoxy-1,2 éthane maintenue sous atmosphère d'azote à une température voisine de -75°C, on ajoute 29 $cm^3$ d'une solution 1,5 M de n-butyllithium dans l'hexane. La solution orangée obtenue est agitée ensuite pendant 15 minutes à une température voisine de 0°C, puis refroidie à une température voisine de -40°C. On ajoute alors 2,2 $cm^3$ de benzaldéhyde, puis agite le mélange réactionnel pendant 1 heure à une température voisine de 20°C. On ajoute alors sous agitation 100 $cm^3$ d'une solution aqueuse 1N d'acide chlorhydrique puis 100 $cm^3$ d'éther éthylique. La phase aqueuse est séparée par

décantation; la phase organique est extraite par 25 $cm^3$ d'une solution aqueuse 0,1N d'acide chlorhydrique. Les phases aqueuses sont réunies, lavées par 2 fois 50 $cm^3$ d'éther, puis alcalinisées par de la lessive de soude jusqu'à un pH voisin de 10, et extraites par 3 fois 50 $cm^3$ d'acétate d'éthyle. Les phases organiques sont réunies, séchées sur du sulfate de magnésium, filtrées puis concentrées à sec sous pression réduite (2,7 kPa) à 40°C. On obtient ainsi 5,2 g de diméthylamino-4 diphényl-1,2 butène-2 ol-1 (Z), sous forme de cristaux blancs fondant à 75°C.

La tris-isopropyl-2,4,6 benzènesulfonylhydrazone de la β-diméthylaminopropiophénone peut être préparée de la manière suivante: A une suspension de tris-isopropyl-2,4,6 benzène sulfonylhydrazine dans un mélange de 130 $cm^3$ de méthanol et de 130 $cm^3$ d'une solution 3N d'acide chlorhydrique gazeux dans l'éther éthylique, on ajoute 77,3 g de chlorhydrate de β-diméthylaminopropiophénone, puis 33 $cm^3$ d'eau distillée et agite le mélange réactionnel à une température voisine de 20°C pendant 20 heures. Le mélange réactionnel est refroidi à une température voisine de + 5°C; les cristaux formés sont séparés par filtration puis ajoutés à un mélangé de 550 $cm^3$ de chlorure de méthylène, de 110 $cm^3$ d'une solution aqueuse 4N de soude et de 275 $cm^3$ d'eau distillée et le mélange est agité vigoureusement. La phase organique est ensuite séparée par décantation; la phase aqueuse est extraite par 3 fois 150 $cm^3$ de chlorure de méthylène. Les phases organiques sont réunies, séchées sur du sulfate de magnésium, filtrées puis concentrées à sec sous pression réduite (2,7 kPa) à 40°C. Le résidu obtenu est recristallisé dans l'isopropanol; On obtient ainsi 79 g de tris-isopropyl-2,4,6 benzènesulfonylhydrazone de la β-diméthylaminopropiophénone sous forme d'une poudre blanche fondant à 164°C.

**EXEMPLE 23**

En opérant d'une manière analogue à celle décrite à l'exemple 22, mais à partir de 30 g de tris-isopropyl-2,4,6 benzènesulfonylhydrazone de la β-diéthylaminopropiophénone et de 6,9 $cm^3$ de benzaldéhyde, on obtient, après recristallisation dans l'acétone, 3,2 g de chlorhydrate de diéthylamino-4 diphényl-1,2 butène-2 ol-1 (Z), sous forme de cristaux blancs fondant à 140°C.

La tris-isopropyl-2,4,6 benzènesulfonylhydrazone de la β-diéthylaminopropiophénone peut être préparée d'une manière analogue à celle décrite à l'exemple 22 pour la préparation de la tris-isopropyl-2,4,6 benzènesulfonylhydrazone de la β-diméthylaminopropiophénone mais à partir de 51,8 g de tris-isopropyl-2,4,6 benzènesulfonylhydrazine et de 64,4 g de chlorhydrate de β-diéthylaminopropiophénone. On obtient ainsi 53,6 g de tris-isopropyl-2,4,6 benzènesulfonylhydrazone de la β-diéthylaminopropiophénone sous forme de cristaux créme fondant à 134°C.

Le chlorhydrate de β-diéthylaminopropiophénone peut être préparé selon la méthode décrite par C.E. MAXWELL Org. Synt. Coll. Vol. III, John. WILEY & SONS, Londres (1955) page 305.

**EXEMPLE 24**

En opérant d'une manière analogue à celle décrite à l'exemple 22, mais à partir de 20 g de tris-isopropyl-2,4,6 benzène sulfonylhydrazone de la β-(pyrrolidinyl-1) propiophénone et de 4,6 $cm^3$ de benzaldéhyde, on obtient, après recristallisation dans un mélange de propanol-2 et d'éther isopropylique (40/60 en volumes), 9,9 g de chlorhydrate de diphényl-1,2 (pyrrolidinyl-1)-4 butène-2 ol-1 (Z), sous forme de crrstaux blancs fondant à 106°C.

La tris-isopropyl-2,4,6 benzènesulfonylhydrazone de la β-(pyrrolidinyl-1) propiophénone peut être préparée par une méthode analogue à celle décrite à l'exemple 22 pour la préparation de la tris-isopropyl-2,4,6 benzènesulfonylhydrazone de la β-diméthylaminopropiophénone mais à partir de 17,9 g de tris-isopropyl-2,4,6 benzènesulfonylhydrazine et de 14,4 g de chlorhydrate de β-(pyrrolidinyl-1) propiophénone. On obtient ainsi 20 g de tris-isopropyl-2,4,6 benzènesulfonylhydrazone de la β-(pyrrolidinyl-1) propiophénone sous forme de cristaux blancs fondant à 142°C.

Le chlorhydrate de β-(pyrrolidinyl-1) propiophénone peut être préparé d'une manière analogue à celle décrite par C.E. MAXWELL Org. Synt., Coll. Vol III, John WILEY & SONS, Londres (1955) page 305 mais à partir de 58,8 g de chlorhydrate de pyrrolidine, de 60,1 g d'acétophénone et de 22,5 g de polyoxyméthylène. On obtient ainsi 66 g de chlorbydrate de β-(pyrrolidinyl-1) propiophénone, sous forme de cristaux blancs fondant à 164°C.

**EXEMPLE 25**

En opèrant comme à l'exemple 22 mais à partir de 18,9 g de tri-isopropyl-2,4,6 benzènesulfonylhydrazone de la β-diméthylamino propiophénone, de 61 $cm^3$ d'une solution 1,5 M de n-butyllithium et de 6,4 g de chloro-3 benzaldéhyde, dans 190 $cm^3$ de diméthoxy-1,2 éthane et après recristallisation dans 125 $cm^3$ d'isopropanol, on obtient 8,9 g de chlorhydrate de (chloro-3 phényl)-1 diméthylamino-4-phényl-2 butène-2 ol-1 (Z) fondant à

185°C.

**EXEMPLE 26**

En opérant comme à l'exemple 2; mais à partir de 15 g de tris-isopropyl-2,4,6 benzénesulfonylhydrazone de β-diméthylamino propiophénole, de 48 $cm^3$ d'une solution 1,5 M de n-butyllithium dans l'hexane et de 4,5 g de fluoro-3 benzaldéhyde, dans 150 $cm^3$ de diméthoxy-1,2 éthane, et après recristallisation dans 215 $cm^3$ d'un mélange d'acétone et d'isopropanol (93-7 en volumes), on obtient 5,3 g de chlorhydrate de (fluoro-3 phényl)-1 diméthylamino-4 phènyl-2 butène-2 ol-1 (Z) fondant à 165°C.

**EXEMPLE 27**

En opérant comme à l'exemple 22 mais à partir de 20 g de tris-isopropyl-2,4,6 benzènesulfonylhydrazone de la β-diméthylamino propiophénone, de 64 $cm^3$ d'une solution 1,5 M de n-butyllithium dans l'hexane et de 6,8 g de chloro-2 benzaldéhyde, dans 200 $cm^3$ de diméthoxy-1,2 éthane, et après recristallisation du produit brut à l'état de base dans 62 $cm^3$ d'un mélange d'oxyde d'isopropyle et d'éther de pétrole (50 - 50 en volumes), on obtient 5,2 g de (chloro-2 phényl)-1 diméthylamino-4 phényl-2 butène-2 ol-1 (Z) fondant à 90°C.

**EXEMPLE 28.**

En opérant comme à l'exemple 22 mais à partir de 20 g de tris-isopropyl-2,4,6 benzènesulfonylhydrazone de la β-diméthylamino propiophénone, de 64 $cm^3$ d'une solution 1,5 M de n-butyllithium dans l'hexane et de 6,5 g de méthoxy-3 benzaldéhyde, dans 200 $cm^3$ de diméthoxy-1,2 éthane, et après recristallisation du produit brut à l'état de base dans 200 $cm^3$ d'acétone, on obtient 4,7 g de (méthoxy-3 phényl)-1 diméthylamino-4 phényl-2 butène-2 ol-1 (Z) fondant à 160°C.

**EXEMPLE 29**

En opérant comme à l'exemple 22 mais à partir de 20 g de tris-isopropyl-2,4,6 benzènesulfonylhydrazone de la β-diméthylaminopropiophénone, de 64 $cm^3$ d'une solution 1,5 M de n-butyllithium dans l'hexane et de 8 g de diméthoxy-2,3 benzaldéhyde, dans 200 $cm^3$ de diméthoxy-1,2 éthane, et après recristallisation du produit brut à l'état de base dans 100 $cm^3$ d'oxyde d'isopropyle, on obtient 6,7 g de (diméthoxy-2,3 phényl)-1 diméthylamino-4 phényl-2 butène-2 ol 1 (Z) fondant à 105°C.

**EXEMPLE 30**

A une solution de 9,7 g de diphényl-2,3 méthylamino-5 dihydro-2,5 furanne dans 90 $cm^3$ de méthanol et 10 $cm^3$ d'eau distillée, on ajoute 10,3 g de borohydrure de sodium en 30 minutes environ à une température voisine de 5°C. Le mélange réactionnel est agité ensuite pendant 18 heures à une température voisine de 20°C, puis concentré à sec sous pression réduite (2,7 kPa) à 40°C. Le résidu obtenu est trituré avec 100 $cm^3$ de chlorure de méthylène; la suspension obtenue est filtrée et le filtrat est concentré à sec sous pression réduite (2,7 kPa) à 40°C. Le résidu est purifié par chromatographie sur 400 g de gel de silice contenus dans une colonne de 4 cm de diamètre [éluant: méthanol-ammoniaque (98 - 2 en volumes)]. On élue d'abord avec 400 $cm^3$ de solvant: l'éluat correspondant est éliminé; on élue ensuite avec 600 $cm^3$ de solvant: l'éluat correspondant est concentré à sec sous pression réduite (2,7 kPa) à 40°C. Le résidu obtenu est recristallisé dans un mélange de 70 $cm^3$ d'hexane et de 10 $cm^3$ d'isopropanol. On obtient ainsi 3,8 g de diphényl-1,2 méthylamino-4 butène-2 ol-1 (Z), sous forme de cristaux blancs fondant à 90°C.

Le diphényl-2,3 méthylamino-5 dihydro-2,5 furanne peut etre préparé de la manière suivante: A une suspension de 3,4 g d'hydrure de sodium dans 50 $cm^3$ de tétrahydrofuranne maintenue sous atmosphère d'azote à une température voisine de 0°C, on ajoute 29,7 g de benzoïne en solution dans 400 $cm^3$ de tétrahydrofuranne et on agite le mélange réactionnel pendant 30 minutes en laissant remonter la témpérature à 24°C. On ajoute ensuite 27 g de méthylimino-2 éthylphosphonate de diéthyle dans 270 $cm^3$ de tétrahydrofuranne. Le mélange réactionnel est alors porté à reflux pendant 1 heure puis refroidi à une température voisine de 20°C. On ajoute ensuite 1 litre d'eau et le mélange est extrait deux fois par 200 $cm^3$ au total d'acétate d'éthyle. La phase organique est lavée avec 2 fois 100 $cm^3$ d'eau puis séchée sur du sulfate de

magnésium, filtrée puis évaporée à sec sous pression réduite (2,7 kPa) à 40°C. Le résidu est lavé avec 100 $cm^3$ d'éther éthylique puis filtré. Le filtrat est évaporé à sec sous pression réduite (2,7 kPa) à 40°C pour donner une huile qui est purifiée par chromatographie "flash" [éluant: acétate d'éthyle]. Après évaporation à sec sous pression réduite (2,7 kPa) à 40°C des fractions 8 à 19, on obtient une huile orange qui est mise en solution dans 20 $cm^3$ d'un mélange d'acétate d'éthyle et d'éther éthylique (50 - 50 en volumes). La solution est additionnée de 3,9 $cm^3$ d'une solution 5,7N d'acide chlorhydrique gazeux dans l'éther éthylique. Le solide obtenu est séparé par filtration puis recristallisé dans 120 $cm^3$ d'isopropanol. On obtient ainsi 2,8 g de chlorhydrate de diphényl-2,3 méthylamino-5 dihydro 2,5 furanne sous forme d'un solide blanc fondant à 190°C.

Le méthylimino-2 éthylphosphonate de diéthyle peut être préparé de la manière suivante: A une solution de 32,4 g de diéthylphosphonoacétaldèhyde dans 100 $cm^3$ de méthanol, on ajoute 16,5 $cm^3$ d'une solution à 33 % de méthylamine. Le mélange réactionnel est agité pendant 3 heures à une température voisine de 20°C, puis est concentré à sec sous pression réduite (2,7 kPa) à 40°C. Le résidu est dissous dans la quantité minimum d'éther éthylique; la solution obtenue est séchée sur du carbonate de potassium, filtrée puis concentrée au tiers du volume initial sous pression réduite (2,7 kPa) à 20°C. Les cristaux obtenus sont séparés par filtration puis séchés à une température voisine de 20°C. On obtient ainsi 18,4 g de méthyl-imino-2 éthylphosphonate de diéthyle sous forme d'une poudre jaune fondant à 66°C.

Le diéthylphosphonoacétaldéhyde peut être préparé selon la méthode décrite par N.D. DAWSON et A. BURGER, J. Am. Chem. Soc., 74, 5312, (1952).

**EXEMPLE 31**

En opérant d'une manière analogue à celle décrite à l'exemple 30, mais à partir de 25 g de diphényl-2,3 éthylamino-5 dihydro-2,5 furanne et de 25 g de borohydrure de sodium, et après purification par chromatographie sur gel de silice (éluant: méthanol) puis recristallisation dans l'oxyde d'isopropyle, on obtient 8,4 g de diphényl-1,2 éthylamino-4 butène-2 ol-1 (Z), sous forme d'une poudre blanche fondant à 82°C.

Le diphényl-2,3 éthylamino-5 dihydro-2,5 furanne peut être préparé par une méthode analogue à celle décrite à l'exemple 30 mais à partir de 63 g d'éthylimino-2 éthylphosphonate de diéthyle, de 64 g de benzoïne et de 6,55 g d'hydrure de sodium. On obtient une huile que l'on purifie par chromatographie sur gel de silice [éluant: acétate d'éthyle - cyclohexane (50 - 50 en volumes)]. On obtient ainsi 25 g de diphényl-2,3 éthylamino-5 dihydro-2,5 furanne sous forme d'une huile jaune-orangée [Rf = 0,4; chromatographie sur gel de silice; éluant: acétate d'éthyle - cyclohexane (50 - 50 en volumes)].

L'éthylimino-2 éthylphosphonate de diéthyle peut être préparé par une méthode analogue à celle décrite à l'exemple 30 mais à partir de 60 g de diéthylphosphonoacétaldéhyde et de 15 g d'éthylamine. Après concentration à sec sous pression réduite (2,7 kPa) à 40°C du mélange réactionnel, on obtient 63,8 g d'éthylimino-2 éthylphosphonate de diéthyle sous forme d'une huile jaune [Rf = 0,47; chromatographie sur gel de silice; éluant: acétate d'éthyleméthanol (92,5 - 7,5 en volumes)].

**EXEMPLE 32**

En opérant d'une manière analogue à celle décrite à l'exemple 30, mais à partir de 33,3 g de diphényl-2,3 (propyl-2 amino)-5 dihydro-2,5 furanne et de 31,6 g de borohydrure de sodium, on obtient, après passage au chlorhydrate dans l'éther éthylique et recristallisation de ce chlorhydrate dans l'éthanol, 9,4 g de chlorhydrate de diphényl-1,2 (propyl-2 amino)-4 butène-2 ol-1 (Z), sous forme de cristaux blancs fondant à 188°C.

Le diphényl-2,3 (propyl-2 amino)-5 dihydro-2,5 furanne peut être préparé d'une manière analogue à celle décrite à l'exemple 30 mais à partir de 66 g de (propyl-2 imino)-2 éthylphosphonate de diéthyle, de 63,3 g de benzoïne et de 12,9 g d'hydrure de sodium.On obtient ainsi 33,3 g de diphényl-2,3 (propyl-2 amino)-5 dihydro-2,5 furanne sous forme d'une huile jaune [Rf = 0,46; chromatographie sur couche mince de gel de silice, éluant: acétate d'éthyle - cyclohexane (50 - 50 en volumes)].

Le (propyl-2 imino)-2 éthylphosphonate de diéthyle peut être préparé par une méthode analogue à celle décrite à l'exemple 30 mais à partir de 60 g de diéthylphosphonoacétaldéhyde et de 19,7 g d'isopropylamine. On obtient ainsi 67,9 g de (propyl-2 imino)-2 éthylphosphonate de diéthyle sous forme d'une huile jaune [Rf = 0,53; chromatographie sur couche mince degel de silice, éluant: acétate d'éthyle - méthanol (92,5 - 7,5 en volumes).]

**EXEMPLE 33**

En opérant d'une manière analogue à celle décrite à l'exemple 30, mais à partir de 29,6 g de butylamino-5 diphényl-2,3 dihydro-2,5 furanne et de 26,7 g de borohydrure de sodium, et après passage au chlorhydrate dans l'éther éthylique et recristallisation de ce chlorhydrate dans l'acétone, on obtient 4,3 g de chlorhydrate de

butylamino-4 diphényl-1,2 butène-2 ol-1 (Z), sous forme de cristaux blancs fondant à 155° C.

Le butylamino-5 diphényl-2,3 dihydro-2,5 furanne peut être préparé par une méthode analogue à celle décrite à l'exemple 30 mais à partir de 64 g de butylimino-2 éthylphosphonate de diéthyle, de 57,7 g de benzoïne et de 11,8 g d'hydrure de sodium; on obtient ainsi 29,6 g de butylamino-5 diphényl-2,3 dihydro-2,5 furanne sous forme d'une huile orangée [Rf = 0,50; chromatographie sur couche mince de gel de silice, éluant: acétate d'éthyle - cyclohexane (50 - 50 en volumes)].

Le butylimino-2 éthylphosphonate de diéthyle peut être préparé par une méthode analogue à celle décrite à l'exemple 30 mais à partir de 54 g de diéthylphosphonoacétaldéhyde et de 21,9 g de butylamine. On obtient ainsi 66,2 g de butylimino-2 éthylphosphonate de diéthyle sous forme d'une huile jaune [Rf = 0,40; chromatographie sur couche mince de gel de silice, éluant: acétate d'éthyle - méthanol (92,5 - 7,5 en volumes)].

**EXEMPLE 34**

En opérant d'une manière analogue à celle décrite à l'exemple 30, mais à partir de 26,9 g d'allylamino-5 diphényl-2,3 dihydro-2,5 furanne et de 25 g de borohydrure de sodium, on obtient un produit brut que l'on purifie par chromatographie sur 100 g de gel de silice contenus dans une colonne de 8 cm de diamètre (éluant: méthanol). L'huile jaune obtenue (6 g) est dissoute dans 100 $cm^3$ d'éther éthylique.

A la solution obtenue on ajoute 20 $cm^3$ d'une solution 3,5N d'acide chlorhydrique gazeux dans l'éther éthylique. Les cristaux qui précipitent sont séparés par filtration et recristallisés dans un mélange d'éther isopropylique et d'acétone (50 - 50 en volumes). On obtient ainsi 4,2 g de chlorhydrate d'allylamino-4 diphényl-1,2 butène-2 ol-1 (Z), sous forme d'une poudre blanche fondant à 113° C.

L'allylamino-5 diphényl-2,3 dihydro-2,5 furanne peut être préparé d'une manière analogue à celle décrite à l'exemple 30 pour la synthèse du diphényl-2,3 méthylamino-5 dihydro-2,5 furanne mais à partir de 32,3 g d'allylimino-2 éthylphosphonate de diéthyle, de 31,3 g de benzoïne et de 3,2 g d'hydrure de sodium. On obtient ainsi 26,9 g d'allylamino-5 diphényl-2,3 dihydro-2,5 furanne sous forme d'une huile jaune [Rf = 0,5; chromatographie sur couche mince de gel de silice, éluant: cyclohexane - acétate d'éthyle (50 - 50 en volumes)].

L'allylimino-2 éthylphosphonate de diéthyle peut être préparé d'une manière analogue à celle décrite à l'exemple 30 pour la préparation du méthylimino-2 éthylphosphonate de diéthyle mais à partir de 29 g de diéthylphosphonoacétaldéhyde et de 11,8 $cm^3$ d'allylamine. On obtient 32,3 g d'allylimino-2 éthylphosphonate de diéthyle sous forme d'une huile jaune [Rf = 0,38; chromatographie sur couche mince de gel de silice, éluant: acétate d'éthyle - méthanol (92,5 - 7,5 en volumes)].

**EXEMPLE 35**

A une solution de 5 g de diméthylamino-3 acrylophénone dans 16 $cm^3$ de chlorure de méthylène, on ajoute goutte à goutte à 0° C, 2,5 $cm^3$ d'oxychlorure de phosphore en solution dans 6 $cm^3$ de chlorure de méthylène. Après agitation pendant 15 minutes à 0° C puis 30 minutes à 20° C, la température est à nouveau ramenée à 0° C. Le précipité formé est dissous par addition de 5 $cm^3$ de méthanol. Cn ajoute goutte à goutte 8 $cm^3$ de triéthylamine et 3,15 $cm^3$ de thiophénol en solution dans 10 $cm^3$ de chlorure de méthylène. Après 2 heures 30 minutes d'agitation à 20° C, on ajoute 30 $cm^3$ de méthanol puis 1 g de cyanoborohydrure de sodium à 0° C et poursuit l'agitation pendant 3 heures à 20° C. Le mélange est ensuite concentré sous pression réduite (2,7 kPa) à 40° C. Le solide obtenu est repris au chloroforme et la solution est filtrée. La phase organique est évaporée sous pression réduite (2,7 kPa) à 40° C. Le résidu obtenu est repris à l'eau et la la solution est alcalinisée à pH 10 par addition d'une solution de soude aqueuse à 35 %, puis extraite au chlorure de méthylène. La phase organique est séchée sur sulfate de sodium, filtrée puis évaporée à sec sous pression réduite (2,7 kPa) à 40° C. Le résidu obtenu est dissous dans 10 $cm^3$ d'éther éthylique. A la solution obtenue, on ajoute 10 $cm^3$ d'une solution 3N d'acide chlorhydrique gazeux dans l'éther éthylique. Le précipité formé est séparé par filtration puis recristallisé dans un mélange de 15 $cm^3$ d'éthanol et 20 $cm^3$ d'éther éthylique. On obtient ainsi 3 g de chlorhydrate de diméthylamino-1 phényl-3 phénylthio-3 propène-2 (Z), sous forme de cristaux blancs fondant à 176° C.

La diméthylamino-3 acrylophénone peut être préparée par la méthode décrite par H. MEERWEIN, W. FLORIAN, N. SCHON et G. STOPP, Ann. Chem. 641, 1, (1961).

**EXEMPLE 36**

En opérant d'une manière analogue à celle décrite à l'exemple 35 mais à partir de 80 g de diméthylamino-3 acrylophénone, de 40 $cm^3$ d'oxychlorure de phosphore, de 50,3 $cm^3$ de thiophénol et de 16 g de cyanoborohydrure de sodium, et cristallisation de l'isomère (Z), on isole après évaporation des liqueurs-mères

sous pression réduite (2,7 kPa) à 30°C puis cristallisation du résidu obtenu dans un mélange d'acétone et d'éther éthylique (25 - 75 en volumes) un solide jaune qui est recristallisé dans 230 $cm^3$ de méthyléthylcétone. On obtient ainsi 14,3 g de chlorhydrate de diméthylamino-1 phényl-3 phénylthio-3 propène-2 (E), sous forme d'un solide blanc fondant à 146 - 147°C.

**EXEMPLE 37**

En opérant d'une manière analogue à celle décrite à l'exemple 35, mais à partir de 5 g de diméthylamino-3 acrylophénone, de 2,5 $cm^3$ d'oxychlorure de phosphore, de 4,13 g de p-chlorothiophénol et de 1 g de cyanoborohydrure de sodium, et après évaporation sous pression réduite (2,7 kPa) à 40°C du mélange réactionnel, on obtient un résidu que l'on dissout dans 50 $cm^3$ d'éthanol. A cette solution, on ajoute 12 $cm^3$ d'une solution 3N d'acide chlorhydrique gazeux dans l'éther éthylique. Le précipité qui se forme est séparé par filtration puis recristallisé dans 70 $cm^3$ d'éthanol. On obtient ainsi 3,4 g de chlorhydrate de (chloro-4 phénylthio)-3 diméthylamino-1 phénylthio-3 propène-2 (Z), sous forme d'une poudre blanche fondant à 226°C.

**EXEMPLE 38**

En opérant d'une manière analogue à celle décrite à l'exemple 35, mais à partir de 10 g de diméthylamino-3 acrylophénone, de 5 $cm^3$ d'oxychlorure de phosphore, de 6,35 g de mercapto-4 pyridine et de 2 g de cyanoborohydrure de sodium, on obtient un résidu marron qui est repris avec 100 $cm^3$ d'eau. La solution est acidifiée à pH 2 par addition d'une solution aqueuse d'acide chlorhydrique concentré puis lavée avec 2 fois 50 $cm^3$ d'éther éthylique. La phase aqueuse est ajustée à pH 10 par addition d'une solution aqueuse de soude concentrée puis extraite au chlorure de méthylène. La phase organique résultante est séchée sur sulfate de sodium, filtrée puis concentrée à sec sous pression réduite (2,7 kPa) à 30°C, pour donner un résidu rouge qui est chromatographié sur gel de silice [étuant: chlorure de méthylène - méthanol (95 - 5 en volumes)] en recueillant des fractions de 200 $cm^3$. Les fractions 13 à 25 sont concentrées sous pression réduite (2,7 kPa) à 30°C. On obtient un résidu que l'on dissout dans 15 $cm^3$ d'éthanol. A cette solution, on ajoute 20 $cm^3$ d'une solution éthanolique 1,2N d'acide oxalique; le solide qui précipite est séparé par filtration et recristallisé dans 110 $cm^3$ d'un mélange d'acétonitrile et d'éthanol (20 - 80 en volumes). On obtient ainsi 3 g de sesquioxalate du diméthylamino-1 phényl-3 (pyridyl-4 thio)-3 propène-2 (Z), sous forme de cristaux blancs fondant à 173°C.

**EXEMPLE 39**

En opérant d'une manière analogue à celle décrite à l'exemple 35, mais à partir de 10 g de (chloro-4 phényl)-1 diméthylamino-3 propène-2-one-1, de 4,2 $cm^3$ d'oxychlorure de phosphore, de 5,2 $cm^3$ de thiophénol et de 1,79 g de cyanoborohydrure de sodium, on obtient un résidu orangé que l'on reprend avec 100 $cm^3$ d'eau. La solution est alcalinisée à pH 10 par addition d'une solution aqueuse de soude concentrée puis extraite au chlorure de méthylène. La phase organique est séchée sur sulfate de sodium, filtrée, puis évaporée à sec sous pression réduite (2,7 kPa) à 30°C. Le résidu obtenu est purifié par chromatographie "flash" [éluant: chlorure de méthylèneméthanol (95 - 5 en volumes)]. Après évaporation à sec sous pression réduite (2,7 kPa) à 30°C des fractions 33 à 60, on obtient 7,8 g d'une huile jaune. Cette huile est dissoute dans 50 $cm^3$ d'acétone et le solide obtenu est additionné de 20 $cm^3$ d'une solution 1,3 M d'acide oxalique dans l'acétone. Le solide blanc qui précipite est séparé par filtration et recristallisé dans 130 $cm^3$ d'éthanol. On obtient ainsi 3 g d'oxalate acide du (chloro-4 phényl)-3 diméthyl-amino-1 phénylthio-3 propène-2 (Z), sous forme de cristaux blancs fondant à 183°C.

La (chloro-4 phényl)-1 diméthylamino-3 propène-2 one-1 peut être obtenue par la méthode décrite par H. MEERWEIN, W. FLORIAN, N. SCHON et C. STOPP, Ann. Chem. 641, 1, (1961).

**EXEMPLE 40**

En opérant d'une manière analogue à celle décrite à l'exemple 35 mais à partir de 10 g de (fluoro-4 phényl)-1 diméthylamino-3 propène-2-one-1, de 4,76 $cm^3$ d'oxychlorure de phosphore de 5,57 $cm^3$ de thiophénol et de 1,92 g de cyanoborohydrure de sodium, on obtient un résidu que l'on reprend au chloroforme. La solution est ensuite ajustée à pH 10 par addition d'une solution aqueuse de soude concentrée. La phase organique est décantée puis la phase aqueuse extraite au chlorure de méthylène. Les phases organiques sont rassemblées, séchées sur du sulfate de sodium, filtrées puis évaporées à sec sous pression réduite (2,7 kPa) à 30°C. On obtient un résidu que l'on dissout dans 30 $cm^3$ d'isopropanol. A cette solution on ajoute 12 $cm^3$ d'une solution

3N d'acide chlorhydrique gazeux dans l'éther éthylique. Le solide qui précipite est séparé par filtration et recristallisé dans 30 $cm^3$ d'isopropanol. On obtient ainsi 3,3 g de chlorhydrate de diméthylamino-1 (fluoro-4 phényl)-3 phénylthio-3 propène-2 (E), sous forme d'un solide blanc fondant à 160° C.

La (fluoro-4 phényl)-1 diméthylamino-3 propène-2-one-1 peut être obtenue par application de la méthode décrite par R. MEERWEIN, W. FLORIAN, N. SCHON et G. STOPP, Ann. Chem. 641, 1, (1961).

## EXEMPLE 41

A partir des eaux-mères de cristallisation obtenues à l'exemple 40 et évaporation des solvants, on obtient un résidu que l'on dissout dans 50 $cm^3$ d'eau. La solution obtenue est alcalinisée à pH 10 avec une solution de soude concentrée. Après extraction au chlorure de méthylène, la phase organique est séchée sur sulfate de sodium, filtrée et évaporée à sec. On obtient une huile orangée que l'on purifie par chromatographie "flash" [éluant: chlorure de méthylène - méthanol (95 - 5 en volumes)]. Les fractions 33 à 100 sont concentrées sous pression réduite (2,7 kPa) à 30° C pour donner un résidu que l'on dissout dans 40 $cm^3$ d'éthanol. A cette solution, on ajoute 30 $cm^3$ d'une solution 1,1 M d'acide oxalique dans l'acétone. Le solide qui précipite est séparé par filtration et recristallisé dans 40 $cm^3$ d'éthanol. On obtient ainsi 2,5 g d'oxalate de diméthylamino-1 (fluoro-4 phénylthio)-3 phényl-3 propène-2 (Z), sous la forme d'un solide blanc fondant à 163° C.

## EXEMPLE 42

En opérant d'une manière analogue à celle décrite à l'exemple 35 mais à partir de 10 g de (bromo-4 phényl)-1 diméthylamino-3 propène-2-one-1, de 3,64 g d'oxychlorure de phosphore, de 4,2 $cm^3$ de thiophénol et de 1,47 g de cyanoborohydrure de sodium, on obtient un résidu que l'on reprend au chloroforme. La solution est ajustée à pH 10 par addition d'une solution aqueuse de soude concentrée. La phase organique est décantée puis la phase aqueuse est extraite au chlorure de méthylène. Les phases organiques sont rassemblées, séchées sur sulfate de sodium, filtrées puis évaporées à sec sous pression réduite (2,6 kPa) à 30° C pour donner un résidu que l'on dissout dans 30 $cm^3$ d'isopropanol. A la solution obtenue, on ajoute 13 $cm^3$ d'une solution éthérée 3N d'acide chlorhydrique gazeux. Le solide blanc qui précipite est séparé par filtration et recristallisé 2 fois dans 40 $cm^3$ d'isopropanol. On obtient ainsi 4,8 g de chlorhydrate de (bromo-4 phényl)-3 diméthylamino-1 phénylthio-3 propène-2 (E), sous forme de cristaux blancs fondant à 187° C.

La (bromo-4 phényl)-1 diméthylamino-3 propène-2-one-1 peut être obtenue par application de la méthode de H. MEERWEIN, W. FLORIAN, N. SCHON et G. STOPP, Ann. Chem. 641, 1, (1961).

## EXEMPLE 43

A partir des eaux-mères de cristallisation obtenues à l'exemple 42 et évaporation des solvants, on obtient un résidu que l'on dissout dans 100 $cm^3$ d'eau. La solution obtenue est alcalinisée à pH 10 par addition d'une solution de soude aqueuse. Après extraction au chlorure de méthylène, la phase organique est séchée sur sulfate de sodium, filtrée puis concentrée sous pression réduite (2,7 kPa) à 30° C. On obtient un résidu que l'on dissout dans 20 $cm^3$ d'éthanol. A cette solution, on ajoute 10 $cm^3$ d'une solution 1,7 M d'acidé oxalique dans l'acétone. Le solide blanc qui précipite est séparé par filtration et recristallisé dans 160 $cm^3$ d'éthanol. On obtient ainsi 3,7 g d'un mélange (75 - 25) des oxalates de (bromo-4 phényl)-3 diméthylamino-1 phénylthio-3 propène-2 (Z) et de (bromo-4 phényl)-3 diméthylamino-1 phénylthio-3 propène-2 (E), sous forme de cristaux blancs fondant à 281° C.

## EXEMPLE 44

En opérant d'une manière analogue à celle décrite à l'exemple 35, mais à partir de 5 g de diméthylamino-3 acrylophénone, de 2,47 $cm^3$ d'oxychlorure de phosphore, de 5,39 g de bromo-4 thiophénol et 1 g de cyanoborohydrure de sodium, on obtient un résidu marron que l'on reprend au chloroforme. La solution est alcalinisée avec une solution de soude aqueuse jusqu'à pH 10. Après décantation de la phase chloroformique, la phase aqueuse est extraite au chlorure de méthylène. Les phases organiques sont rassemblées, séchées sur sulfate de sodium, filtrées puis évaporées à sec sous pression réduite (2,7 kPa) à 30° C. On obtient un résidu que l'on dissout dans 60 $cm^3$ d'éthanol. A cette solution, on ajoute 7,7 $cm^3$ d'une solution éthérée 3N d'acide chlorhydrique gazeux. Le solide blanc qui précipite est séparé par filtration et recristallisé dans un mélange d'isopropanol et de méthanol (60 - 40 en volumes). On obtient ainsi 1,8 g de chlorhydrate de (bromo-4 phénylthio)-3 diméthyl-amino-1 phényl-3 propène-2 (Z), sous forme d'un solide blanc fondant à 252° C.

**EXEMPLE 45**

A une solution agitée de 40,5 g de phényl-3 phénylthio-3 propène-2 al (Z) dans 370 cm$^3$ de méthanol, on ajoute sous atmosphère d'argon 139 g de chlorhydrate de diméthylamine et 2 g de tamis moléculaire 3Å. Après 5 minutes d'agitation, on ajoute 10,7 g de cyanoborohydrure de sodium et poursuit l'agitation pendant 18 heures. On ajoute alors 26 cm$^3$ d'une solution aqueuse d'acide chlorhydrique concentré puis concentre le mélange à sec sous pression réduite (2,7 kPa) à 40°C. Le résidu obtenu est repris à l'eau puis lavé à l'éther éthylique. La phase aqueuse est alcalinisée à pH 10 par addition d'une solution aqueuse de soude à 35 % puis reprise au chlorure de méthylène. La phase organique est décantée et filtrée sur terre de diatomée, lavée avec 100 cm$^3$ d'eau puis séchée sur sulfate de sodium. Après filtration, la phase organique est évaporée à sec sous pression réduite (2,7 kPa) à 40°C. On obtient ainsi une huile marron que l'on purifie par chromatographie sur gel de silice [éluant : chlorure de méthylène - méthanol (90 - 10 en volumes)] en recueillant des fractions de 50 cm$^3$. Après concentration à sec des fractions 20 à 30, on obtient un résidu que l'on dissout dans 10 cm$^3$ d'éthanol et 70 cm$^3$ d'éther éthylique. A cette solution, on ajoute 17 cm$^3$ d'une solution 3,5N d'acide chlorhydrique gazeux dans l'éther éthylique. Le précipité formé est séparé par filtration puis recristallisé dans un mélange de 38 cm$^3$ d'éthanol et de 50 cm$^3$ d'éther éthylique. On obtient ainsi 8,9 g de chlorhydrate de diméthylamino-1 phényl-3 phénylthio-3 propène-2 (Z), sous forme de cristaux blancs fondant à 176°C.

Le phényl-3 phénylthio-3 propène-2 al (Z) peut être préparé de la manière suivante: A une solution de 9,4 g de chloro-3 phényl-3 propène-2-al (Z) dans 50 cm$^3$ de dichlorométhane, on ajoute à 0°C, 9,66 cm$^3$ de triéthylamine puis 5,7 cm$^3$ de thiophénol. Après 14 heures d'agitation à 20°C, le mélange est versé sur 50 cm$^3$ d'eau; la solution est ajustée à pH 7 par addition d'une solution aqueuse d'acide chlorhydrique concentré puis reprise au chlorure de méthylène. La phase organique est décantée, séchée sur sulfate de sodium, filtrée puis évaporée à sec sous pression réduite (2,7 kPa) à 40°C. On obtient ainsi un résidu que l'on purifie par chromatographie sur gel de silice [éluant: hexane puis acétate d'éthyle]; après élution des impuretés non polaires à l'hexane (400 cm$^3$), les fractions 8 à 15 provenant de l'élution à l'acétate d'éthyle (400 cm$^3$) sont réunies et évaporées à sec sous pression réduite (2,7 kPa) à 40°C. On obtient ainsi 7,8 g de phényl-3 phénylthio-3 propène-2 al (Z), sous forme d'une huile rouge de Rf = 0,9 en chromatographie sur couche mince de gel de silice [éluant: acétate d'éthyle - hexane (50 - 50 en volumes)].

Le chloro-3 phényl-3 propène-2 al (Z) peut être préparée par la méthode décrite par C.M. BEATON, N.B. CHAPMAN. et K. CLARKE, J.Chem. Soc. Perkin I, 2355, (1976).

**EXEMPLE 46**

A une suspension de 3,85 g de magnésium en tournures dans 100 cm$^3$ d'éther éthylique, on ajoute goutte à goutte en 1 heure environ une solution de 24,8 g de bromobenzène dans 120 cm$^3$ d'éther éthylique. A la solution noire obtenue, on ajoute en 15 minutes envion 10 g de diméthylamino-4 phényl-1 butyne-2 ol-1 dans 30 cm$^3$ d'éther éthylique. Le mélange réactionnel est ensuite porte au reflux pendant 3 heures et 30 minutes, puis versé sur un mélange de 400 g de glace et de 200 cm$^3$ d'une solution aqueuse saturée de chlorure d'ammonium. La phase organique est séparée par décantation; la phase aqueuse est extraite 2 fois par 200 cm$^3$ de chlorure de méthylène; les phases organiques sont réunies, séchées sur du sulfate de magnésium, filtrées et concentrées à sec sous pression réduite (2,7 kPa) à 40°C. Le résidu est purifié par chromatographie "flash" [éluant: méthanol - acétate d'éthyle (60 - 40 en volumes)], les fractions 18 à 39 sont réunies et concentrées à sec sous pression réduite (2,7 kPa) à 40°C. On obtient, après recristallisation du résidu dans l'éther isopropylique, 3,9 g de diméthylamino-4 diphényl-1,2 butène-2 ol-1 (E), sous forme de cristaux blancs fondant à 113°C.

Le diméthylamino-4 phényl-1 butyne-2 ol-1 peut être préparé de la manière suivante: A une solution de 33,2 g de N,N-diméthyl popargylamine dans 400 cm$^3$ de diméthoxy-1,2 éthane maintenue sous atmosphère d'azote à une température voisine de -70°C, on ajoute 280 cm$^3$ d'une solution 1,55 M de n-butyllithium dans l'hexane. A la suspension obtenue, on ajoute à une température voisine de -30°C une solution de 42,4 g de benzaldéhyde dans 100 cm$^3$ de dinéthoxy-1,2 éthane. Le mélange réactionnel est agité pendant 30 minutes à une température voisine de 0°C, puis on ajoute 20 cm$^3$ d'eau distillée. La suspension obtenue est filtrée. La solution est séchée sur du sulfate de magnésium, filtrée et concentrée à sec sous pression réduite (2,7 kFa) à 40°C. On obtient ainsi 72,3 g de diméthylamino-4 phényl-1 butyn-2 ol-1 sous forme d'une huile jaune [Rf = 0,56; chromatographie sur couche mince de gel d'alumine; éluant: acétate d'éthyle].

**EXEMPLE 47**

A une solution de 4,7 g de chlorhydrate de diméthylamino-4 diphényl-1,2 butène-2 ol-1 (Z) dans 60 cm$^3$ de pyridine anhydre, maintenue à une température voisine de 0°C, on ajoute 4,3 g de chlorure de benzoyle. Le mélange réactionnel est ensuite agité pendant 18 heures à une température voisine de 20°C puis versé dans 300 cm$^3$ d'une solution aqueuse saturée de bicarbotate de sodium. Le mélange obtenu est extrait 3 fois avec

100 $cm^3$ d'acétate d'éthyle.

Les phases organiques sont réunies, lavées 2 fois avec 25 $cm^3$ d'eau, séchées sur sulfate de sodium et évaporées à sec sous pression réduite (2,7 kPa) à une température voisine de 40°C. Le résidu obtenu est ensuite dissous dans 50 $cm^3$ d'isopropanol. A cette solution portée à ébullition, on ajoute une solution de 1,4 g d'acide oxalique dans 10 $cm^3$ d'isopropanol. Le produit qui cristallise par refroidissement est séparé par filtration puis lavé 3 fois avec 3 $cm^3$ d'isopropanol. Par recristallisation du produit ainsi obtenu dans 90 $cm^3$ d'isopropanol, on obtient 5,3 g d'oxalate de benzoyloxy-4 diméthylamino-1 diphényl-3,4 butène-2 (Z), fondant à 170°C.

Le chlorhydrate de diméthylamino-4 diphényl-1,2 butène-2 ol-1 (Z) peut être préparé comme à l'exemple 1.

**EXEMPLE 48**

En opérant comme à l'exemple 47 mais à partir de 4,5 g de chlorhydrate de diméthylamino-4 diphényl-1,2 butène-2 ol-1 (Z), de 3,5 g de chlorure d'acétyle et de 1,35 g d'acide oxalique, on obtient, après recristallisation dans 300 $cm^3$ d'isopropanol, 3 g d'oxalate d'acétoxy-4 diméthylamino-1 diphényl-3,4 butène-2 (Z), fondant à 156°C.

Le chlorhydrate de diméthylamino-4 diphényl-1,2 butène-2 ol-1 (Z) peut être préparé comme à l'exemple 1.

**EXEMPLE 49**

A une solution de 4,4 g de diméthylamino-4 diphényl-1,2 butène-2 ol-1 (Z) sous forme de base libre, dans 50 $cm^3$ de toluène, on ajoute 1,9 $cm^3$ de chloroformiate d'éthyle. Le mélange est agité à une température de 90 - 95°C pendant 3 heures puis refroidi à température ambiante. Le précipité formé est filtré sur verre fritté puis lavé à l'oxyde d'isopropyle. On obtient ainsi 3,86 g de chlorhydrate de diméthylamino-4 éthoxycarbonyloxy-1 diphényl-1,2 butène-2 (Z), sous forme d'un solide blanc fondant à 178°C.

Le diméthylamino-4 diphényl-1,2 butène-2-ol-1 (Z) peut être obtenu de manière connue à partir de son chlorhydrate préparé comme à l'exemple 1.

**EXEMPLE 50**

A une solution de 41,6 $cm^3$ de tert-butylamine dans 100 $cm^3$ d'éthanol, on ajoute lentement à 0°C, 10,4 g de chloro-3 phényl-1 phénylthio-1 propène-1 (Z) brut en solution dans 40 $cm^3$ d'éthanol, et maintient l'agitation pendant 14 heures à température ambiante (22°C). Le mélange réactionnel est évaporé à sec sous pression réduite (2,7 kPa) à 30°C. On obtient alors ainsi un résidu jaune orangé que l'on purifie par chromatographie "flash" [éluant: chlorure de méthylène - méthanol (95 - 5 en volumes)]. Les fractions 21 à 60 sont réunies et concentrées à sec sous pression réduite (2,7 kPa) à 30°C. On obtient un solide que l'on recristallise dans l'acétonitrile. On obtient ainsi 1,4 g de phényl-3 phénylthio-3 tert-butyl-amino-1 propène-2 (Z), sous forme d'un solide blanc fondant à 206°C.

Le chloro-3 phényl-1 phénylthio-1 propène-1 (Z) peut être obtenu de la manière suivante: A une solution de 17,6 g de diméthyl-amino-1 phényl-3 phénylthio-3 propène-2 (Z) dans 230 $cm^3$ de toluène, on ajoute 9,4 $cm^3$ de chloroformiate d'éthyle. Le mélange est chauffé à 90°C pendant 2 heures 30 minutes puis évaporé à sec sous pression réduite (2,7 kPa) à 30°C. On obtient ainsi 21,8 g d'une huile jaune pêle contenant le chloro-1 phényl-3 phénylthio-3 propène-2 (Z) attendu en mélange avec le diméthyl carbamate d'éthyle formé au cours de la réaction. Cette huile brute est employée sans autre purification dans les synthèses ultérieures.

Spectre de R.M.N. (60 MHz, $CDCl_3$):

4,5 ppm, 2H: $-CH_2$

6,5 ppm, 1H: $-CH=$

6,9 - 75 ppm, 10 H: aromatiques

**EXEMPLE 51**

A 150 $cm^3$ d'une solution 4,2 N d'éthanol ammoniacal refroidie à 0°C, on ajoute goutte à goutte une solution de 17,2 g de chloro-3 phényl-1 phénylthio-1 propène-1 (Z) dans 68 $cm^3$ d'éthanol. Après 14 heures d'agitation à température ambiante, le mélange réactionnel est évaporé à sec sous pression réduite (2,7 kPa) à 30°C. On obtient ainsi une pâte jaune clair que l'on reprend par 100 $cm^3$ d'acétate d'éthyle. Le solide blanc qui se forme est séparé par filtration et recristallisé dans 250 $cm^3$ de méthyléthylcétone. On obtient ainsi 3,5 g d'un mélange de 75 % de chlorhydrate d'amino-3 phényl-1 phénylthio-1 propène-1 (Z) et de 25 % de chlorhydrate d'amino-3

phényl-1 phénylthio-1 propène-1 (E) sous forme d'un solide blanc fondant à 184° C.

## EXEMPLE 52

Un mélange de 0,6 g de chloro-4 diphényl-1,2 éthoxy-carbonyloxy-1 butène-2 (Z) et 6 $cm^3$ d'une solution 4,2N éthanolique d'ammoniac gazeux est chauffé en autoclave pendant 6 heures à 100° C puis refroidi à température ambiante. Le mélange réactionnel est évaporé à sec sous pression réduite (2,7 kPa) à 30° C, puis le résidu obtenu est purifié par chromatographie sur une colonne de gel de silice [éluant: méthanol - acétate d'éthyle (60 - 40 en volumes)] en recueillant des fractions de 2 $cm^3$. Les fractions 7 à 10 sont réunies et concentrées à sec sous pression réduite (2,7 kPa) à 30° C. On obtient ainsi 0,1 g d'amino-1 diphényl-3,4 éthoxycarbonyloxy-4 butène-2 (Z) sous forme d'huile jaune pâle [Rf: 0,15 acétate d'éthyle - méthanol (40 - 60 en volumes)].

Le chloro-4 diphényl-1,2 éthoxycarbonyl-1 butène-2 (Z) peut être obtenu de la manière suivante: A une solution de diméthyl-amino-1 éthoxycarbonyloxy-4 diphényl-3,4 butène-2 (Z) sous forme de base libre dans 30 $cm^3$ de toluène, on ajoute 1,2 $cm^3$ de chloroformiate d'éthyle. Le mélange est agité pendant 1 heure et 30 minutes à 90° C puis évaporé à sec sous pression réduite (2,7 kPa) à 30° C. On obtient un résidu que l'on chromatographie sur colonne de gel de silice [éluant cyclohexane - acétate d'éthyle (50 - 50 en volumes)] en recueillant des fractions de 30 $cm^3$. Les fractions 3 à 6 sont réunies et concentrées sous pression réduite (2,7 kPa) à 30° C. On obtient ainsi 1,56 g de chloro-4 diphényl-1,2 éthoxycarbonyloxy-1 butène-2 (Z) sous forme d'une huile jaune clair [Rf = 0,5, éluant cyclohexane - acétate d'éthyle (50 - 50 en volumes)].

Le diméthylamino-1 éthoxycarbonyloxy-4 diphényl-3,4 butène-2 (Z) peut être obtenu de manière connue à partir de son chlorhydrate préparé comme décrit à l'exemple 49.

## EXEMPLE 53

A une solution de 0,5 g d'azido-4 diphényl-1,2 éthoxy-carbonyloxy-1 butène-2 (Z) dans 7,5 $cm^3$ de méthanol, on ajoute 1,05 $cm^3$ de triéthylamine et 0,74 $cm^3$ de propanedithiol-1,3. Le mélange est agité sous atmosphère d'azote pendant 18 heures à température ambiante. Le mélange réactionnel est alors versé sur 10 $cm^3$ d'eau, ajusté à pH 12 avec une solution aqueuse de soude 4N puis extrait 3 fois au chlorure de méthylène. La phase organique est séchée sur sulfate de magnésium, filtrée puis évaporée sous pression réduite (2,7 kPa) à 30° C. Le résidu obtenu est purifié par chromatographie sur gel de silice [éluant: acétate d'éthyleméthanol (40 - 60 en volumes)] en recueillant des fractions de 5 $cm^3$. Les fractions 15 à 25 sont évaporées à sec sous pression réduite (2,7 kPa) à 30° C. On obtient ainsi 0,168 g d'amino-1 diphényl-3,4 éthoxycarbonyloxy-4 butène-2 (E) sous forme d'une huile jaune pâle [Rf = 0,15, éluant: méthanol - acétate d'éthyle (60 - 40 en volumes)].

L'azido-4 diphényl-1,2 éthoxycarbonyloxy-1 butène-2 (Z) peut être préparé de la manière suivante: A une solution de 0,65 g de chloro-4 diphényl-1,2 éthoxycarbonyloxy-1 butène-2 (Z) dans 6,5 $cm^3$ de N-N-diméthylformamide, refroidie à 0° C, on ajoute 0,2 g d'azoture de sodium puis chauffe le mélange sous agitation à 80° C pendant 15 minutes. Le mélange réactionnel est ensuite refroidi, additionné de 10 $cm^3$ d'eau et extrait avec 3 fois 10 $cm^3$ de chlorure de méthylène. La phase organique est séchée sur du sulfate de magnésium, filtrée puis évaporée à sec sous pression réduite (2,7 kPa) à 30° C. On obtient un résidu que l'on chromatographie sur une colonne de gel de silice [éluant: acétate d'éthyle - cyclohexane (30 - 70 en volumes)] en recueillant des fractions de 30 $cm^3$. Les fractions 1 à 5 sont réunies et concentrées à sec sous pression réduite (2,7 kPa) à 30° C. On obtient ainsi 0,54 g d'azido-4 diphényl-1,2 éthoxycarbonyloxy-1 butène-2 (Z) sous forme d'une huile rose pâle [Rf = 0,7; éluant acétate d'éthyle - cyclohexane (30 - 70 en volumes)].

Le chloro-4 diphényl-1,2 éthoxycarbonyloxy-1 butène-2 (Z) peut être obtenu comme à l'exemple 52.

## EXEMPLE 54

A une solution de 0,02 g d'amini-1 éthoxycarbonyloxy-4 diphényl-3,4 butène-2 (E) dans 1 $cm^3$ de méthanol, on ajoute 0,1 g de carbonate de potassium. La suspension obtenue est agitée pendant 70 heures à une température voisine de 20° C. Le mélange réactionnel est filtré puis concentré à sec sous pression réduite (2,7 kPa) à 40° C. Le résidu obtenu est dissous dans 0,2 $cm^3$ de méthanol; la solution obtenue est versée sur 1 g de gel de silice contenu dans une colonne de 5 mm de diamètre. On élue d'abord avec; $cm^3$ de méthanol, l'éluat correspondant est éliminé, puis avec 2 $cm^3$ de méthanol: l'éluat correspondant est concentré à sec sous pression réduite (2,7 kPa) à 40° C. On obtient ainsi 0,006 g d'amino-4 diphényl-1,2 butène-2 ol-1 (E) sous forme d'une poudre blanche.

Spectre RMN (250 MHz) $CDCl_3$:

3,20 ppm (d, 2H : $-CH_2$ N

5,40 ppm (s, 1H : -CHOH)

6 ppm (t, 1H : -CH=C)

6,9 ppm (m, 10H : protons aromatiques)

7,30 ppm (m, 10H : protons aromatiques)

L'amino-1 éthoxycarbonyloxy-4 diphényl-3,4 butène-2 (E) peut être préparé comme décrit à l'exemple 53.

**EXEMPLE 55**

A une solution de 10,5 g de chlorhydrate de diméthylamino-4 diphényl-1,2 butène-2 ol-1 (Z) dans 150 $cm^3$ de chloroforme, on ajoute 2,1 $cm^3$ d'isocyanate de méthyle et 10 mg de diméthylamino-4 pyridine. Le mélange réactionnel est agité pendant 18 heures à une température voisine de 20°C puis traité avec 100 $cm^3$ d'une solution aqueuse saturée de bicarbonate de sodium. La phase aqueuse est décantée, lavée 2 fois avec 50 $cm^3$ de chlorure de méthylène puis les phases organiques sont réunies, lavées 2 fois avec 25 $cm^3$ d'eau, séchées sur sulfate de sodium et évaporées à sec sous pression réduite (2,7 kPa) à une température voisine de 40°C. Le résidu obtenu est ensuite dissous dans 100 $cm^3$ d'isopropanol; on porte cette solution à ébullition et y ajoute une solution de 3,6 g d'acide oxalique dans 50 $cm^3$ d'isopropanol. Le produit qui cristallise par refroidissement est séparé par filtration puis lavé 3 fois avec 5 $cm^3$ d'isopropanol. Par recristallisation du produit ainsi obtenu dans 150 $cm^3$ d'éthanol, on obtient 9,1 g d'oxalate de diméthylamino-1 méthylcarbamoyloxy-4 diphényl-3,4 butène-2 (Z) fondant à 194°C.

Le chlorhydrate de diméthylamino-4 diphényl-1,2 butène-2 ol-1 (Z) peut être préparé comme à l'exemple 1.

**EXEMPLE 56**

A une solution de 0,5 g de chlorhydrate d'allylamino-4 diphényl-1,2 butène-2 ol-1 (Z) dans 4,5 $cm^3$ d'eau, on ajoute 0,036 g de palladium sur charbon à 10 % (poids/volume) et agite le mélange à une température de 100°C sous atmosphère d'azote pendant 18 heures. On ajoute alors 0,018 g de palladium sur charbon à 10 % (poids/volume), et on poursuit le chauffage à reflux pendant 12 heures. Après refroidissement à température ambiante, le catalyseur est séparé par filtration et la solution est additionnée de 5 $cm^3$ d'une solution 4N de soude aqueuse puis de chlorure de sodium jusqu'à saturation. La phase aqueuse est extraite avec 3 fois 20 $cm^3$ de chlorure de méthylène. La phase organique est séchée sur sulfate de magnésium, filtrée puis évaporée à sec sous pression réduite (2,7 kPa) à 30°C pour donner une huile jaune clair que l'on chromatographie sur une colonne de gel de silice (éluant: méthanol) en recueillant des fractions de 2 $cm^3$. Les fractions 22 à 30 sont réunies et concentrées à sec sous pression réduite (2,7 kPa) à 30°C. On obtient 0,035 g d'amino-4 diphényl-1,2 butène-2-ol-1 (Z) sous forme d'un solide blanc.

Spectre de R.M.N. (250 MHz, $CDCl_3$):

3,35 ppm (m, 2H : - $CH_2NH_2$)

5,75 ppm (s, 1H :-CHOH-)

5,90 ppm (t, 1H :-CH=)

7,25 ppm (m, 10H : protons aromatiques)

7,40 ppm (m, 10H: protons aromatiques)

L'allylamino-4 diphényl-1,2 butène-2-ol-1 (Z) peut être obtenu comme décrit à l'exemple 34.

**EXEMPLE 57**

A une solution de 6 g de chlorhydrate de diméthylamino-1 phényl-3 phénylthio-3 propène-2 (Z) dans 68 $cm^3$ de dichlorométhane, on ajoute à 0°C, 4,7 g d'acide m-chloroperbenzoïque. Après 24 heures d'agitation à 20°C, on ajoute 4,7 g d'acide m-chloroperbenzoïque supplémentaires et poursuit l'agitation pendant 3 heures. Le mélange réactionnel est versé sur 100 $cm^3$ d'eau; la solution est ajustée à pH 10 par addition d'une solution aqueuse de soude à 35 % puis reprise au chlorure de méthylène. La phase organique est décantée, séchée sur

sulfate de sodium, filtrée puis évaporée à sec sous pression réduite (2,7 kPa) à 40° C. On obtient ainsi un résidu que l'on dissout dans 10 $cm^3$ d'éthanol. A cette solution, on ajoute 6 $cm^3$ d'une solution 3N d'acide chlorhydrique gazeux dans l'éther éthylique. Le solide qui précipite est séparé par filtration puis recristallisé dans un mélange de 60 $cm^3$ d'éthanol et de 5 $cm^3$ d'acétonitrile. On obtient ainsi 2 g de chlorhydrate de diméthylamino-1 phényl-3 phénylsulfinyl-3 propène-2 (Z), sous forme de cristaux blancs fondant à 268° C (décomposition).

Le chlorhydrate de diméthylamino-1 phényl-3 phénylthio-3 propène-2 (forme Z) peut être obtenu comme décrit à l'exemple 35.

**EXEMPLE 58**

En opérant d'une manière analogue à celle décrite à l'exemple 22 mais à partir de 10 g de tris-isopropyl-2,4,6 benzènesulfonylhydrazone de la β-diméthylaminopropiophénone et de 2,6 $cm^3$ de fluoro-2 benzaldéhyde, on obtient, après recristallisation dans 90 $cm^3$ d'un mélange d'isopropanol et d'oxyde d'isopropyle (60 - 40 en volumes), 3,5 g de chlorhydrate de diméthylamino-4 (fluoro-2 phényl)-1 phényl-2 butène-2 ol-1 (Z), sous forme d'une poudre blanche fondant à 175° C.

**EXEMPLE 59**

En opérant d'une manière analogue à celle décrite à l'exemple 22 mais à partir de 20 g de tris-isopropyl-2,4,6 benzène sulfonylhydrazone de la β-diméthylaminopropiophénone et de 5,6 $cm^3$ de bromo-3 benzaldéhyde, on obtient, après recristallisation dans 200 $cm^3$ d'isopropanol, 11,3 g de chlorhydrate de (bromo-3 phényl)-1 diméthylamino-4 phényl-2 butène-2 ol-1 (Z), sous forme d'un solide blanc fondant à 188° C.

**EXEMPLE 60**

En opérant d'une manière analogue à celle décrite à l'exemple 22, mais à partir de 20 g de tris-isopropyl-2,4,6 benzènesulfonylhydrazone de la β-diméthylaminopropiophénone et de 5,78 g de o-tolualdéhyde, on obtient, après recristallisation dans 100 $cm^3$ d'éthanol, 10,2 g de chlorhydrate de diméthylamino-4 (méthyl-2 phényl)-1 phényl-2 butène-2 ol-1 (Z), sous forme d'une poudre blanche fondant à 204° C.

**EXEMPLE 61**

En opérant d'une manière analogue à celle décrite à l'exemple 22, mais à partir de 20 g de tris-isopropyl-2,4,6 benzènesulfonylhydrazone de la β-diméthylaminopropiophénone et de 6,5 g d'o-anisaldéhyde, on obtient, après recristallisation dans 85 $cm^3$ d'un mélange d'éther de pétrole et d'oxyde d'isopropyle (70 - 30 en volumes), 7,2 g de diméthylamino-4 (méthoxy-2 phényl)-1 phényl-2 butène-2 ol-1 (Z), sous forme de poudre blanche fondant à 77° C.

**EXEMPLE 62**

En opérant d'une manière analogue à celle décrite à l'exemple 22 mais à partir de 21,7 g de tris-isopropyl-2,4,6 benzènesulfonylhydrazone de la β-diméthylamino méthoxy-3 propiophénone et de 5,4 $cm^3$ de benzaldéhyde, on obtient, après recristallisation dans 100 $cm^3$ d'isopropanol, 11,9 g de chlorhydrate de diméthylamino-4 (méthoxy-3 phényl)-2 phényl-1 butène-2 ol-1 (Z), sous forme d'un solide blanc fondant à 166° C.

La tris-isopropyl-2,4,6 benzènesulfonylhydrazone de la β-diméthylamino méthoxy-3 propiophénone peut être préparée d'une manière analogue à celle décrite à l'exemple 22 pour la préparation de la tris-isopropyl-2,4,6 benzènesulfonylhydrazone de la β-diméthylaminopropiophénone, mais à partir de 25,3 g de tris-isopropyl-2,4,6 benzènesulfonylhydrazine et de 20,7 g de chlorhydrate de la β-diméthylamino méthoxy-3 propiophénone. On obtient ainsi 26,1 g de chlorhydrate de tris-isopropyl-2,4,6 benzènesulfonyhydrazone de la β-diméthylamino méthoxy-3 propiophénone, sous forme d'un solide blanc fondant à 188° C.

Le chlorhydrate de la β-diméthylamino méthoxy-3 propiophénone peut être préparé par la méthode décrite dans le brevet allemand 2 360 545.

**EXEMPLE 63**

En opérant d'une manière analogue à celle décrite à l'exemple 22, mais à partir de 20 g de tris-isopropyl-2,4,6 benzènesulfonylhydrazone de la β-diméthylaminopropiophénone et de 3,37 g de cyclopropanecarboxaldéhyde, on obtient, après évaporation à sec sous pression réduite (2,7 kPa) à 30°C des phases organiques, une huile orange qui est parifiée par chromatographie "flash" [éluant: acétate d'éthyle - méthanol (80 - 20 en volumes)]. Après évaporation à sec sous pression réduite (2,7 kPa) à 30°C des fractions 20 à 80, on obtient une huile. Cette huile est dissoute dans 50 $cm^3$ d'acétone; à la solution obtenue, on ajoute 3,93 g d'acide oxalique en solution dans 50 $cm^3$ d'acétone. Le solide formé est séparé par filtration puis recristallisé dans 80 $cm^3$ d'acétonitrile. On obtient ainsi 7,2 g d'oxalate de cyclopropyl-1 diméthylamino-4 phényl-2 butène-2 ol-1 (Z), sous forme d'une poudre blanche fondant à 127°C.

Le cyclopropanecarboxaldéhyde peut être préparé selon la méthode décrite par A.J. MANCUSO, D.S. BROWNFAIR, D. SWERN, J. Org. Chem. 23, 4148, (1979).

**EXEMPLE 64**

En opérant d'une manière analogue à celle décrite à l'exemple 22, mais à partir de 20 g de tris-isopropyl-2,4,6 benzènesulfonylhydrazone de la β-diméthylaminopropiophénone et de 6,4 $cm^3$ d'α-tétralone, on obtient, après recristallisation dans 65 $cm^3$ d'un mélange d'éther de pétrole et d'isopropanol (95 - 5 en volumes), 3,8 g d'[hydroxy-1 tétrahydro-1,2,3,4 naphtyl-1]-1 diméthylamino-3 phényl-1 propène-1 (Z), sous forme d'une poudre blanche fondant à 108°C.

**EXEMPLE 65**

En opérant d'une manière analogue à celle décrite à l'exemple 22, mais à partir de 20 g de tris-isopropyl-2,4,6 benzènesulfonylhydrazone de la β-diméthylaminopropiophénone et de 5 $cm^3$ de cyclohexanone, on obtient, après recristallisation dans 50 $cm^3$ d'un mélange d'isopropanol et d'oxyde d'isopropyle (60 - 40 en volumes) 6 g de chlorhydrate d'(hydroxy-1 cyclohexyl-1)-1 diméthylamino-3 phényl-1 propène-1 (Z), sous forme d'une poudre blanche fondant à 197°C.

**EXEMPLE 66**

En opérant d'une manière analogue à celle décrite à l'exemple 22, mais à partir de 20 g de tris-isopropyl-2,4,6 benzènesulfonylhydrazone de la β-diméthylaminopropiophénone et de 3,47 g d'isobutyraldéhyde, on obtient, après recristallisation dans 150 $cm^3$ d'acétone, 5,6 g de chlorhydrate de diméthylamino-1 méthyl-5 phényl-3 hexène-2 ol-4 (Z), sous forme d'une poudre blanche fondant à 163°C.

**EXEMPLE 67**

En opérant d'une manière analogue à celle décrite à l'exemple 22, mais à partir de 13 g de tris-isopropyl-2,4,6 benzènesulfonylhydrazone de 1'α-(méthyl-1 pyrrolidinyl-2) acétophénone et de 3,5 $cm^3$ de benzaldéhyde, on obtient, après recristallisation dans 100 $cm^3$ d'éthatol, 3,45 g de chlorhydrate de diphényl-1,2 (méthyl-1 pyrrolidinyl-2)-3 propène-2 ol-1 (Z), sous forme d'un solide blanc fondant à 240°C.

La tris-isopropyl-2,4,6 benzènesulfonylhydrazone de 1'α-(méthyl-1 pyrrolidinyl-2) acétophénone peut être préparée d'une manière analogue à celle décrite à l'exemple 22 pour la préparation de la tris-isopropyl-2,4,6 benzènesulfonylhydrazone de la β-diméthylaminopropiophénone mais à partir de 30,6 g de tris-isopropyl-2,4,6 benzènesulfonylhydrazine et de 28,3 g de chlorhydrate de l'α-(méthyl-1 pyrrolidinyl-2) acétophénone. On obtient ainsi 31,1 g de chlorhydrate de tris-isopropyl-2,4,6 benzènesulfonylhydrazone de l'α-(méthyl-1 pyrrolidinyl-2) acétophénone, sous forme d'un solide blanc fondant à 210°C.

Le chlorhydrate de l'α-(méthyl-1 pyrrolidinyl-2) acétophénone peut être préparé suivant la méthode décrite par A. S. RADWAN, F. R. MELEK, S. NEGM, J. Prakt. Chem., (1980), 322, 475.

**EXEMPLE 68**

A une solution de 10,9 g de tris-isopropyl-2,4,6 benzènesulfonylhydrazone de la β-diméthylamino α-méthyl propiophénone dans 109 $cm^3$ de diméthoxy-1,2 éthane, maintenue sous atmosphère d'azote à une température voisine de -75°C, on ajoute 30,4 $cm^3$ d'une solution 1,9 M de tert-butyllithium dans le pentane. Une fois l'addition terminée, le mélange réactionnel est réchauffé lentement jusqu'à 15°C. Lorsque le dégagement gazeux est terminé, le mélange réactionnel est refroidi jusqu'à une température voisine de 40°C. On ajoute alors 4,2 g de benzaldéhyde et on poursuit l'agitation pendant 20 minutes en laissant remonter la température jusqu'à environ -5°C. On ajoute alors successivement 50 $cm^3$ d'eau distillée, 7 $cm^3$ d'une solution aqueuse d'acide chlorhydrique concentrée, puis 200 $cm^3$ d'éther éthylique. La phase organique est séparée par décantation puis extraite par 2 fois 25 $cm^3$ d'une solution aqueuse d'acide chlorhydrique 4N. Les phases aqueuses sont réunies, alcalinisées par une solution aqueuse 10N de soude jusqu'à un pH voisin de 10 puis extraites par 3 fois 150 $cm^3$ d'acétate d'éthyle. Les phases organiques sont réunies, séchées sur du sulfate de magnésium, filtrées, puis concentrées à sec sous pression réduite (2,7 kPa) à 30°C. L'huile orange obtenue est dissoute dans 91 $cm^3$ d'acétone; à la solution obtenue, on ajoute 4,5 $cm^3$ d'une solution 5,6N d'acide chlorhydrique gazeux dans l'éther éthylique. Le solide formé est séparé par filtration puis recristallisé dans 48 $cm^3$ d'un mélange d'isopropanol et d'oxyde d'isopropyle (80 - 20 en volumes). On obtient ainsi 1,85 g de chlorhydrate de diméthylamino-4 diphényl-1,2 méthyl-3 butène-2 ol-1 (Z), sous forme d'une poudre blanche fondant à 212°C.

La tris-isopropyl- 2,4,6 benzènesulfonylhydrazone de la B-diméthylamino α-méthylpropiophénone peut être préparée de la manière suivante: On ajoute 23,8 g de tris-isopropyl-2,4,6 benzènesulfonylhydrazine à 16,3 g de chlorhydrate d'α-méthyl β-diméthylaminopropiophénone en solution dans 150 $cm^3$ de chlorure de méthylène et 5 $cm^3$ d'acide acétique. La solution est agitée à température ambiante pendant 24 heures puis est versée sur 50 $cm^3$ d'eau. La phase aqueuse est extraite avec 2 fois 50 $cm^3$ de chlorure de méthylène puis les phases organiques sont rassemblées, séchées sur sulfate de magnésium, filtrées et évaporées à sec sous pression réduite (2,7 kPa) à 40°C pour donner une huile jaune qui est cristallisée dans 120 $cm^3$ d'un mélange d'acétate d'éthyle et d'oxyde d'isopropyle (50 - 50 en volumes). On obtient ainsi 16 g de chlorhydrate de tris-isopropyl-2,4,6 benzènesulfonylhydrazone de la β-diméthylamino α-méthylpropiophénone, sous forme d'un solide blanc fondant à 147°C.

La base peut être libérée de son chlorhydrate de la façon suivante: Le chlorhydrate obtenu précédemment est dissous dans 100 $cm^3$ d'eau puis la solution obtenue est additionnée d'une solution aqueuse de soude 4N jusqu'à pH 10 puis extraite avec 3 fois 75 $cm^3$ d'acétate d'éthyle. Les phases organiques sont rassemblées, séchées sur sulfate de magnésium, filtrées puis évaporées à sec sous pression réduite (2,7 kPa) à 40°C. On obtient ainsi 13 g de tris-isopropyl-2,4,6 benzènesulfonylhydrazone de la β-diméthylamino α-méthylpropiophénone, à l'état de base, sous forme d'un solide blanc fondant à 113°C.

La β-diméthylamino α-méthylpropiophénone peut être préparée par la méthode décrite par S. MIYANO, H. HOKARI et H. HASHIMOTO, Bull. Chem. Soc. Japan, 55, 534, (1982).

**EXEMPLE 69**

A une solution de 9,5 g de chloro-4 éthoxycarbonyloxy-1 (fluoro-3 phényl)-1 phényl-2 butène-2 (Z) dans 50 $cm^3$ d'éthanol, on ajoute 100 $cm^3$ d'une solution éthanolique à 33 % de méthylamine. Après 2 heures d'agitation à l'abri de la lumière, le mélange réactionnel est évaporé sous pression réduite (2,7 kPa) à 30°C pour donner une huile. Cette huile est dissoute dans 50 $cm^3$ d'eau distillée et la solution obtenue est alcalinisée à pH 11 par addition d'une solution de soude 4N puis extraite avec 3 fois 100 $cm^3$ d'éther éthylique. Les phases organiques sont réunies, séchées sur sulfate de magnésium, filtrées puis évaporées à sec sous pression réduite (2,7 kPa) à 30°C pour donner une huile orange. Cette huile est dissoute dans 40 $cm^3$ d'acétate d'éthyle puis est additionnée de 5 $cm^3$ d'une solution 5,7N d'acide chlorhydrique gazeux dans l'éther éthylique. Le solide formé est séparé par filtration puis recristallisé dans 20 $cm^3$ d'acétonitrile. On obtient ainsi 3 g de chlorhydrate de (fluoro-3 phényl)-1 méthylamino-4 phényl-2 butène-2 ol-1 (Z), sous forme d'un solide blanc fondant à 167°C.

Le chloro-4 éthoxycarbonyloxy-1 (fluoro-3 phényl)-1 phényl-2 butène-2 (Z) peut être obtenu comme décrit à l'exemple 52 pour la préparation du chloro-4 diphényl-1,2 éthoxycarbonyloxy-1 butène-2 mais à partir de 26,2 g de diméthylamino-1 éthoxycarbonyloxy-4 (fluoro-3 phényl)-4 phényl-3 butène-2 (Z) et de 10,6 $cm^3$ de chloroformiate d'éthyle. Après chromatographie "flash" [éluant: cyclohexane - acétate d'éthyle (90 - 10 en volumes)] et évaporation à sec sous pression réduite (2,7 kPa) à 30°C des fractions 12 à 18, on obtient 21,1 g de chloro-4 éthoxycarbonyloxy-1 (fluoro-3 phényl)-1 phényl-2 butène-2 (Z), sous forme d'une huile jaune pâle [Rf = 0,5; éluant: cyclohexane - acétate d'éthyle (50 - 50 en volumes)].

Le chlorhydrate de diméthylamino-1 éthoxycarbonyloxy-4 (fluoro-3 phényl)-4 phényl-3 butène-2 (Z) peut être obtenu comme décrit à l'exemple 49 pour la préparation du chlorhydrate de diméthylamino-4 éthoxycarbonyloxy-1 diphényl-1,2 butène-2 (Z) mais à partir de 12,6 g de diméthylamino-4 (fluoro-3 phényl)-4 phényl-3 butène-2 ol-1 (Z) et de 5,1 $cm^3$ de chloroformiate d'éthyle. On obtient ainsi 7,7 g de chlorhydrate de diméthylamino-1 éthoxycarbonyloxy-4 (fluoro-3 phényl)-4 phényl-3 butène-2 (Z), sous forme d'un solide beige fondant à 160°C.

**EXEMPLE 70**

En opérant d'une manière analogue à celle décrite à l'exemple 35, mais à partir de 5 g de diméthylamino-3 acrylophénone, de 2,5 $cm^3$ d'oxychlorure de phosphore, de 4,13 g de thiophénol et de 1 g de cyanoborohydrure de sodium et après évaporation à sec sous pression réduite (2,7 kPa) à 40°C de la phase organique, on obtient une huile jaune que l'on dissout dans 35 $cm^3$ d'un mélange d'éther éthylique et d'éthanol (70 - 30 en volumes). A la solution obtenue, on ajoute 2,2 g d'acide oxalique. Le précipité formé est séparé par filtration et recristallisé dans 60 $cm^3$ d'éthanol. On obtient ainsi 5,8 g d'oxalate de diméthylamino-1 (fluoro-4 phénylthio)-3 phényl-3 propène-2 (Z) (contenant 25 % de son isomère E), sous forme d'un solide blanc fondant à 133°C.

**EXEMPLE 71**

En opérant d'une manière analogue à celle décrite à l'exemple 35, mais à partir de 6,1 g de diméthylamino-3 acrylophénone, de 3 $cm^3$ d'oxychlorure de phosphore, de 5 g de chloro-2 thiophénol et de 1,3 g de cyanoborohydrure de sodium, et après évaporation à sec sous pression réduite (2,7 kPa) à 40°C de la phase organique, on obtient une huile jaune que l'on dissout dans 20 $cm^3$ d'isopropanol. A cette solution, on ajoute 7,8 $cm^3$ d'une solution 5,6N d'acide chlorhydrique gazeux dans l'éther éthylique. Le précipité formé est séparé par filtration puis recristallisé dans 20 $cm^3$ d'isopropanol. On obtient ainsi 2,5 g de chlorhydrate de (chloro-2 phénylthio)-3 diméthylamino-1 phényl-3 propène-2 (Z), sous forme d'un solide blanc fondant à 162°C.

**EXEMPLE 72**

En opérant d'une manière analogue à celle décrite à l'exemple 35, mais à partir de 5 g de diméthylamino-3 acrylophénone, de 2,5 $cm^3$ d'oxychlorure de phosphore, de 4,13 g de chloro-3 thiophénol et de 1 g de cyanoborohydrure de sodium, et après évaporation à sec sous pression réduite (2,7 kPa) à 40°C de la phase organique, on obtient un résidu que l'on dissout dans 20 $cm^3$ d'un mélange d'acétone et d'éther éthylique (50 - 50 en volumes). A cette solution, on ajoute 6 $cm^3$ d'une solution 5,6N d'acide chlorhydrique gazeux dans l'éther éthylique. Le précipité formé est séparé par filtration puis recristallisé dana 30 $cm^3$ d'isopropanol. On obtient ainsi 2,6 g de chlorhydrate de (chloro-3 phénylthio)-3 diméthylamino-1 phényl-3 propène-2 (Z), sous forme d'un solide blanc fondant à 156 - 157°C.

**EXEMPLE 73**

En opérant d'une manière analogue à celle décrite à l'exemple 35, mais à partir de 5 g de diméthylamino-3 acrylophénone, de 2,47 $cm^3$ d'oxychlorure de phosphore, de 3,55 g de méthyl-4 thiophénol et de 1 g de cyanoborohydrure de sodium, et après évaporation à sec sous pression réduite (2,7 kPa) à 40°C de la phase organique, on obtient une huile orangée que l'on dissout dans 40 $cm^3$ d'isopropanol. A cette solution, on ajoute 7 $cm^3$ d'une solution 5,6N d'acide chlorhydrique gazeux dans l'éther éthylique. Le précipité formé est séparé par filtration puis recristallisé dans 40 $cm^3$ d'isopropanol. On obtient ainsi 3,3 g de chlorhydrate de diméthylamino-1 (méthyl-4 phénylthio)-3 phényl-3 propène-2 (Z), sous forme d'un solide blanc fondant à 197 - 198°C.

**EXEMPLE 74**

En opérant d'une manière analogue à celle décrite à l'exemple 50, mais à partir de 45 g de chloro-3 phényl-1 phénylthio-1 propène-1 (Z) et de 143 $cm^3$ d'une solution méthanolique de méthylamine à 33 %, on obtient, après évaporation à sec sous pression réduite (2,7 kPa) à 30°C, une huile orange qui est dissoute dans 150 $cm^3$ d'acétate d'éthyle. On ajoute à cette solution 18,5 $cm^3$ d'une solution d'acide chlorhydrique gazeux 5,6N dans l'éther éthylique. Le précipité formé est séparé par filtration puis recristallisé dans 70 $cm^3$ d'isopropanol. On obtient ainsi 12,8 g de chlorhydrate de méthyl-amino-1 phényl-3 phénylthio-3 propène-2 (Z) sous forme d'un solide blanc fondant à 141°C.

**EXEMPLE 75**

A une solution de 5,1 g de bromo-3 (chloro-3 phényl)-3 diméthylamino-1 propène-2 (Z) dans 50 $cm^3$ de pentane maintenue sous atmosphère d'azote à une température voisine de -78°C, on ajoute une solution de 20 $cm^3$ de n-butyllithium 1,5N dans l'hexane et on agite pendant 25 minutes à -50°C. On ajoute alors 4 $cm^3$ de benzaldéhyde en solution dans 6 $cm^3$ de pentane et on poursuit l'agitation pendant 30 minutes en laissant la température remonter à 25°C. On ajoute alors 80 $cm^3$ d'eau distillée puis 30 $cm^3$ d'une solution aqueuse d'acide chlorhydrique 2N. La phase aqueuse est séparée par décantation, lavée avec 3 fois 25 $cm^3$ d'éther éthylique, neutralisée avec une solution aqueuse saturée de bicarbonate de sodium, puis extraite avec 3 fois 50 $cm^3$ d'éther éthylique. Les phases organiques sont réunies, séchées sur sulfate de magnésium, filtrées et concentrées à sec sous pression réduite (2,7 kPa) à 40°C. L'huile obtenue est dissoute dans 30 $cm^3$ d'éthanol. A cette solution, on ajoute 5,7 $cm^3$ d'une solution 5,7N d'acide chlorhydrique gazeux dans l'éther éthylique. Le solide formé est séparé par filtration puis recristallisé dans 80 $cm^3$ d'isopropanol. On obtient ainsi 3,7 g de chlorhydrate de (chloro-3 phényl)-2 diméthylamino-4 phényl-1 butène-2 ol-1 (Z), sous forme d'un solide blanc fondant à 199°C.

Le bromo-3 (chloro-3 phényl)-3 diméthylamino-1 propène-2 (Z) peut être préparé d'une manière analogue à celle décrite à l'exemple 16, mais à partir de 44 g de (chloro-3 phényl)-1 diméthyl-amino-3 propène-2 one-1, de 60 g d'oxybromure de phosphore et de 7,9 g de cyanoborohydrure de sodium. Après évaporation à sec sous pression réduite (2,7 kPa) à 30°C du mélange réactionnel, on obtient un solide beige qui est repris par 400 $cm^3$ de chloroforme. La suspension obtenue est filtrée et le filtrat est évaporé à sec sous pression réduite (2,7 kPa) à 30°C. Après lavage du produit ainsi obtenu avec 75 $cm^3$ d'isopropanol, on obtient 46 g de bromhydrate de bromo-3 (chloro-3 phényl)-3 diméthylamino-1 propène-2 (Z), sous forme d'un solide blanc fondant à 146°C.

La (chloro-3 phényl)-1 diméthylamino-3 propène-2 one-1 peut être préparée par la méthode décrite dans le brevet américain 4 209 621.

**EXEMPLE 76**

En opérant d'une manière analogue à celle décrite à l'exemple 75, mais à partir de 5,1 g de bromo-3 (chloro-3 phényl)-3 diméthyl-amino-1 propène-2 (Z), de 15 $cm^3$ d'une solution de n-butyllithium 1,5 M dans l'hexane et de 5 g de dichloro-3,5 benzaldéhyde, on obtient, après recristalliaation dans 100 $cm^3$ d'isopropanol, 2,1 g de chlorhydrate de (chloro-3 phényl)-2 (dichloro-3,5 phényl)-1 diméthyl-amino-4 butène-2 ol-1 (Z), sous forme d'un solide blanc fondant à 215°C.

**EXEMPLE 77**

En opérant d'une manière analogue à celle décrite à l'exemple 75, mais à partir de 10,2 g de bromo-3 (chloro-3 phényl)-3 diméthylamino-1 propène-2 (Z), de 40 $cm^3$ d'une solution de n-butyllithium 1,5 M dans l'hexane et de 9,9 g de fluoro-3 benzaldéhyde, on obtient, après recristallisation dans 85 $cm^3$ d'isopropanol, 6,6 g de chlorhydrate de (chloro-3 phényl)-2 diméthylamino-4 (fluoro-3 phényl)-1 butène-2 ol-1 (Z), sous forme d'un solide blanc fondant à 200°C.

**EXEMPLE 78**

En opérant d'une manière analogue à celle décrite à l'exemple 16, mais à partir de 34 g de bromo-3 (chloro-2 phényl)-3 diméthylamino-1 propène-2 (Z) brut (contenant de l'isomère E), de 90 $cm^3$ d'une solution de n-butyllithium 1,5 M dans l'hexane, de 16 $cm^3$ de benzaldéhyde et après purification par chromatographie "flash" [éluant: acétate d'éthyle - méthanol (50 - 50 en volumes)] et concentration à sec des fractions 18 à 25 sous pression réduite (2,7 kPa) à 40°C, on obtient un résidu que l'on dissout dans 20 $cm^3$ d'acétate d'éthyle. A cette solution, on ajoute 4 $cm^3$ d'une solution 5,6N d'acide chlorhydrique gazeux dans l'éther éthylique. Le précipité formé est séparé par filtration puis recristallisé dans 70 $cm^3$ d'un mélange d'acétonitrile et d'acétate d'éthyle (43 - 57 en volumes). On obtient ainsi 2,9 g de chlorhydrate de (chloro-2 phényl)-2 diméthyl-amino-4 phényl-1 butène:2 ol-1 (Z), sous forme d'un solide blanc fondant à 168°C.

Le bromo-3 (chloro-2 phényl)-3 diméthylamino-1 propène-2 (Z) peut être préparé d'une manière analogue à celle décrite à l'exemple 16, mais à partir de 35,2 g de (chloro-2 phényl)-1 diméthylamino-3 propène-2 one-1, de 48,4 g d'oxybromure de phosphore et de 6,35 g de cyanoborohydrure de sodium. Après évaporation à sec sous pression réduite (2,7 kPa) à 3 C° du mélange réactionnel, on obtient un solide qui est repris avec 400 $cm^3$ de chloroforme. La suspension obtenue est filtrée. Le filtrat est additionnée de 100 $cm^3$ d'eau destillée et décanté. La phase aqueuse est alcalinisée à pH 9 par une solution aqueuse de soude concentrée, puis extraite avec 2 fois 100 $cm^3$ de chloroforme. Les phases chloroformiques sont réunies, séchées sur sulfate de magnésium,

filtrées puis évaporées à sec sous pression réduite (2,7 kPa) à 30°C. On obtient ainsi 34 g d'un mélange d'isomères Z et E du bromo-3 (chloro-2 phényl)-3 diméthylamino-1 propène-2, sous forme d'une huile brune [Rf = 0,68; éluant: dichlorométhane - méthanol (95 - 5 en volumes)].

La (chloro-2 phényl)-1 diméthylamino-3 propène-2 one-1 peut être préparée par la méthode décrite dans le brevet américain 4 209 621.

**EXEMPLE 79**

A une solution de 22,7 g d'azido-4 diphényl-1,2 butène-2 ol-1 (mélange de 75 % d'isomère Z et 25 % d'isomère E) dans 370 $cm^3$ de méthanol, on ajoute 60 $cm^3$ de triéthylamine et 43 $cm^3$ de propanedithiol-1,3. Le mélange est agité sous atmosphère d'azote pendant 20 heures à une température voisine de 20°C puis est versé sur 600 $cm^3$ d'eau distillée. Le mélange est ajusté à pH 12 avec une solution aqueuse de soude 10N puis extrait 3 fois avec 500 $cm^3$ de chlorure de méthylène. La phase organique est séchée sur du sulfate de magnésium, filtrée puis concentrée à sec sous pression réduite (2,7 kPa) à 30°C. L'huile obtenue est agitée avec du chlorure de méthylène et le précipité formé est séparé par filtration. Le filtrat obtenu est concentré à sec sous pression réduite (2,7 kPa) à 30°C pour donner une huile jaune qui est purifiée par chromatographie sur gel de silice [éluant: méthanol - acétate d'éthyle - ammoniaque 11N (60 - 38 - 2 en volumes)]. On élue d'abord avec 460 $cm^3$ d'éluant; l'éluat correspondant est éliminé. On élue ensuite avec 360 $cm^3$ d'éluant; l'éluat correspondant est évaporé à sec sous pression réduite (2,7 kPa) à 30°C pour donner un solide jaune que l'on dissout dans 130 $cm^3$ d'un mélange d'éthanol et d'éther éthylique [(25 - 75 en volumes)]. A la solution obtenue, on ajoute 4,8 $cm^3$ d'une solution 3,5N d'acide chlorhydrique gazeux dans l'éther éthylique. Le solide blanc formé est séparé par filtration puis recristallisé dans un mélange de 11 $cm^3$ d'éthanol et de 16 $cm^3$ d'oxyde d'isopropyle. On obtient ainsi 1,88 g de chlorhydrate d'amino-4 diphényl-1,2 butène-2 ol-1 (Z), sous forme d'une poudre blanche fondant à 180°C.

L'azido-4 diphényl-1,2 butène-2 ol-1 (mélange de 75 % d'isomère-Z et 25 % d'isomère E) peut être préparé de la manière suivante: A une solution de 28,5 g d'azido-4 diphényl-1,2 éthoxy-carbonyloxy-1 butène-2 (Z) préparé comme décrit à l'exemple 53, dans 300 $cm^3$ de méthanol, on ajoute 117 g de carbonate de potassium. Le mélange est agité pendant 16 heures à l'abri de la lumière à une température voisine de 20°C, puis filtré. Le filtrat est concentré à sec sous pression réduite (2,7 kPa) à 30°C. L'huile obtenue est dissoute dans 300 $cm^3$ de chlorure de méthylène puis lavée avec 200 $cm^3$ d'eau distillée. La phase aqueuse est ajustée à pH 7 par addition d'une solution aqueuse d'acide chlorhydrique 2N puis extraite avec 2 fois 300 $cm^3$ de chlorure de méthylène. Les phases organiques sont réunies, séchées sur du sulfate de magnésium, filtrées puis concentrées à sec sous pression réduite (2,7 kPa) à 30°C. On obtient ainsi 21,77 g d'azido-4 diphényl-1,2 butène-2 ol-1 (mélange de 75 % d'isomère Z et 25 % d'isomère E), sous forme d'huile jaune pâle [Rf = 0,6; éluant: cyclohexane - acétate d'éthyle (70 - 30 en volumes)].

La présente invention concerne également les médicaments constitués par un produit de formule générale (1), sous forme libre ou sous forme de sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale ou rectale.

Comme compositions solides pour administration orale peuvent être utilisés des comprimés, pilules, poudres (notamment dans des capsules de gélatine ou des cachets) ou granulés. Dans ces compositions, le produit actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tel que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent également comprendre des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuveut être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des

polyéthyléneglycols.

En thérapeutique humaine, les produits selon l'invention sont particulièrement utiles dans le traitement des syndromes des états dépressifs divers et des états psychasthéniques. Les doses dépendent de l'effet recherché et de la durée du traitement; elles sont généralement comprises entre 25 et 250 mg par jour par voie orale, intramusculaire ou intraveineuse pour un adulte en une ou plusieurs prises.

D'une façon générale, le médecin déterminera la posologie qu'il estime la plus appropriée en fonction de l'age, du poids et de tous les autres facteurs propres au sujet à traiter.

Les exemples suivants, donnés à titre non limitatif, illustrent des compositions selon l'invention.

**Exemple A**

On prépare, selon la technique habituelle, des comprimés dosés à 25 mg de produit actif ayant la composition suivante:

| | |
|---|---|
| - Chlorhydrate de diméthylamino-4 diphényl-1,2 butène-2 ol-1 (Z) | 32 mg |
| - Amidon | 60 mg |
| - Lactose | 50 mg |
| - Stéarate de magnésium | 2 mg |

**Exemple B**

On prépare une solution injectable contenant 25 mg de produit actif ayant la composition suivante:

| | |
|---|---|
| - Méthylamino-4 diphényl-1,2 butène-2 ol-1 (Z) | 25 mg |
| - Acide chlorhydrique 1N | 0,1 $cm^3$ |
| - Soluté injectable | q.s.p. 2 $cm^3$ |

**Revendications** pour les etats contractants. BE, CH, DE, FR, GB, IT, LI, LU, NL, SE.

1. Nouveau dérivé de la phényl-3 propène-2 amine, caractérisé en ce qu'il répond à la formule générale:

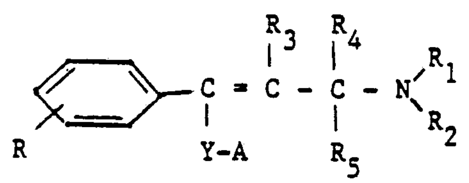
(I)

dans laquelle R reprėsete un atome d'hydrogène ou d'halogène ou un radical alcoyle, alcoyloxy, alcoylthio, amino, alcoylamino, dialcoylamino ou trifluorométhyle, $R_3$ représente un atome d'hydrogène ou un radical alcoyle,

- soit $R_4$ et $R_5$ représentent un atome d'hydrogène et $R_1$ et $R_2$, identiques ou différents, représentent un atome d'hydrogène ou un radical alcoyle éventuellement substitué par un radical alcényle contenant 2 à 4 atomes de carbone, ou bien $R_1$ et $R_2$ forment ensemble avec l'atome d'azote auquel ils sont liés un radical hétérocyclique saturé contenant 4 à 7 chaînons et contenant éventuellement un autre hétéroatome tel que l'oxygène, le soufre ou l'azote éventuellement substitué par un radical alcoyle,

- soit $R_4$ représente un atome d'hydrogène, $R_1$ représente un atome d'hydrogène ou un radical alcoyle et $R_2$ et $R_5$ forment ensemble un radical alcoylène contenant 3 ou 4 atomes de carbone et i) ou bien A représente un radical alcoyle ou un radical phényle non substitué ou substitué par un ou deux substituants choisis parmi les atomes d'halogène et les radicaux alcoyle, alcoyloxy, alcoylthio, amino, alcoylamino, dialcoylamino, nitro ou trifluorométhyle ou bien représente un radical pyridyle, benzyle ou cycloalcoyle contenant 3 à 6 atomes de carbone, Y représente un atome de soufre, un radical sulfinyle ou sulfonyle ou encore un radical de formule générale:

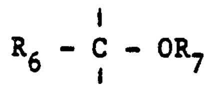

(II)

dans laquelle $R_6$ représente un atome d'hydrogène ou un radical alcoyle et $R_7$ représente un atome d'hydrogène ou un radical alcoylcarbonyle, alcoyloxycarbonyle, alcoylaminocarbonyle ou benzoyle éventuellement substitué par un ou deux substituants choisis parmi les atomes d'halogène et les radicaux alcoyle, alcoyloxy, alcoylthio, amino, alcoylamino, dialcoylamino, nitro ou trifluorométhyle, ii) ou bien Y et A forment ensemble un radical hydroxy-1 cycloalcoyle dont le cycle contient 5 ou 6 atomes de carbone, éventuellement accolé à un cycle benzénique,

- ainsi que ses sels pharmaceutiquement acceptables, étant entendu que dans les définitions qui précèdent les radicaux alcoyle et portions alcoyle contiennent 1 à 4 atomes de carbone en chaîne droite ou ramifiée et que l'invention concerne tous les isomères géométriques et optiques possibles ainsi que leurs mélanges.

2. Procédé de préparation d'un produit selon la revendication 1 dans la formule duquel Y représente un radical de formule générale (II) dans laquelle $R_6$ est défini comme à la revendication 1 et $R_7$ représente un atome d'hydrogène et A, R, $R_1$ et $R_2$ sont définis comme à la revendication 1 à l'exception pour $R_1$ et/ou $R_2$ de représenter un atome d'hydrogène, et qui se présete sous la configuration Z, caractérisé en ce que l'on fait agir un produit de formule générale:

$A - CO - R_6$ (III)

dans laquelle A et $R_6$ sont définis comme à la revendication 1 sur un carbanion de formule générale:

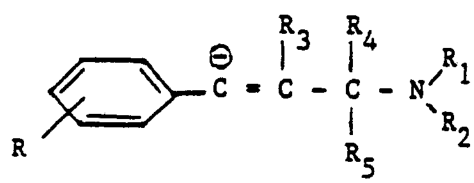

(IV)

dans laquelle les symboles sont définis comme à la revendication 1 à l'exception pour $R_1$ et/ou $R_2$ de représenter un atome d'hydrogène, puis isole le produit obtenu et le transforme éventuellement en un sel pharmaceutiquement acceptable.

3. Procédé de préparation d'un produit selon la revendication 1 dans la formule duquel Y représente un radical de formule générale (II) dans laquelle $R_6$ et $R_7$ représentent un atome d'hydrogène, A est défini comme à la revendication 1 en i), $R_1$ représente un atome d'hydrogène, $R_2$ représente un atome d'hydrogène ou un radical alcoyle éventuellement substitué par un radical alcényle contenant 2 à 4 atomes de carbone, R représente un atome d'hydrogène, $R_3$ est défini comme à la revendication 1 et $R_4$ et $R_5$ représentent un atome d'hydrogène, et qui se présente sous la configuration Z, caractérisé en ce que l'on ouvre le cycle dihydrofuranne dans un produit de formule générale:

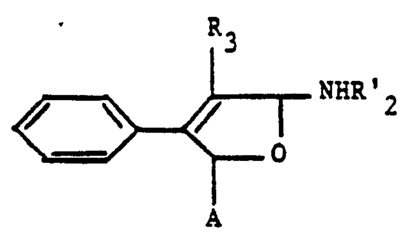

(XIV)

dans laquelle A est défini comme à la revendication 1 en i) et $R'_2$ représente un radical alcoyle éventuellement substitué par un radical alcényle contenant 2 à 4 atomes de carbone, et $R_3$ est défini comme précédemment, puis isole le produit obtenu et le transforme éventuellement en un sel pharmaceutiquement acceptable.

4. Procédé de préparation d'un produit selon la revendication 1 dans la formule duquel Y représente un atome de soufre, $R_4$ et $R_5$ représentent un atome d'hydrogène, A est défini comme à la revendication 1 en i) et les autres symboles sont définis comme à la revendication 1 à l'exception pour $R_1$ et/ou $R_2$ de représenter un atome d'hydrogène et qui se présente sous la configuration Z ou E, caractérisé en ce que l'on fait agir un mercaptan de formule générale:

$A - SH$ (XVII)

dans laquelle A est défini comme à la revencation 1 en i), sur un produit de formule générale:

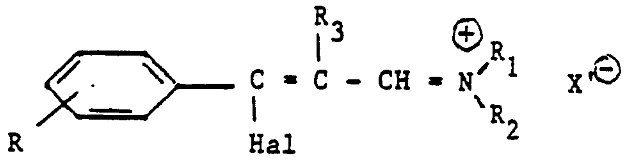

(XVIII)

dans laquelle R, $R_1$, $R_2$ et $R_3$ sont définis comme à la revendication 1 à l'exception pour $R_1$ et/ou $R_2$ de représenter un atome d'hydrogène, Hal représente un atome d'halogène et $X'^{\ominus}$ représente un anion, puis l'on réduit l'intermédiaire obtenu de formule générale:

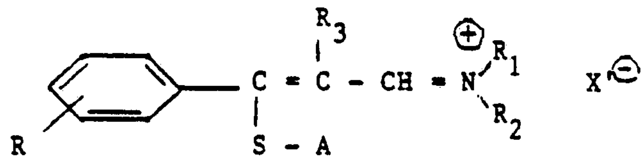

(XIX)

dans laquelle les symboles ont les définitions correspondantes, puis isole le produit obtenu et le transforme en un sel pharmaceutiquement acceptable.

5. Procédé de préparation d'un produit selon la revendication 1 dans la formule duquel Y représente un atome de soufre, $R_4$ et $R_5$ représentent un atome d'hydrogène, A est défini comme à la revendication 1 en i) et les autres symboles sont définis comme à la revendication 1, et qui se présentent sous la configuration E ou Z, caractérisé en ce que l'on fait agir l'ammoniac ou une amine de formule générale:

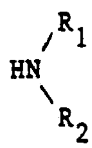

(XX)

dans laquelle $R_1$ et $R_2$ sont définis comme à la revendication 1, sur un aldéhyde de formule générale:

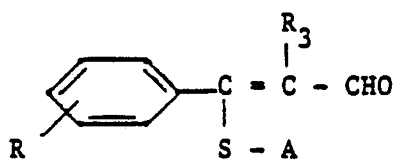

(XXI)

dans laquelle A est défini comme à la revendication 1 en i) et R et $R_3$ sont définis comme à la revendication 1, et qui se présente sous la configuration E ou Z, puis isole le produit obtenu et le transforme éventuellement en un sel thérapeutiquement acceptable.

6. Procédé de préparation d'un produit selon la revendication 1 dans la formule duquel Y représente un radical de formule générale (II) dans laquelle $R_7$ représente un atome d'hydrogène ou bien Y est défini comme à la revendication 1 en ii), $R_3$ représente un atome d'hydrogène et les autres symboles sont définis comme à la revendication 1, caractérisé en ce que l'on fait agir un dérivé organomagnésien de formule générale:

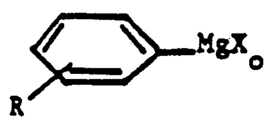

(XXIII)

dans laquelle R est défini comme à la revendication 1 et $X_o$ représente un atome d'halogène, sur un produit de formule générale:

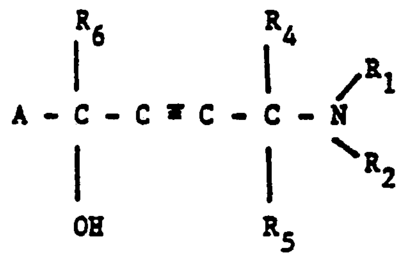

(XXIV)

dans laquelle les symboles sont définis comme à la revendication 1, puis isole le produit obtenu et le transforme éventuellement en un sel pharmaceutiquement acceptable.

7. Procédé de préparation d'un produit selon la revendication 1 dans la formule duquel Y représente un radical de formule générale (II) dans laquelle $R_7$ représente un radical alcoylcarbonyle, alcoyloxycarbonyle ou benzoyle et les autres symboles sont définis comme à la révendication 1, caractérisé en ce que l'on fait agir un produit de formule générale:

$R'_7X_1$ (XXVII)

dans laquelle $R'_7$ représente un radical alcoylcarbonyle, alcoyloxycarbonyle ou benzoyle et $X_1$ représente un reste d'ester réactif, sur un produit selon la revendication 1 dans la formule duquel Y représente un radical de formule générale (II) dans laquelle $R_7$ représente un atome d'hydrogène et les autres symboles sont définis comme à la revendication 1, c'est-à-dire un produit de formule générale:

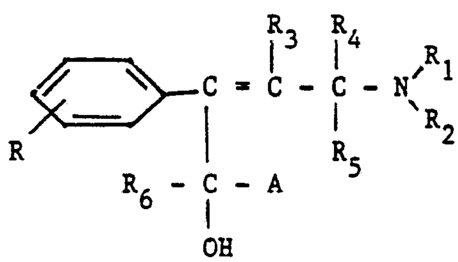

(XXVIII)

puis isole le produit obtenu et le transforme éventuellement en un sel pharmaceutiquement acceptable.

8. Procédé de préparation d'un produit selon la revendication 1 dans la formule duquel Y représente un atome de soufre ou un radical de formule générale (II) défini comme à la revendication 1, $R_4$ et $R_5$ représentent un atome d'hydrogène et les autres symboles sont définis comme à la revendication 1, et qui se présente sous la configuration E ou Z, caractérisé en ce que l'on fait agir l'ammoniac, un précurseur d'amine ou une amine de formule générale:

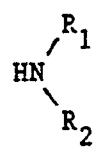

(XX)

sur un produit de formule générale:

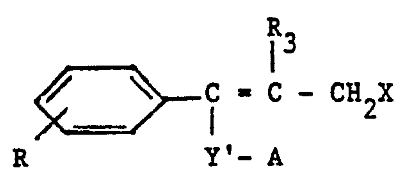

(XXIX)

dans laquelle A est défini comme à la revendication A en i), R est défini comme à la revendication 1 et

a) - soit X représente un atome de chlore et Y' représente un atome de soufre ou un radical de formule (II) dans laquelle $R_6$ et $R_7$ sont définis comme à la revendication 1 à l'exception pour $R_7$ de représenter un atome d'hydrogène,

b) - soit X représente un atome de brome et Y' représente un atome de soufre, et qui se présente sous la configuration E ou Z, puis éventuellement hydrolyse le produit obtenu lorsqu'on veut obtenir le produit selon la revendication 1 dans la formule duquel Y représente un radical de formule générale (II) dans laquelle $R_7$

représente un atome d'hydrogène, $R_4$ et $R_5$ représentent un atome d'hydrogène, et les autres symboles sont définis comme à la revendication 1, puis isole le produit obtenu et le transforme éventuellement en un sel thérapeutiquement acceptable.

9. Procédé de préparation d'un produit selon la revendication 1 dans la formule duquel Y représente un radical de formule générale (II) dans laquelle $R_7$ représente un radical alcoylaminocarbonyle et les autres symboles sont définis comme à la revendication 1, caractérisé en ce que l'on fait agir un isocyanate de formule générale:

$$O = C = N - R''_7 \qquad (XX)$$

dans laquelle $R''_7$ représente un radical alcoyle sur un produit selon la revendication 1 dans la formule duquel Y représente un radical de formule générale (II) dans laquelle $R_7$ représente un atome d'hydrogène et les autres symboles sont définis comme à la revendication 1, c'est-à-dire un produit de formule générale:

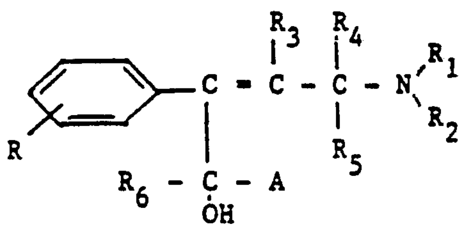

(XXVIII)

puis isole le produit et le transforme en un sel pharmaceutiquement acceptable.

10. Procédé de préparation d'un produit selon la revendication 1 dans la formule duquel Y représente un radical sulfinyle ou sulfonyle et les autres symboles sont définis comme à la revendication 1, caractérisé en ce que l'on oxyde un produit selon la revendication 1 dans la formule duquel Y représente un atome de soufre et les autres symboles sont définis comme à la revendication 1, c'est-à-dire un produit de formule générale:

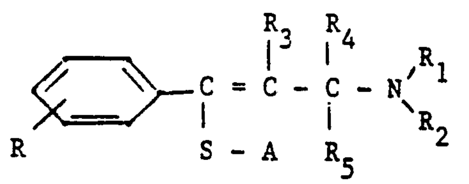

(XXXI)

puis isole le produit obtenu et le transforme éventuellement en un sel thérapeutiquement actif.

11. Composition pharmaceutique, caractérisée en ce qu'elle contient au moins un produit selon la revendication 1 en association avec un ou plusieurs diluants ou adjuvants compatibles et pharmaceutiquement acceptables.

**Revendications** pour l'état contractant: AT

1. Procédé de préparation d'un nouveau dérivé de la phényl-3 propène-2 amine de formule générale:

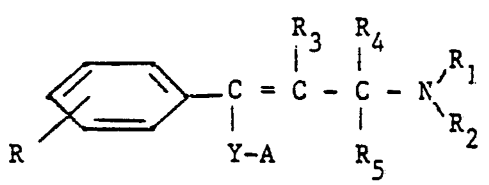

(I)

dans laquelle R représente un atome d'hydrogène ou d'halogène ou un radical alcoyle, alcoyloxy, alcoylthio, amino, alcoylamino, dialcoylamino ou trifluorométhyle, $R_3$ représente un atome d'hydrogène ou un radical alcoyle,

- soit $R_4$ et $R_5$, représentent un atome d'hydrogène et $R_1$ et $R_2$, identiques ou différents, représentent un atome d'hydrogène ou un radical alcoyle éventuellement substitué par un radical alcényle contenant 2 à 4 atomes de carbone, ou bien $R_1$ et $R_2$ forment ensemble avec l'atome d'azote auquel ils sont liés un radical hétérocyclique saturé contenant 4 à 7 chaînons et contenant éventuellement un autre hétéroatome tel que

l'oxygène, le soufre ou l'azote éventuellement substitué par un radical alcoyle,

-- soit $R_4$ représente un atome d'hydrogène, $R_1$ représente un atome d'hydrogène ou un radical alcoyle et $R_2$ et $R_5$ forment ensemble un radical alcoylène contenant 3 ou 4 atomes de carbone et i) ou bien A représente un radical alcoyle ou un radical phényle non substitué ou substitué par un ou deux substituants choisis parmi les atomes d'halogène et les radicaux alcoyle, alcoyloxy, alcoylthio, amito, alcoylamino, dialcoylamino, nitro ou trifluorométhyle ou bien représente un radical pyridyle, benzyle ou cycloalcoyle contenant 3 à 6 atomes de carbone, Y représente un atome de soufre, un radical sulfinyle ou sulfonyle ou encore un radical de formule générale:

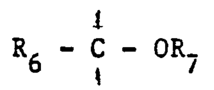

(II)

dans laquelle $R_6$ représente un atome d'hydrogène ou un radical alcoyle et $R_7$ représente un atome d'hydrogène ou un radical alcoylcarbonyle, alcoyloxycarbonyle, alcoylaminocarbonyle ou benzoyle eventuellement substitué par un ou deux substituants choisis parmi les atomes d'halogène et les radicaux alcoyle, alcoyloxy, alcoylthio, amino, alcoylamino, dialcoylamino, nitro ou trifluorométhyle, ii) ou bien Y et A forment ensemble un radical hydroxy-1 cycloalcoyle dont le cycle contient 5 ou 6 atomes de carbone, éventuellement accolé à un cycle benzénique,

ainsi que ses sels pharmaceutiquement acceptables, étant entendu que dans les définitions qui précèdent les radicaux alcoyle et portions alcoyle contiennent 1 à 4 atomes de carbone en chaîne droite ou ramifiée et que l'invention concerne tous les isomères géométriques et optiques possibles ainsi que leurs mélanges, caractérisé en ce que

I - pour la préparation d'un produit de formule générale (I) dans laquelle Y représente un radical de formule générale (II) dans laquelle $R_6$ est défini comme précédemment et $R_7$ représente un atome d'hydrogène et A, R, $R_1$ et $R_2$ sont définis comme précédemment à l'exception pour $R_1$ et/ou $R_2$ de représenter un atome d'hydrogène, et qui se présente sous la configuration Z, l'on fait agir un produit de formule générale:

$$A - CO - R_6 \quad (III)$$

dans laquelle A et $R_6$ sont définis comme précédemment sur un carbanion de formule générale:

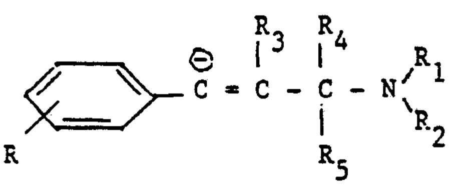

(IV)

dans laquelle les symboles sont définis comme précédemment à l'exception pour $R_1$ et/ou $R_2$ de représenter un atome d'hydrogène, puis isole le produit obtenu et le transforme éventuellement en un sel pharmaceutiquement acceptable, ou en ce que

II - pour la préparation d'un produit de formule générale (I) dans laquelle Y représente un radical de formule générale (II) dans laquelle $R_6$ et $R_7$ représentent un atome d'hydrogène, A est défini comme précédement en i), $R_1$ représente un atome d'hydrogène, $R_2$ représente un atome d'hydrogène ou un radical alcoyle éventuellement substitué par un radical alcényle contenant 2 à 4 atomes de carbone, R représente un atome d'hydrogène, $R_3$ est défini comme précédemment et $R_4$ et $R_5$ représentent un atome d'hydrogène, et qui se présente sous la configuration Z, l'on ouvre le cycle dihydrofuranne dans un produit de formule générale:

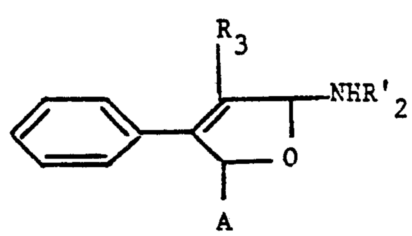

(XIV)

dans laquelle A est défini comme précédemment en i) et $R'_2$ représente un adical alcoyle éventuellement substitué par un radical alcényle contenant 2 à 4 atomes de carbone, et $R_3$ est défini comme précédemment, puis isole le produit obtenu et le transforme éventuellement en un sel pharmaceutiquement acceptable, ou en

ce que

III - pour la préparation d'un produit de formule générale (I) dans laquelle Y représente un atome de soufre, $R_4$ et $R_5$ représentent un atomme d'hydrogène, A est défini comme précédemment en i) et les autres symboles sont définis comme précédemment à l'exception pour $R_1$ et/ou $R_2$ de représenter un atome d'hydrogène, et qui se présente sous la configuration Z ou E, l'on fait agir un mercaptan de formule générale:

A - SH (XVII)

dans laquelle A est défini comme à la revencation 1 en i), sur un produit de formule générale:

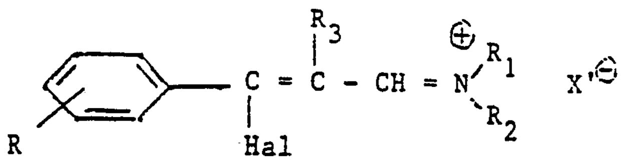
(XVIII)

dans laquelle R, $R_1$, $R_2$ et $R_3$ sont définis comme précédemment à l'exception pour $R_1$ et/ou $R_2$ de représenter un atome d'hydrogène, Hal représente un atome d'halogène et $X^{\ominus}$ représente un anion, puis l'on réduit l'intermédiaire obtenu de formule générale:

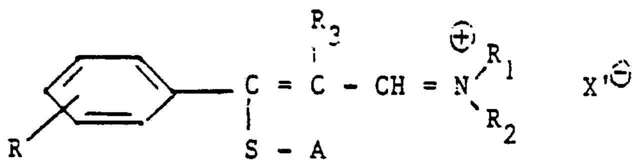
(XIX)

dans laquelle les symboles ont les définitions correspondantes, puis isole le produit obtenu et le transforme en un sel pharmaceutiquement acceptable, ou en ce que

IV - pour la préparation d'un produit de formule générale (I) dans laquelle Y représente un atome de soufre, $R_4$ et $R_5$ représentent un atome d'hydrogène, A est défini comme précédemment en i) et les autres symboles sont définis comme précédemment, et qui se présentent sous la configuration E ou Z, l'on fait agir l'ammoniac ou une amine de formule générale:

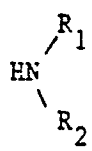
(XX)

dans laquelle $R_1$ et $R_2$ sont définis comme précédemment, sur un aldéhyde de formule générale:

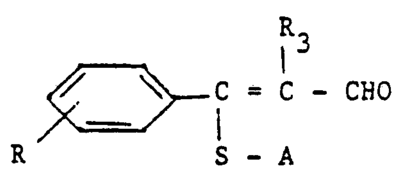
(XXI)

dans laquelle A est défini comme précédemment en i) et R et $R_3$ sont définis comme précédemment, et qui se présente sous la configuration E ou Z, puis isole le produit obtenu et le transforme éventuellement en un sel thérapeutiquement acceptable, ou en ce que

V - pour la préparation d'un produit de formule générale (I) dans laquelle Y représente un radical de formule générale (II) dans laquelle R représent un atome d'hydrogène ou bien est défini comme précédemment en ii) $R_3$ représente un atome d'hydrogène et les autre symboles sont définis comme précédemment, l'on fait agir un dérivé organomagnésien de formule générale:

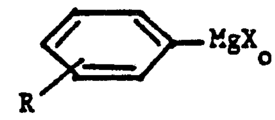 (XXIII)

dans laquelle R est défini comme précédemment et X représente un atome d'halogène, sur un produit de formule générale:

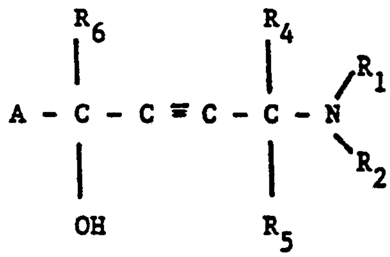 (XXIV)

dans laquelle les symboles sont définis comme précédemment, puis isole le produit obtenu et le transforme éventuellement en un sel pharmaceutiquement acceptable, ou en ce que

VI - pour la préparation d'un produit de formule générale (I) dans laquelle Y représente un radical de formule générale (II) dans laquelle $R_7$ représente un radical alcoylcarbonyle, alcoyloxycarbonyle ou benzoyle et les autres symboles sont définis comme précédemment, l'on fait agir un produit de formule générale:

$R'_7X_1$ (XXVII)

dans laquelle $R'_7$ représente un radical alcoylcarbonyle, alcoyloxycarbonyle ou benzoyle et $X_1$ représente un reste d'ester réactif, sur un produit de formule générale (I) dans laquelle Y représente un radical de formule générale (II) dans laquelle $R_7$ représente un atome d'hydrogène et les autres symboles sont définis comme précédemment, c'est-à-dire un produit de formule générale:

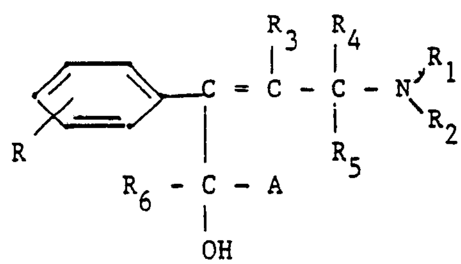 (XXVIII)

puis isole le produit obtenu et le transforme éventuellement en un sel pharmaceutiquement acceptable, ou en ce que

VII - pour la préparation d'un produit de formule générale (I) dans laquelle Y représente un atome de soufre ou un radical de formule générale (II) défini comme précédemment, $R_4$ et $R_5$ représentent un atome d'hydrogène et les autres symboles sont définis comme précédemment, et qui se présente sous la configuration E ou Z, l'on fait agir l'ammoniac, un précurseur d'amine ou une amine de formule générale:

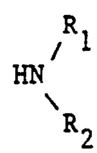 (XX)

sur un produit de formule générale:

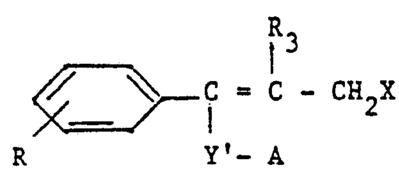 (XXIX)

dans laquelle A est défini comme précédemment en i), R est défini comme à la revendication 1 et

a) - soit X représente un atome de chlore et Y' représente un atome de soufre ou un radical de formule (II)

dans laquelle $R_6$ et $R_7$ sont définis comme précédemment à l'exception pour $R_7$ de représenter un atome d'hydrogène,

b) - soit X représente un atome de brome et Y' représente un atome de soufre, et qui se présente sous la configuration E ou Z, puis éventuellement hydrolyse le produit obtenu lorsqu'on veut obtenir le produit de formule générale (I) dans laquelle Y représente un radical de formule générale (II) dans laquelle $R_7$ représente un atome d'hydrogène et les autres symboles sont définis comme précédemment, puis isole le produit obtenu et le transforme éventuellement en un sel thérapeutiquement acceptable, ou en ce que

VIII - pour la préparation d'un produit de formule générale (I) dans laquelle Y représente un radical de formule générale (II) dans laquelle $R_7$ représente un radical alcoylaminocarbonyle et les autres symboles sont définis comme précédemment, l'on fait agir un isocyanate de formule générale:

$$O = C = N - R''_7 \qquad \text{(XXX)}$$

dans laquelle $R''_7$ représente un radical alcoyle sur un produit de formule générale (I) dans laquelle Y représente un radical de formule générale (II) dans laquelle $R_7$ représente un atome d'hydrogène et les autres symboles sont définis comme précédemment, c'est-à-dire un produit de formule générale:

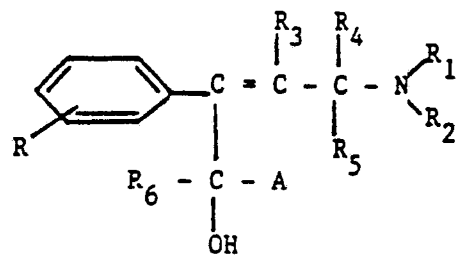

(XXVIII)

puis isole le produit et le transforme en un sel pharmaceutiquement acceptable, ou en ce que

IX - pour la préparation d'un produit de formule générale (I) dans laquelle Y représente un radical sulfinyle ou sulfonyle et les autres symboles sont définis comme précédemment, l'on oxyde un produit de formule générale (I) dans laquelle Y représente un atome de soufre et les autres symboles sont définis comme précédemment, c'est-à-dire un produit de formule générale:

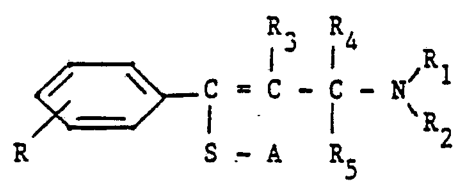

(XXXI)

puis isole le produit obtenu et le transforme éventuellement en un sel thérapeutiquement actif.

**Claims** for the Contracting States BE, CH, DE, FR, GB, IT, LI, LU, NL, SE

1. New 3-phenyl-2-propeneamine derivative, characterized in that it corresponds to the general formula:

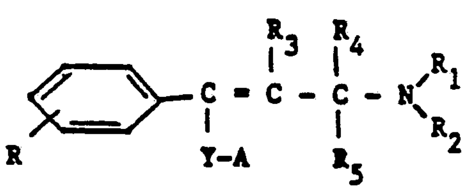

(I)

in which R denotes a hydrogen or halogen atom or an alkyl, alkyloxy, alkylthio, amino, alkylamino, dialkylamino or trifluoromethyl radical, $R_3$ denotes a hydrogen atom or an alkyl radical,

- either $R_4$ and $R_5$ denote a hydrogen atom and $R_1$ and $R_2$, which are identical or different, denote a hydrogen atom or an alkyl radical optionally substituted by an alkenyl radical containing 2 to 4 carbon atoms, or alternatively $R_1$ and $R_2$ form together with the nitrogen atom to which they are linked a saturated heterocyclic radical containing 4 to 7 ring members and optionally containing another hetero atom such as oxygen, sulphur or nitrogen, optionally substituted by an alkyl radical,

- or $R_4$ denotes a hydrogen atom, $R_1$ denotes a hydrogen atom or an alkyl radical and $R_2$ and $R_5$ together form an alkylene radical containing 3 or 4 carbon atoms, and i) either A denotes an alkyl radical or a phenyl radical which is unsubstituted or substituted by one or two substituents chosen from halogen atoms and alkyl, alkyloxy, alkylthio, amino, alkylamino, dialkylamino, nitro or trifluoromethyl radicals or denotes a pyridyl, benzyl or cycloalkyl radical containing 3 to 6 carbon atoms, Y denotes a sulphur atom, a sulphinyl or sulphonyl radical or a radical of general formula:

$$R_6 - \overset{|}{\underset{|}{C}} - OR_7 \qquad (II)$$

in which $R_6$ denotes a hydrogen atom or an alkyl radical and $R_7$ denotes a hydrogen atom or an alkylcarbonyl, alkyloxycarbonyl, alkylaminocarbonyl or benzoyl radical optionally substituted by one or two substituents chosen from halogen atoms and alkyl, alkyloxy, alkylthio, amino, alkylamino, dialkylamino, nitro or trifluoromethyl radicals, ii) or Y and A together form a 1-hydroxycycloalkyl radical the ring of which contains 5 or 6 carbon atoms, optionally joined to a benzene ring,

and its pharmaceutically acceptable salts, it being understood that in the preceding definitions the alkyl radicals and alkyl parts contain 1 to 4 carbon atoms as a straight-or branched chain and that the invention relates to all the possible geometric and optical isomers as well as their mixtures.

2. Process for the preparation of a product according to Claim 1 in the formula of which Y denotes a radical of general formula (II) in which $R_6$ is defined as in Claim 1 and $R_7$ denotes a hydrogen atom and A, R, $R_1$ and $R_2$ are defined as in Claim 1, except for $R_1$ and/or $R_2$ denoting a hydrogen atom, and which is in the Z configuration, characterized in that a product of general formula:

$$A - CO - R_6 \qquad (III)$$

in which A and $R_6$ are defined as in Claim 1, is reacted with a carbanion of general formula:

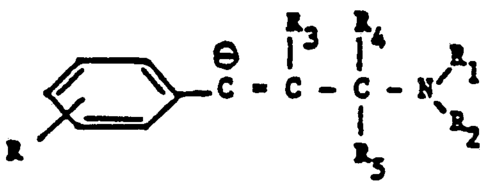

(IV)

in which the symbols are defined as in Claim 1, except for $R_1$ and/or $R_2$ denoting a hydrogen atom, and the product obtained is then isolated and optionally converted into a pharmaceutically acceptable salt.

3. Process for the preparation of a product according to Claim 1, in the formula of which Y denotes a radical of general formula (II) in which $R_6$ and $R_7$ denote a hydrogen atom, A is defined as in Claim 1 under i), $R_1$ denotes a hydrogen atom, $R_2$ denotes a hydrogen atom or an alkyl radical optionally substituted by an alkenyl radical containing 2 to 4 carbon atoms, R denotes a hydrogen atom, $R_3$ is defined as in Claim 1 and $R_4$ and $R_5$ denote a hydrogen atom, and which is in the Z configuration, characterized in that the dihydrofuran ring in a product of general formula:

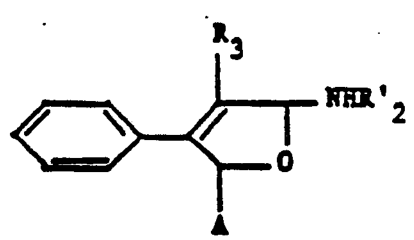

(XIV)

in which A is defined as in Claim 1 under i) and $R'_2$ denotes an alkyl radical optionally substituted by an alkenyl radical containing 2 to 4 carbon atoms and $R_3$ is defined as above, is opened, and then the product obtained is isolated and optionally converted into.a pharmaceutically acceptable salt.

4. Process for the preparation of a product according to Claim 1, in the formula of which Y denotes a sulphur atom, $R_4$ and $R_5$ denote a hydrogen atom, A is defined as in Claim 1 under i) and the remaining symbols are defined as in Claim 1, except for $R_1$ and/or $R_2$ denoting a hydrogen atom, and which is in the Z or E configuration, characterized in that a mercaptan of general formula:

$$A - SH \qquad (XVII)$$

in which A is defined as in Claim 1 under i) is reacted with a product of general formula:

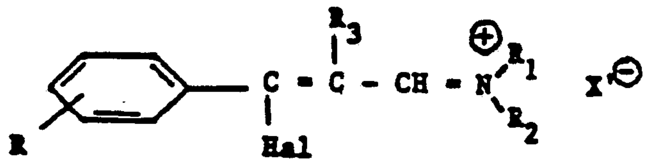

(XVIII)

in which R, $R_1$, $R_2$ and $R_3$ are defined as in Claim 1, except for $R_1$ and/or $R_2$ denoting a hydrogen atom, Hal denotes a halogen atom and $X'^{\ominus}$ denotes an anion, and then the intermediate obtained of general formula:

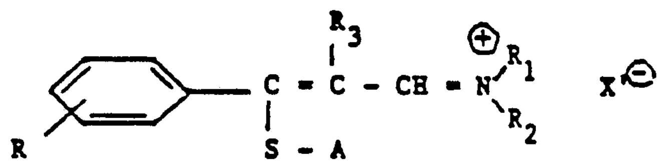

(XIX)

in which the symbols have the corresponding definitions is reduced and then the product obtained is isolated and converted into a pharmaceutically acceptable salt.

5. Process for the preparation of a product according to Claim 1, in the formula of which Y denotes a sulphur atom, $R_4$ and $R_5$ denote a hydrogen atom, A is defined as in Claim 1 under i) and the remaining symbols are defined as in Claim 1, and which is in the E or Z configuration, characterized in that ammonia or an amine of general formula:

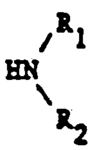

(XX)

in which $R_1$ and $R_2$ are defined as in Claim 1, is reacted with an aldehyde of general formula:

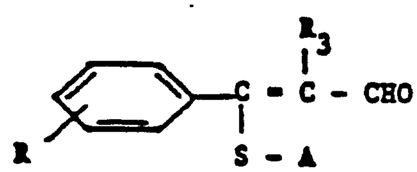

(XXI)

in which A is defined as in Claim 1 under i) and R and $R_3$ are defined as in Claim 1, and which is in the E or Z configuration, and then the product obtained is isolated and optionally converted into a therapeutically acceptable salt.

6. Process for the preparation of a product according to Claim 1, in the formula of which Y denotes a radical of general formula (II) in which $R_7$ denotes a hydrogen atom or Y is defined as in Claim 1 under ii), $R_3$ denotes a hydrogen atom, and the remaining symbols are defined as in Claim 1, characterized in that an organomagnesium derivative of general formula:

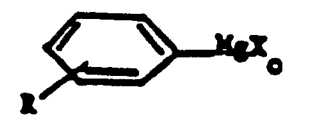

(XXIII)

in which R is defined as in Claim 1 and $X_o$ denotes a halogen atom, is reacted with a product of general formula:

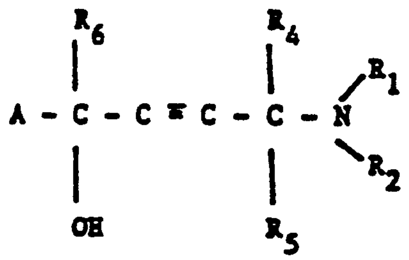

(XXIV)

in which the symbols are defined as in Claim 1, and then the product obtained is isolated and optionally converted into a pharmaceutically acceptable salt.

7. Process for the preparation of a product according to Claim 1, in the formula of which Y denotes a radical of general formula (II) in which $R_7$ denotes an alkylcarbonyl, alkyloxycarbonyl or benzoyl radical and the remaining symbols are defined as in Claim 1, characterized in that a product of general formula:

$R'_7X_1$ (XXVII)

in which $R'_7$ denotes an alkylcarbonyl, alkyloxycarbonyl or benzoyl radical and $X_1$ denotes a reactive ester residue, is reacted with a product according to Claim 1 in the formula of which Y denotes a radical of general formula (II) in which $R_7$ denotes a hydrogen atom and the remaining symbols are defined as in Claim 1, that is to say a product of general formula:

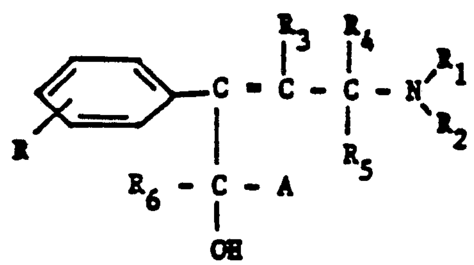

(XXVIII)

and then the product obtained is isolated and optionally converted into a pharmaceutically acceptable salt.

8. Process for the preparation of a product according to Claim 1, in the formula of which Y denotes a sulphur atom or a radical of general formula (II) defined as in Claim 1, $R_4$ and $R_5$ denote a hydrogen atom and the remaining symbols are defined as in Claim 1, and which is in the E or Z configuration, characterized in that ammonia, an amine-precursor or an amine of general formula:

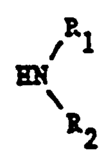

(XX)

is reacted with a product of general formula:

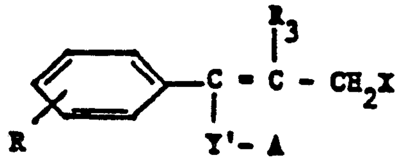

(XXIX)

in which A is defined as in Claim 1 under i), R is defined as in Claim 1 and

a) - either X denotes a chlorine atom and Y' denotes a sulphur atom or a radical of formula (II) in which $R_6$ and $R_7$ are defined as in Claim 1 except for $R_7$ denoting a hydrogen atom,

b) - or X denotes a bromine atom and Y' denotes a sulphur atom and which is in the E or Z configuration, and then the product obtained is optionally hydrolysed when the intention is to obtain the product according to Claim 1 in the formula of which Y denotes a radical of general formula (II) in which $R_7$ denotes a hydrogen atom, $R_4$ and $R_5$ denote a hydrogen atom and the remaining symbols are defined as in Claim 1, and then the product obtained is isolated and optionally converted into a therapeutically acceptable salt.

9. Process for the preparation of a product according to Claim 1, in the formula of which Y denotes a radical of general formula (II) in which $R_7$ denotes an alkylaminocarbonyl radical and the remaining symbols are defined as in Claim 1, characterized in that an isocyanate of general formula:

$O = C = N - R''_7$ (XXX)

in which $R''_7$ denotes an alkyl radical is reacted with a product according to Claim 1, in the formula of which Y denotes a radical of general formula (II) in which $R_7$ denotes a hydrogen atom and the remaining symbols are defined as in Claim 1, that is to say a product of general formula:

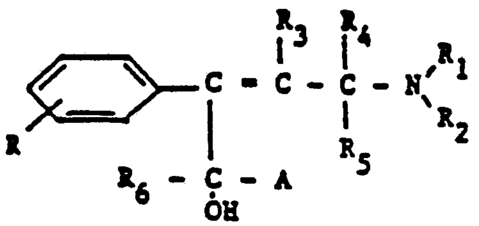

(XXVIII)

and then the product is isolated and is converted into a pharmaceutically acceptable salt.

10. Process for the preparation of a product according to Claim 1, in the formula of which Y denotes a sulphinyl or sulphonyl radical and the remaining symbols are defined as in Claim 1, characterized in that a product according to Claim 1 in the formula of which Y denotes a sulphur atom and the remaining symbols are defined as in Claim 1, that is to say a product of general formula:

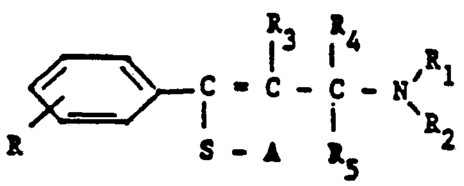

(XXXI)

is oxidized and then the product obtained is isolated and optionally converted into a therapeutically active salt.

11. Pharmaceutical composition, characterized in that it contains at least one product according to Claim 1 in combination with one or more compatible and pharmaceutically acceptable diluents or adjuvants.

**Claim** for the Contracting State: AT

Process for the preparation of a new 3-phenyl-2-propeneamine derivative, of general formula:

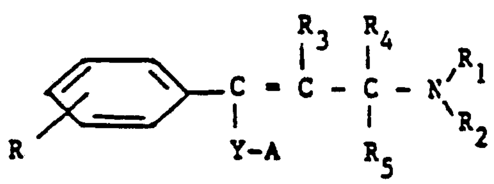

(I)

in which R denotes a hydrogen or halogen atom or an alkyl, alkyloxy, alkylthio, amino, alkylamino, dialkylamino or trifluoromethyl radical, $R_3$ denotes a hydrogen atom or an alkyl radical,

- either $R_4$ and $R_5$ denote a hydrogen atom and $R_1$ and $R_2$, which are identical or different, denote a hydrogen atom or an alkyl radical optionally substituted by an alkenyl radical containing 2 to 4 carbon atoms, or alternatively $R_1$ and $R_2$ form together with the nitrogen atom to which they are linked a saturated heterocyclic radical containing 4 to 7 ring members and optionally containing another hetero atom such as oxygen, sulphur or nitrogen, optionally substituted by an alkyl radical,

- or $R_4$ denotes a hydrogen atom, $R_1$ denotes a hydrogen atom or an alkyl radical and $R_2$ and $R_5$ together

form an alkylene radical containing 3 or 4 carbon atoms, and i) either A denotes an alkyl radical or a phenyl radical which is unsubstituted or substituted by one or two substituents chosen from halogen atoms and alkyl, alkyloxy, alkylthio, amino, alkylamino, dialkylamino, nitro or trifluoromethyl radicals or denotes a pyridyl, benzyl or cycloalkyl radical containing 3 to 6 carbon atoms, Y denotes a sulphur atom, a sulphinyl or sulphonyl radical or a radical of general formula:

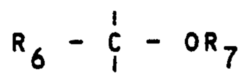 (II)

in which $R_6$ denotes a hydrogen atom or an alkyl radical and $R_7$ denotes a hydrogen atom or an alkylcarbonyl, alkyloxycarbonyl, alkylaminocarbonyl or benzoyl radical optionally substituted by one or two substituents chosen from halogen atoms and alkyl, alkyloxy, alkylthio, amino, alkylamino, dialkylamino, nitro or trifluoromethyl radicals, ii) or Y and A together form a 1-hydroxycycloalkyl radical the ring of which contains 5 or 6 carbon atoms, optionally joined to a benzene ring,

and its pharmaceutically acceptable salts, it being understood that in the preceding definitions the alkyl radicals and alkyl parts contain 1 to 4 carbon atoms as a straight or branched chain and that the invention relates to all the possible geometric and optical isomers as well as their mixtures, characterized in that

I - for the preparation of a product of general formula (I) in which Y denotes a radical of general formula (II) in which $R_6$ is defined as above and $R_7$ denotes a hydrogen atom and A, R, $R_1$ and $R_2$ are defined as above, except for $R_1$ and/ or $R_2$ denoting a hydrogen atom, and which is in the Z configuration, a product of general formula:

$A - CO - R_6$ (III)

in which A and $R_6$ are defined as above, is reacted with a carbanion of general formula:

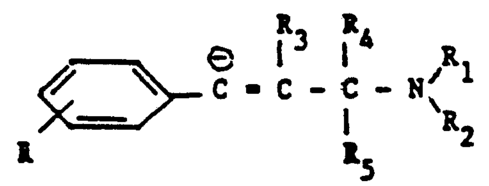 (IV)

in which the symbole are defined as above, except for $R_1$ and/or $R_2$ denoting a hydrogen atom, and the product obtained is then isolated and optionally converted into a pharmaceutically acceptable salt, or in that

II - for the preparation of a product of general formula (I) in which Y denotes a radical of general formula (II) in which $R_6$ and $R_7$ denote a hydrogen atom, A is defined as above under i), $R_1$ denotes a hydrogen atom, $R_2$ denotes a hydrogen atom or an alkyl radical optionally substituted by an alkenyl radical containing 2 to 4 carbon atoms, R denotes a hydrogen atom, $R_3$ is defined as above and $R_4$ and $R_5$ denote a hydrogen atom, and which is in the Z configuration, the dihydrofuran ring in a product of general formula:

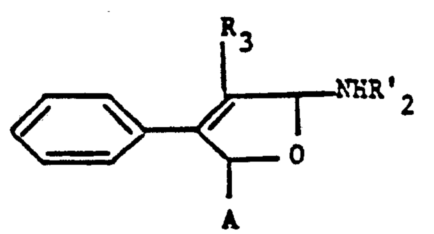 (XIV)

in which A is defined as above under i) and $R'_2$ denotes an alkyl radical optionally substituted by an alkenyl radical containing 2 to 4 carbon atoms and $R_3$ is defined as above, is opened and then the product obtained is isolated and optionally converted into a pharmaceutically acceptable salt, or in that

III - for the preparation of a product of general formula (I) in which Y denotes a sulphur atom, $R_4$ and $R_5$ denote a hydrogen atom, A is defined as above under i) and the remaining symbols are defined as above, except for $R_1$ and/or $R_2$ denoting a hydrogen atom, and which is in the Z or E configuration, a mercaptan of general formula:

$A - SH$ (XVII)

in which A is defined as in Claim 1 under i) is reacted with a product of general formula:

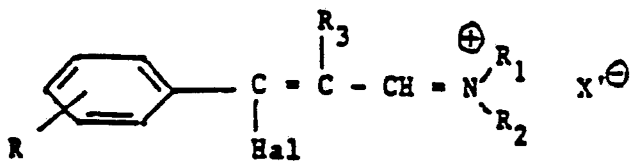

(XVIII)

in which R, $R_1$, $R_2$ and $R_3$ are defined as above, except for $R_1$ and/or $R_2$ denoting a hydrogen atom, Hal denotes a halogen atom and $X'^{\ominus}$ denotes an anion, and then the intermediate obtained of general formula:

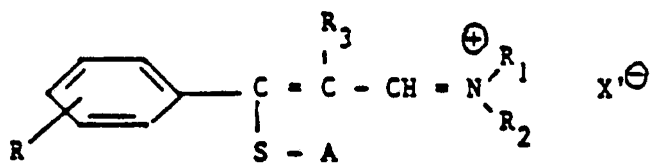

(XIX)

in which the symbols have the corresponding definition, is reduced and then the product obtained is isolated and converted into a pharmaceutically acceptable salt, or in that IV - for the preparation of a product of general formula (I) in which Y denotes a sulphur atom, $R_4$ and $R_5$ denote a hydrogen atom, A is defined as above under i) and the remaining symbols are defined as above, and which is in the E or Z configuration, ammonia or an amine of general formula:

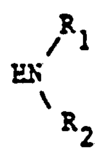

(XX)

in which $R_1$ and $R_2$ are defined as above, is reacted with an aldehyde of general formula:

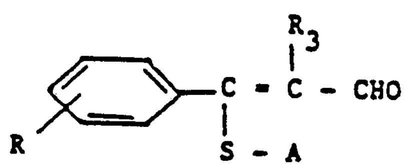

(XXI)

in which A is defined as above under i) and R and $R_3$ are defined as above, and which is in the E or Z configuration, and then the product obtained is isolated and optionally converted into a therapeutically acceptable salt, or in that

V - for the preparation of a product of general formula (I) in which Y denotes a radical of general formula (II) in which $R_7$ denotes a hydrogen atom or is defined as above under ii), $R_3$ denotes a hydrogen atom, and the remaining symbols are defined as above, an organomagnesium derivative of general formula:

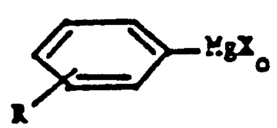

(XXIII)

in which R is defined as above and $X_o$ denotes a halogen atom, is reacted with a product of general formula:

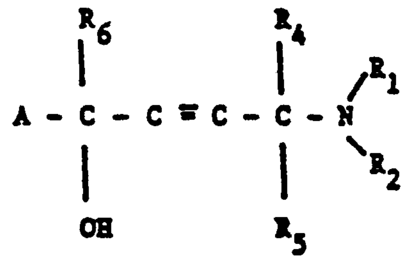 (XXIV)

in which the symbols are defined as above, and then the product obtained is isolated and optionally converted into a pharmaceutically acceptable salt, or in that

VI - for the preparation of a product of general formula (I), in which Y denotes a radical of general formula (II) in which $R_7$ denotes an alkylcarbonyl, alkyloxycarbonyl or benzoyl radical and the remaining symbols are defined as above, a product of general formula:

$R'_7X_1$ (XXVII)

in which $R'_7$ denotes an alkylcarbonyl, alkyloxycarbonyl or benzoyl radical and $X_1$ denotes a reactive ester residue, is reacted with a product of general formula (I) in which Y denotes a radical of general formula (II) in which $R_7$ denotes a hydrogen atom and the remaining symbols are defined as above, that is to say a product of general formula:

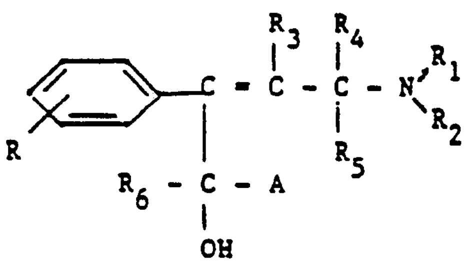 (XXVIII)

and then the product obtained is isolated and optionally converted into a pharmaceutically acceptable salt, or in that

VII - for the preparation of a product of general formula (I), in which Y denotes a sulphur atom or a radical of general formula (II) defined as above, $R_4$ and $R_5$ denote a hydrogen atom and the remaining symbols are defined as above, and which is in the E or Z configuration, ammonia, an amine-precursor or an amine of general formula:

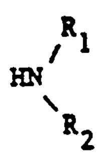 (XX)

is reacted with a product of general formula:

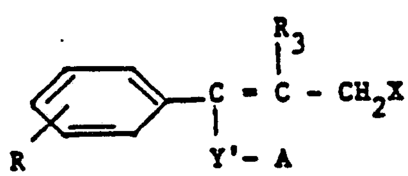 (XXIX)

in which A is defined as above under i), R is defined as in Claim 1 and

a) - either X denotes a chlorine atom and Y' denotes a sulphur atom or a radical of formula (II) in which $R_6$ and $R_7$ are defined as above except for $R_7$ denoting a hydrogen atom,

b) - or X denotes a bromine atom and Y' denotes a sulphur atom and which is in the E or Z configuration, and then the product obtained is optionally hydrolysed when the intention is to obtain the product of general formula (I) in which Y denotes a radical of general formula (II) in which $R_7$ denotes a hydrogen atom, and the

remaining symbols are defined as above, and then the product obtained is isolated and optionally converted into a therapeutically acceptable salt, or in that

VIII - for the preparation of a product of general formula (I), in which Y denotes a radical of general formula (II) in which $R_7$ denotes an alkylaminocarbonyl radical and the remaining symbols are defined as above, an isocyanate of general formula:

$$O = C = N - R''_7 \qquad (XXX)$$

in which $R''_7$ denotes an alkyl radical is reacted with a product of general formula (I), in which Y denotes a radical of general formula (II) in which $R_7$ denotes a hydrogen atom and the remaining symbols are defined as above, that is to say a product of general formula:

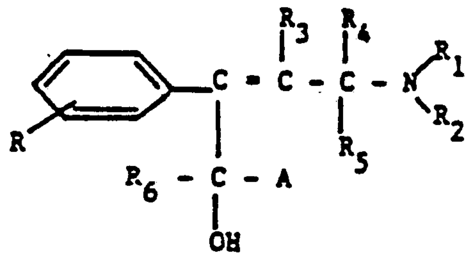

(XXVIII)

and then the product is isolated and is converted into a pharmaceutically acceptable salt, or in that

IX - for the preparation of a product of general formula (I), in which Y denotes a sulphinyl or sulphonyl radical and the remaining symbols are defined as above, a product of general formula (I) in which Y denotes a sulphur atom and the remaining symbols are defined as above, that is to say a product of general formula:

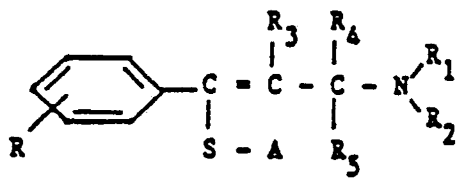

(XXXI)

is oxidized and then the product obtained is isolated and optionally converted into a therapeutically active salt.

**Patentansprüche** für die Vertragsstaaten: BE, CH, DE, FR, GB, IT, LI, LU, NL, SE

1. Neues Derivat des 3-Phenyl-2-propenamins, dadurch gekennzeichnet, daß es der allgemeinen Formel:

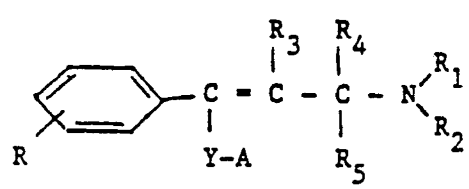

(I)

entspricht, worin R ein Wasserstoff- oder Halogenatom oder einen Alkyl-, Alkyloxy-, Alkylthio-, Amino-, Alkylamino-, Dialkylamino- oder Trifluormethylrest bedeutet, $R_3$ ein Wasserstoffatom oder einen Alkylrest bedeutet,

- entweder $R_4$ und $R_5$ ein Wasserstoffatom bedeuten und $R_1$ und $R_2$, die identisch oder verschieden sind, ein Wasserstoffatom oder einen Alkylrest bedeuten, der gegebenenfalls durch einen Alkenylrest mit 2 bis 4 Kohlenstoffatomen substituiert ist, oder aber $R_1$ und $R_2$ bilden zusammen mit dem Stickstoffatom, an das sie gebunden sind, einen gesättigten heterocyclischen Rest mit 4 bis 7 Kettengliedern und gegebenenfalls ein weiteres Heteroatom wie Sauerstoff, Schwefel oder Stickstoff enthaltend, gegebenenfalls substituiert durch einen Alkylrest, - oder $R_4$ bedeutet ein Wasserstoffatom, $R_1$ bedeutet ein Wasserstoffatom oder einen Alkylrest und $R_2$ und $R_3$ bilden zusammen einen Alkylenrest mit 3 oder 4 Kohlenstoffatomen und i) entweder A bedeutet einen Alkylrest oder einen Phenylrest, nicht-substituiert oder substituiert durch einen oder zwei Substituenten, ausgewählt unter den Halogenatomen, den Alkyl-, Alkyloxy-, Alkylthio-, Amino-, Alkylamino-, Dialkylamino-, Nitro- oder Trifluormethylresten oder aber bedeutet einen Pyridyl-, Benzyl- oder Cycloalkylrest mit 3 bis 6

Kohlenstoffatomen, Y bedeutet ein Schwefelatom, einen Sulfinylrest oder Sulfonylrest oder überdies noch einen Rest der allgemeinen Formel:

$$R_6 - \overset{|}{\underset{|}{C}} - OR_7 \qquad (II)$$

worin $R_6$ ein Wasserstoffatom oder einen Alkylrest und $R_7$ ein Wasserstoffatom oder einen Alkylcarbonyl-, Alkoxycarbonyl-, Alkylaminocarbonyl- oder Benzoylrest bedeutet, gegebenenfalls substituiert durch einen oder zwei Substituenten, ausgewählt unter den Halogenatomen und den Alkyl-, Alkyloxy-, Alkylthio-, Amino-, Alkylamino-, Dialkylamino-, Nitro- oder Trifluormethylresten,

ii) oder aber Y und A bilden zusammen einen 1-Hydroxycycloalkylrest, dessen Ring 5 oder 6 Kohlenstoffatome enthält, gegebenenfalls verbunden mit einem Benzolring, sowie seine pharmazeutisch annehmbaren Salze, wobei in den vorstehenden Definitionen die Alkylreste und Alkylteile 1 bis 4 Kohlenstoffatome in gerader oder verzweigter Kette enthalten und die Erfindung alle möglichen optischen und geometrischen Isomeren sowie ihre Gemische betrifft.

2. Verfahren zur Herstellung eines Produkts gemäß Anspruch 1, in dessen Formel Y einen Rest der allgemeinen Formel (II) bedeutet, worin $R_6$ wie in Anspruch 1 definiert ist und $R_7$ ein Wasserstoffatom bedeutet und A, R, $R_1$ und $R_2$ wie in Anspruch 1 definiert sind, mit Ausnahme der Bedeutung eines Wasserstoffatoms für $R_1$ und/oder $R_2$, und das sich in Z-Konfiguration befindet, dadurch gekennzeichnet, daß man ein Produkt der allgemeinen Formel

$$A - CO - R_6 \qquad (III)$$

worin A und $R_6$ wie in Anspruch 1 definiert sind, auf ein Carbanion der allgemeinen Formel:

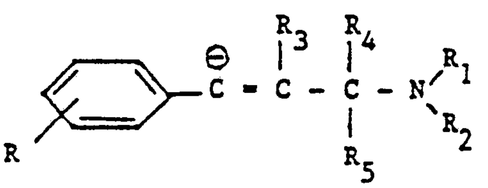

(IV)

einwirken läßt, worin die Symbole wie in Anspruch 1 definiert sind, mit Ausnahme der Bedeutung eines Wasserstoffatoms für $R_1$ und/oder $R_2$, daß man dann das erhaltene Produkt isoliert und es gegebenenfalls in ein pharmazeutisch annehmbares Salz umwandelt.

3. Verfahren zur Herstellung eines Produkts gemäß Anspruch 1, in dessen Formel Y einen Rest der allgemeinen Formel (II) bedeutet, worin $R_6$ und $R_7$ ein Wasserstoffatom darstellen, A wie in Anspruch 1 in i) definiert ist, $R_1$ ein Wasserstoffatom bedeutet, $R_2$ ein Wasserstoffatom oder einen Alkylrest bedeutet, der gegebenenfalls durch einen Alkenylrest mit 2 bis 4 Kohlenstoffatomen substituiert ist, R ein Wasserstoffatom bedeutet, $R_3$ wie Anspruch 1 definiert ist und $R_4$ und $R_5$ ein Wasserstoffatom bedeuten, und das sich in der Z-Konfiguration befindet, dadurch gekennzeichnet, daß man den Dihydrofuranring in einem Produkt der allgemeinen Formel:

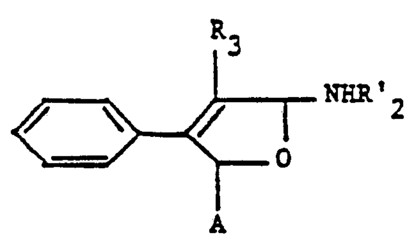

(XIV)

öffnet, worin A wie in Anspruch 1 in i) definiert ist und $R'_2$ einen Alkylrest bedeutet, der gegebenenfalls durch einen Alkenylrest mit 2 bis 4 Kohlenstoffatomen substituiert ist, und $R_3$ wie vorstehend definiert ist, daß man das erhaltene Produkt dann isoliert und es gegebenenfalls in ein pharmazeutisch annehmbares Salz überführt.

4. Verfahren zur Herstellung eines Produkts gemäß Anspruch 1, in dessen Formel Y ein Schwefelatom bedeutet, $R_4$ und R ein Wasserstoffatom bedeuten, A wie in Anspruch 1 in i) definiert ist und die übrigen Symbole wie in Anspruch 1 definiert sind, mit Ausnahme der Bedeutung eines Wasserstoffatoms für $R_1$ und/oder $R_2$, und das sich in der Z- oder E-Konfiguration befindet, dadurch gekennzeichnet, daß man ein Mercaptan der allgemeinen Formel:

A - SH (XVII)

worin A wie in Anspruch 1 in i) definiert ist, auf ein Produkt der allgemeinen Formel:

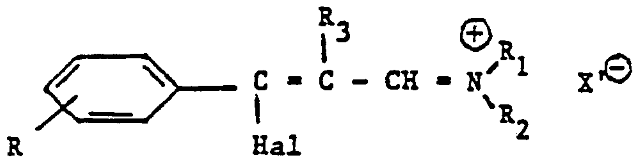 (XVIII)

einwirken läßt, worin R, $R_1$, $R_2$ und $R_3$ wie in Anspruch 1 definiert sind, ausgenommen die Bedeutung eines Wasserstoffatoms für $R_1$ und/oder $R_2$, Hal ein Halogenatom bedeutet und $X'^{\ominus}$ ein Anion bedeutet, daß man dann das erhaltene Zwischenprodukt der allgemeinen Formel:

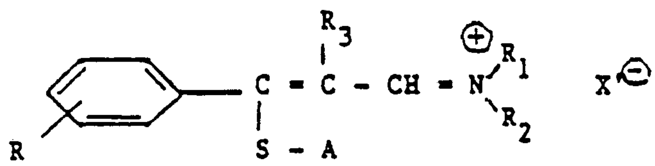 (XIX)

worin die Symbole die entsprechenden Bedeutungen haben, reduziert, das erhaltene Produkt dann isoliert und es in ein pharmazeutisch annehmbares Salz überführt.

5. Verfahren zur Herstellung eines Produkts gemäß Anspruch 1, in dessen Formel Y ein Schwefelatom bedeutet, $R_4$ und $R_5$ ein Wasserstoffatom bedeuten, A wie in Anspruch 1 in i) definiert ist und die anderen Symbole wie in Anspruch 1 definiert sind, und das sich in E- oder Z-Konfiguration befindet, dadurch gekennzeichnet, daß man Ammoniak oder Amin der allgemeinen Formel:

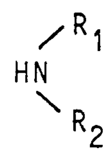 (XX)

worin $R_1$ und $R_2$ wie in Anspruch 1 definiert sind, auf einen Aldehyd der allgemeinen Formel:

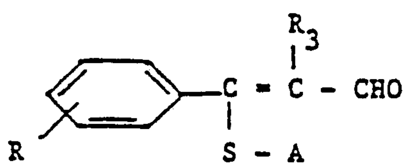 (XXI)

einwirken läßt, worin A wie in Anspruch 1 in i) definiert ist und R und $R_3$ wie in Anspruch 1 definiert sind, und das sich in der E- oder Z-Konfiguration befindet, das erhaltene Produkt dann isoliert und es gegebenenfalls in ein therapeutisch annehmbares Salz überführt.

6. Verfahren zur Herstellung eines Produkts gemäß Anspruch 1, in dessen Formel Y einen Rest der allgemeinen Formel (II) bedeutet, worin $R_7$ ein Wasserstoffatom bedeutet oder Y wie in Anspruch 1 in ii) definiert ist, $R_3$ ein Wasserstoffatom bedeutet und die übrigen Symbole wie in Anspruch 1 definiert sind, dadurch gekennzeichnet, daß man ein Organomagnesiumderivat der allgemeinen Formel:

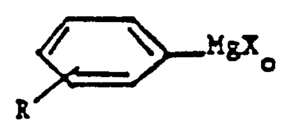 (XXIII)

worin R wie in Anspruch 1 definiert ist und $X_o$ ein Halogenatom bedeutet, auf ein Produkt der allgemeinen Formel:

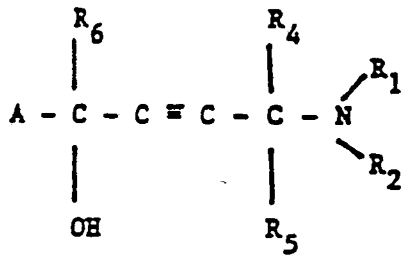

(XXIV)

worin die Symbole wie in Anspruch 1 definiert sind, einwirken läßt, das erhaltene Produkt dann isoliert und es gegebenenfalls in ein pharmazeutisch annehmbares Salz überführt.

7. Verfahren zur Herstellung eines Produkts gemäß Anspruch 1, in dessen Formel Y einen Rest der allgemeinen Formel (II) bedeutet, worin $R_7$ einen Alkylcarbonyl-, Alkyloxycarbonyl- oder Benzoylrest darstellt und die übrigen Symbole wie in Anspruch 1 definiert sind, dadurch gekennzeichnet, daß man ein Produkt der allgemeinen Formel:

$R'_7X_1$ (XXVII)

worin $R'_7$ einen Alkylcarbonyl-, Alkyloxycarbonyl- oder Benzoylrest bedeutet und $X_1$ einen Rest eines reaktionsfähigen Esters darstellt, auf ein Produkt gemäß Anspruch 1 einwirken läßt, in dessen Formel Y einen Rest der allgemeinen Formel (II) bedeutet, worin $R_7$ ein Wasserstoffatom darstellt und die übrigen Symbole wie in Anspruch 1 definiert sind, d. h. auf ein Produkt der allgemeinen Formel:

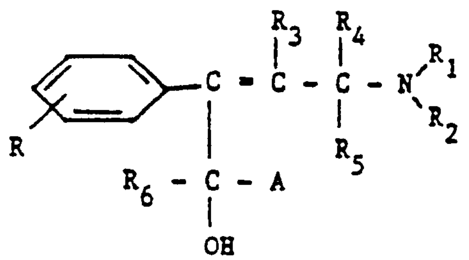

(XXVIII)

dann das erhaltene Produkt isoliert und es gegebenenfalls in ein pharmazeutisch annehmbares Salz überführt.

8. Verfahren zur Herstellung eines Produkts gemäß Anspruch 1, in dessen Formel Y ein Schwefelatom oder einen Rest der allgemeinen Formel (II), wie in Anspruch 1 definiert, bedeutet, $R_4$ und $R_5$ ein Wasserstoffatom bedeuten und die übrigen Symbole wie in Anspruch 1 definiert sind, und das in der E- oder Z-Konfiguration vorliegt, dadurch gekennzeichnet, daß man Ammoniak, einen Vorläufer eines Amins oder ein Amin der allgemeinen Formel:

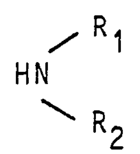

(XX)

auf ein Produkt der allgemeinen Formel:

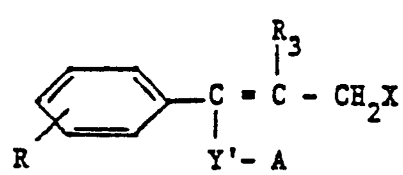

(XXIX)

einwirken läßt, worin A wie in Anspruch 1 in i) definiert ist,

R wie in Anspruch 1 definiert ist und

a) entweder X ein Chloratom bedeutet und Y' ein Schwefelatom oder einen Rest der Formel (II) darstellt, worin $R_6$ und $R_7$ wie in Anspruch 1 definiert sind, mit Ausnahme der Bedeutung eines Wasserstoffatoms für $R_7$,

b) oder X ein Bromatom bedeutet und Y' ein Schwefelatom darstellt, und däs sich in E- oder Z-Konfiguration

befindet, dann das erhaltene Produkt gegebenenfalls hydrolysiert, wenn man das Produkt gemäß Anspruch 1 erhalten will, in dessen Formel Y einen Rest der allgemeinen Formel (II) bedeutet, worin $R_7$ ein Wasserstoffatom darstellt, $R_4$ und $R_5$ ein Wasserstoffatom bedeuten und die übrigen Symbole wie in Anspruch 1 definiert sind, das erhaltene Produkt dann isoliert und es gegebenenfalls in ein therapeutisch annehmbares Salz überführt.

9. Verfahren zur Herstellung eines Produkts gemäß Anspruch 1, in dessen Formel Y einen Rest der allgemeinen Formel (II) bedeutet, worin $R_7$ einen Alkylaminocarbonylrest darstellt und die übrigen Symbole wie in Anspruch 1 definiert sind, dadurch gekennzeichnet, daß man ein Isocyanat der allgemeinen Formel:

$$O = C = N - R''_7 \qquad (XXX-)$$

worin $R''_7$ einen Alkylrest bedeutet, auf ein Produkt gemäß Anspruch 1 einwirken läßt, in dessen Formel Y einen Rest der allgemeinen Formel (II) bedeutet, worin $R_7$ ein Wasserstoffatom bedeutet und die übrigen Symbole wie in Anspruch 1 definiert sind, d. h. auf ein Produkt der allgemeinen Formel:

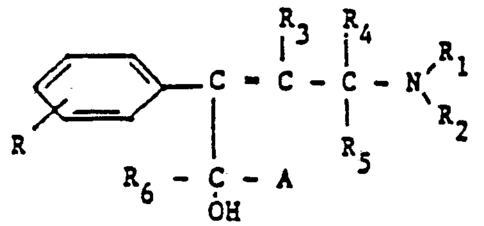

(XXVIII)

das Produkt dann isoliert und es in ein pharmazeutisch annehmbares Salz überführt.

10. Verfahren zur Herstellung eines Produkts gemäß Anspruch 1, in dessen Formel Y einen Sulfinyl- oder Sulfonylrest bedeutet und die übrigen Symbole wie in Anspruch 1 definiert sind, dadurch gekennzeichnet, daß man ein Produkt gemäß Anspruch 1, in dessen Formel Y ein Schwefelatom bedeutet und die übrigen Symbole wie in Anspruch 1 definiert sind, d. h. ein Produkt der allgemeinen Formel:

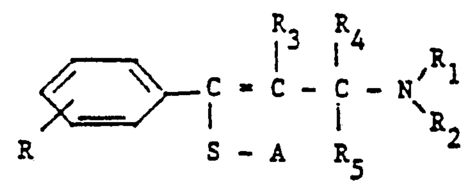

(XXXI)

oxidiert, das erhaltene Produkt dann isoliert und es gegebenenfalls in ein therapeutisch aktives Salz überführt.

11. Pharmazeutische Zusammensetzung, dadurch gekennzeichnet, daß sie mindestens ein Produkt gemäß Anspruch 1 zusammen mit einem oder mehreren verträglichen und pharmazeutisch annehmbaren Verdünnungs- oder Hilfsmitteln enthält.

**Patentansprüche** für den Vertragsstaat: AT

Verfahren zur Herstellung eines neuen 3-Phenyl-2-propenaminderivats der allgemeinen Formel:

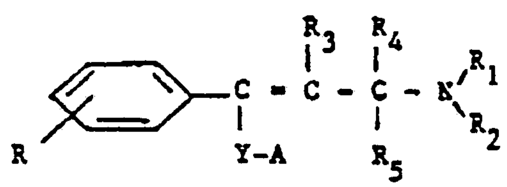

(I)

in welcher R ein Wasserstoff- oder Halogenatom oder einen Alkyl-, Alkyloxy-, Alkylthio-, Amino-, Alkylamino-, Dialkylamino- oder Trifluormethylrest darstellt, $R_3$ ein Wasserstoffatom oder einen Alkylrest darstellt,

- $R_4$ und $R_5$ entweder ein Wasserstoffatom darstellen und $R_1$ und $R_2$, die gleich oder verschieden sein können, ein Wasserstoffatom oder einen gegebenenfalls durch einen Alkenylrest mit 2 bis 4 Kohlenstoffatomen substituierten Alkylrest darstellen oder $R_1$ und $R_2$ zusammen mit dem Stickstoffatom, an das sie gebunden sind, einen gesättigten heterocyclischen Rest mit 4 bis 7 Gliedern, der gegebenenfalls ein weiteres Heteroatom wie Sauerstoff, Schwefel oder Stickstoff enthält und gegebenenfalle durch einen Alkylrestsubstituiert ist, darstellen,
- oder $R_4$ ein Wasserstoffatom darstellt, $R_1$ ein Wasserstoffatom oder einen Alkylrest darstellt und $R_2$ und $R_5$

zusammen einen Alkylenrest mit 3 oder 4 Kohlenstoffatomen bilden

und i) A einen Alkylrest oder einen unsubstituierten oder durch einen oder zwei Substituenten, ausgewählt aus den Halogenatomen und den Alkyl-, Alkyloxy-, Alkylthio-, Amino-, Alkylamino-, Dialkylamino-, Nitro- oder Trifluormethylresten, substituierten Phenylrest darstellt oder einen Pyridyl-, Benzyl- oder Cycloalkylrest mit 3 bis 6 Kohlenstoffatomen darstellt, Y ein Schwefelatom, einen Sulfinyl- oder Sulfonylrest oder auch einen Rest der allgemeinen Formel:

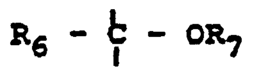

(II)

in welcher $R_6$ ein Wasserstoffatom oder einen Alkylrest darstellt und $R_7$ ein Wasserstoffatom oder einen Alkylcarbonyl-, Alkyloxycarbonyl-, Alkylaminocarbonyl- oder einen gegebenenfalls durch einen oder zwei Substituenten, ausgewählt aus den Halogenatomen und den Alkyl-, Alkyloxy-, Alkylthio-, Amino-, Alkylamino-, Dialkylamino-, Nitro- oder Trifluormethylresten, substituierten Benzoylrest darstellt,

ii) oder Y und A zusammen einen gegebenenfalls an einen Benzolring angefügten 1-Hydroxycycloalkylrest darstellen, dessen Ring 5 oder 6 Kohlenstoffatome enthält,

sowie von dessen pharmazeutisch zulässigen Salzen, wobei die Alkylreste und Alkylteile in den vorstehenden Definitionen 1 bis 4 Kohlenstoffatome in gerader oder verzweigter Kette enthalten und und Erfindung alle möglichen geometrischen und optischen Isomeren sowie deren Mischungen umfaßt, dadurch gekennzeichnet, daß man

I - zur Herstellung einer Verbindung der allgemeinen Formel (I), in welcher Y einen Rest der allgemeinen Formel (II) darstellt, worin $R_6$ die obige Bedeutung hat und $R_7$ ein Wasserstoffatom darstellt und A, R, $R_1$ und $R_2$ die obige Bedeutung haben mit Ausnahme von Wasserstoff für $R_1$ und/oder $R_2$, und welche in Z-Konfiguration vorliegt, eine Verbindung der allgemeinen Formel:

$A - CO - R_6$ (III)

in welcher A und $R_6$ die obige Bedeutung haben, mit einem Carbanion der allgemeinen Formel:

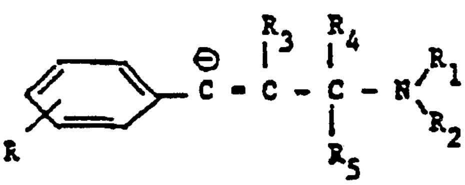

(IV)

in welcher die Symbole die obige Bedeutung haben, mit Ausnahme eines Wasserstoffatoms für $R_1$ und/oder $R_2$, reagieren läßt und dann die erhaltene Verbindung gegebenenfalls in ein pharmazeutisch zulässiges Salz umwandelt, oder daß man

II - zur Herstellung einer Verbindung der allgemeinen Formel (I), in welcher Y einen Rest der allgemeinen Formel (II) darstellt, in welcher $R_6$ und $R_7$ ein Wasseretoffatom darstellen, A die oben unter i) angegebene Bedeutung hat, $R_1$ ein Wasserstoffatom darstellt, $R_2$ ein Wasserstoffatom oder einen gegebenenfalls durch einen Alkenylrest mit 2 bis 4 Kohlenstoffatomen substituierten Alkylrest darstellt, R ein Wasserstoffatom darstellt, $R_3$ die obige Bedeutung hat und $R_4$ und $R_5$ ein Wasserstoffatom darstellen, und welche Verbindung Z-Konfiguration aufweist, den Dihydrofuranring in einer Verbindung der allgemeinen Formel

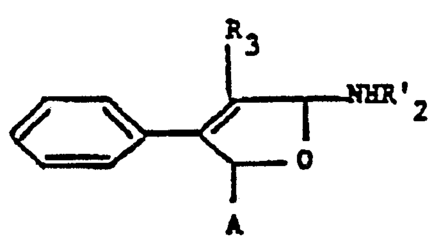

(XIV)

in welcher A die oben unter i) angegebene Bedeutung hat und $R'_2$ einen gegebenenfalls durch einen Alkenylrest mit 2 bis 4 Kohlenstoffatomen substituierten Alkylrest darstellt und $R_3$ die obige Bedeutung hat, öffnet, dann die erhaltene Verbindung isoliert und sie gegebenenfalls in ein pharmazeutisch zulässiges Salz umwandelt oder daß man

III - zur Herstellung einer Verbindung der allgemeinen Formel (I): in welcher Y ein Schwefelatom darstellt, $R_4$ und $R_5$ ein Wasserstoffatom darstellen, A die oben unter i) angegebene Bedeutung hat und die übrigen Symbole die obige Bedeutung, mit Ausnahme eines Wasserstoffatoms für $R_1$ und/oder $R_2$, haben, welche Verbindung in Z- oder E-Konfiguration vorliegt, ein Mercaptan der allgemeinen Formel:

A - SH (XVII),

in welcher A die oben unter i) angegebene Bedeutung hat, mit einer Verbindung der allgemeinen Formel:

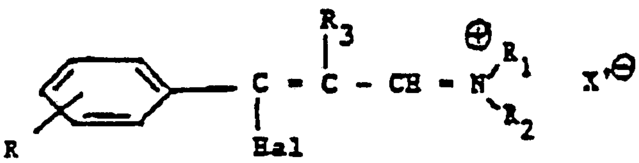
(XVIII)

in welcher R, $R_1$, $R_2$ und $R_3$ die obige Bedeutung, mit Ausnahme eines Wasserstoffatome für $R_1$ und/oder $R_2$, haben, Hal ein Halogenatom darstellt und $X'^{\ominus}$ ein Anion darstellt, reagieren läßt, dann das erhaltene Zwischenprodukt der allgemeinen Formel:

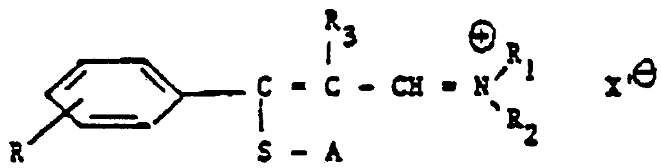
(XIX)

in welcher die Symbole die entsprechenden Bedeutungen haben, reduziert, dann die erhaltene Verbindung isoliert und gegebenenfalls in ein pharmazeutisch zulässiges Salz überführt, oder daß man

IV - zur Herstellung einer Verbindung der allgemeinen Formel (I), in welcher Y ein Schwefelatom darstellt, $R_4$ und $R_5$ ein Wasserstoffatom darstellen, A die oben unter i) angegebene Bedeutung hat und die übrigen Symbole die obige Bedeutung haben, welche Verbindung in E- oder Z-Konfiguration vorliegt, Ammoniak oder ein Amin der allgemeinen Formel:

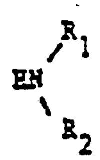
(XX)

in welcher $R_1$ und $R_2$ die obige Bedeutung haben, mit einem Aldehyd der allgemeinen Formel:

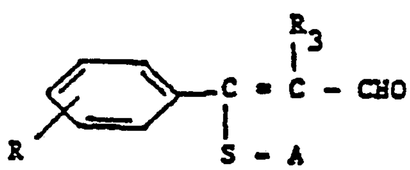
(XXI)

in welcher A die oben unter i) angegebene Bedeutung hat und R und $R_3$ die obige Bedeutung haben, und welcher in B- oder Z-Konfiguration vorliegt, umsetzt, und dann die erhaltene Verbindung isoliert und sie gegebenenfalls in ein therapeutisch zulässiges Salz umwandelt oder daß man

V - zur Herstellung einer Verbindung der allgemeinen Formel (I), in welcher Y einen Rest der allgemeinen Formel (II) darstellt, worin $R_7$ ein Wasserstoffatom darstellt, oder die oben unter ii) angegebene Bedeutung hat, $R_3$ ein Wasserstoffatom darstellt und die übrigen Symbole die obige Bedeutung haben, eine Organomagnesiumverbindung der allgemeinen Formel:

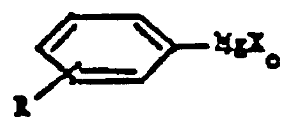
(XXIII)

in welcher R die obige Bedeutung hat und $X_0$ ein Halogenatom darstellt, mit einer Verbindung der allgemeinen Formel:

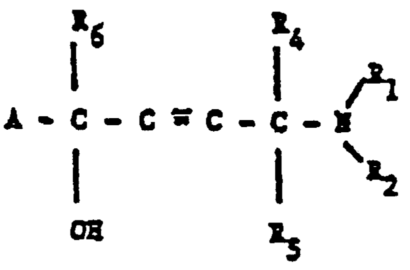

(XXIV)

in welcher die Symbole die obige Bedeutung haben, reagieren läßt, dann die erhaltene Verbindung isoliert und sie gegebenenfalls in ein pharmazeutisch zulässiges Salz überführt, oder daß man
VI - zur Herstellung einer Verbindung der allgemeinen Formel (I), in welcher Y einen Rest der allgemeinen Formel (II) darstellt, in welcher $R_7$ einen Alkylcarbonyl-, Alkyloxycarbonyl- oder Benzoylrest darstellt und die übrigen Symbole die obige Bedeutung haben, eine Verbindung der allgemeinen Formel:

$R'_7X_1$ (XXVII)

in welcher $R'_7$ einen Alkylcarbonyl-, Alkyloxycarbonyl- oder Benzoylrest darstellt und $X_1$ einen reaktiven Esterrest darstellt, mit einer Verbindung der allgemeinen Formel (I), in welcher Y einen Rest der allgemeinen Formel (II) darstellt, in welcher $R_7$ ein Wasserstoffatom darstellt und die übrigen Symbole die obige Bedeutung haben, d. h. einer Verbindung der allgemeinen Formel:

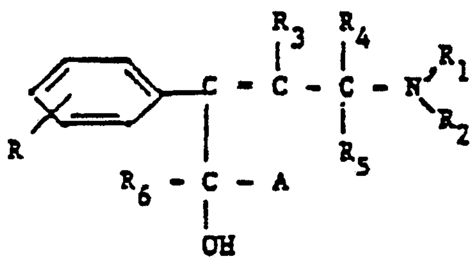

(XXVIII)

umsetzt, dann die erhaltene Verbindung isoliert und sie gegebenenfalls in ein pharmazeutisch zulässiges Salz überführt, oder daß man
VII - zur Herstellung einer Verbindung der allgemeinen Formel (I), in welcher Y ein Schwefelatom oder einen Rest der allgemeinen Formel (II) mit der obigen Bedeutung darstellt, $R_4$ und $R_5$ ein Wasserstoffatom darstellen und die übrigen Symbole die obige Bedeutung haben, welche Verbindung in E- oder Z-Konfiguration vorliegt, Ammoniak, einen Aminvorläufer oder ein Amin der allgemeinen Formel:

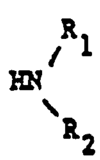

(XX)

mit einer Verbindung der allgemeinen Formel:

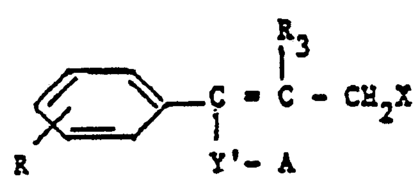

(XXIX)

in welcher A die ohen unter i) angegebene Bedeutung hat, R die oben angegebene Bedeutung hat und

a) - X entweder ein Chloratom darstellt und Y' ein Schwefelatom oder einen Rest der Formel (II) darstellt, worin $R_6$ und $R_7$ die obige Bedeutung, mit Ausnahme eines Wasserstoffatoms für $R_7$, haben,

b) - oder X ein Bromatom darstellt und Y' ein Schwefelatom darstellt,

und welche Verbindung in E- oder Z-Konfiguration vorliegt, umsetzt, dann die erhaltene Verbindung gegebenenfalls hydrolysiert, wenn man die Verbindung der allgemeinen Formel (I) erhalten will, worin Y einen Rest der allgemeinen Formel (II) darstellt, in welcher $R_7$ ein Wasserstoffatom darstellt und die übrigen Symbole die obige Bedeutung haben, dann die erhaltene Verbindung isoliert und sie gegebenenfalls in ein therapeutisch zulässiges Salz umwandelt, oder daß man

VIII - zur Herstellung einer Verbindung der allgemeinen Formel (I), in welcher Y einen Rest der allgemeinen Formel (II) darstellt, worin $R_7$ einen Alkylaminocarbonylrest darstellt und die übrigen Symbole die obige Bedeutung haben, ein Isocyanat der allgemeinen Formel:

$$O = C = N - R''_7 \qquad (XXX)$$

in welcher $R''_7$ einen Alkylrest darstellt, mit einer Verbindung der allgemeinen Formel (I), in welcher Y einen Rest der allgemeinen Formel (II) darstellt, worin $R_7$ für ein Wasserstoffatom steht und die übrigen Symbole die obige Bedeutung haben, d. h. einer Verbindung der allgemeinen Formel:

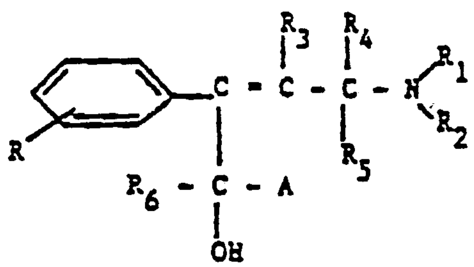

(XXVIII)

umsetzt, dann die Verbindung isoliert und sie in ein pharmazeutisch zulässiges Salz überführt, oder daß man

IX - zur Herstellung einer Verbindung der allgemeinen Formel (I), in welcher Y einen Sulfinyl- oder Sulfonylrest darstellt und die übrigen Symbole die obige Bedeutung haben, eine Verbindung der allgemeinen Formel (I), in welcher Y ein Schwefelatom darstellt und die übrigen Symbole die obige Bedeutung haben, d. h. eine Verbindung der allgemeinen Formel:

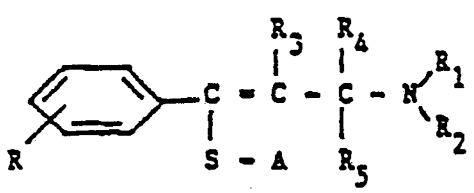

(XXXI)

oxidiert, dann die erhaltene Verbindung isoliert und sie gegebenenfalls in ein therapeutisch aktives Salz umwandelt.